# EUROPEAN PATENT SPECIFICATION

(11) **EP 4 426 280 B1**
(45) Date of publication and mention of the grant of the patent: **18.03.2026**
(21) Application number: 22829908.7
(22) Date of filing: 15.12.2022
(51) Int. Cl.: A61K 31/185, A61K 31/522, A61K 38/02, A61P 33/00, A61K 38/08, A61K 45/06

(54) **PROTECTION AGAINST ARTHROPOD PARASITES WITH PURINERGIC RECEPTOR (OPR) ANTAGONISTS**
SCHUTZ GEGEN ARTHROPODENPARASITEN MIT ANTAGONISTEN DER PURINERGIKREZEPTOREN
PROTECTION CONTRE LES PARASITES ARTHROPODES AVEC LES ANTAGONISTES DES RECEPTEURS PURINERGIQUES

(30) Priority: 16.12.2021 EP 21215129
(43) Date of publication of application: 11.09.2024
(73) Proprietor: Gurba, Alexandre, 3014 Bern (CH); Vernudachi, Alexandre, 37000 Tours (FR); Kröber, Thomas, 2000 Neuchâtel (CH); Guerin, Patrick, 2114 Fleurier (CH)
(72) Inventor: GUERIN, Patrick, 2114 Fleurier (CH); VERNUDACHI, Alexandre, 37000 Tours (FR); KRÖBER, Thomas, 2000 Neuchâtel (CH)
(74) Representative: P&TS SA (AG, Ltd.)
(86) International application number: PCT/IB2022/062297
(87) International publication number: WO 2023/111938

(56) References cited:
- WO-A1-2017/139580
- VIJAYAMAHANTESH ET AL: "Tinkering with targeting nucleotide signaling for control of intracellularLeishmaniaparasites", CYTOKINE, vol. 119, 2019, pages 129 - 143, XP085690290, ISSN: 1043-4666, DOI: 10.1016/J.CYTO.2019.03.005
- MARQUES ADRIANA FONSECA ET AL: "Falcipain-2 inhibition by suramin and suramin analogues", BIOORGANIC, ELSEVIER, AMSTERDAM, NL, vol. 21, no. 13, 25 April 2013 (2013-04-25), pages 3667 - 3673, XP028558897, ISSN: 0968-0896, DOI: 10.1016/J.BMC.2013.04.047
- ALVES VINÍCIUS SANTOS ET AL: "Purinergic signaling in infectious diseases of the central nervous system", BRAIN, BEHAVIOR AND IMMUNITY, ACADEMIC PRESS, SAN DIEGO, CA, US, vol. 89, 24 July 2020 (2020-07-24), pages 480 - 490, XP086288326, ISSN: 0889-1591, [retrieved on 20200724], DOI: 10.1016/J.BBI.2020.07.026
- ENANGA B ET AL: "Sleeping sickness and the brain", CMLS CELLULAR AND MOLECULAR LIFE SCIENCES, BIRKHAUSER VERLAG, HEIDELBERG, DE, vol. 59, no. 5, 1 May 2002 (2002-05-01), pages 845 - 858, XP037558382, ISSN: 1420-682X, [retrieved on 20020501], DOI: 10.1007/S00018-002-8472-0

## Description

### Technical domain

The present invention concerns a product for protecting humans and host animals against arthropod parasites. The invention also concerns a method of selecting suitable active agents for said products.

### Related art

Arthropod parasites comprising arthropod ectoparasites and arthropod endoparasites impact on human and animal health. Mosquitoes, ticks, fleas, lice and mites attack humans or burrow in human skin inflicting injury. In addition, arthropod ectoparasites and endoparasites affect livestock production, reducing animal well-being and performance in terms of weight gain and productivity. Arthropod ectoparasites also serve as vectors of major pathogens that have a substantial worldwide socioeconomic impact causing morbidity and mortality in humans and farmed animals.

Agents employed to control arthropod parasites are primarily chemical, accounting for nearly a quarter of the total animal health market. Yet, with only a few exceptions, active ingredients mostly originate from chemicals developed for crop protection (WO2017/139580 A1). Macrocyclic lactones, such as ivermectin, are used extensively against arthropod endoparasites and ectoparasites. Other ectoparasiticides include pyrethroids, carbamates, neonicotinoids, spinosyns, insect growth regulators and isoxazolines.

With the exception of isoxazolines, one of the few active ingredients introduced this century, resistance to the other chemical classes of parasiticides is widespread and growing rapidly. Control of malaria, transmitted by mosquitoes, is currently highly dependent on arteminisin-based drug combination therapies but resistance to arteminisin has developed rapidly in Asia and Africa over the past decade. Added to this problematic are changing environmental conditions which, allied with transport of people and animals on a global scale, enable arthropod parasites to colonize further geographic zones. The spread of these parasites in combination with their ever-increasing resistances to conventional control agents are of growing concern for human and animal health.

There is therefore an urgent need for the identification and development of advanced compositions and active agents with alternative modes of action against arthropod parasites. In order to protect the health of host species in a changing environment, new strategies for controlling arthropod parasite growth and survival are required.

### Short disclosure of the invention

It is an aim of the present invention to provide an alternative to existing methods and compositions for reducing damage inflicted by arthropod parasites on their host organisms.

It is a further optional aim of the invention to provide an alternative method to control the population of arthropod parasites. In particular, growth of the population should be curtailed and the parasites capacity to spread should be reduced.

Moreover, the alternative should preferably be suited to prevent blood-feeding arthropod parasites from transmitting parasites and/or infectious agents onto and between their hosts.

According to the invention, these aims are attained by the object of the attached claims, and especially by the independent claims.

In particular, the aims are achieved by using a product comprising one or more purinergic receptor antagonists to protect animals and/or humans from arthropod parasites by preventing the parasite from feeding on host tissue, including blood. Any references in the description to methods of treatment refer to the compounds, pharmaceutical compositions and medicaments of the invention for use in a method of treatment of the human body by therapy.

Arthropod parasites all belong to a monophyletic group of molting animals. Arthropod parasites may, for example, be biting insects, ticks, mites and crustaceans.

Hematophagous parasites, also called blood-feeders, comprise amongst others the group of blood-feeding arthropods, such as biting insects, as well as ticks.

Known purinergic receptors (PRs) are plasma-membrane molecules activated by extracellular purines, including nucleotides such as adenosine triphosphate (ATP) and nucleosides such as adenosine. While ATP is well known to drive energy-consuming processes within cells, purines also modulate biological processes through their extracellular action on these PRs.

In humans, for example, extracellular ATP serves as a co-transmitter with classic neurotransmitters within the central nervous system and the peripheral nervous system, as well as a signalling molecule for most human tissues and cell types. Due to their significance in pathologies, human PRs have been subject to extensive studies.

Vertebrate PRs are broadly divided into P1 PR and P2 PR classes.

The first class comprises metabotropic P1 PRs. P1 PRs are adenosine-sensitive PRs formed by a single polypeptide with 7 trans-membrane α-helices set perpendicular to the plasma membrane which respond, for example, to extracellular adenosine. P1 PRs can oligomerise to form homo-oligomers or hetero-oligomers such as the P1A₁-A_{2A} dimer. P1 PRs can also oligomerise with other receptor types. P1 PR subtypes show different affinities for adenosine, with the A₁ subtype showing the highest affinity. Adenosine and adenosine 5'-monophosphate (AMP) are full agonists for human P1A₁ PR.

The second class comprises P2 PRs, which respond to ATP and other nucleotides such as α,β-methylene ATP. This class is further subdivided into ionotropic ATP-gated P2X PRs and metabotropic nucleotide-sensitive P2Y PRs that respond to ATP, adenine 5'-diphophate (ADP) and uridine 5'-triphosphate (UTP). Ligand-gated P2X PRs are composed of P2X₁-P2X₇ subunits that form homomeric or heteromeric trimers. ATP is a full agonist for P2X₂, P2X₃ and P2X_{2/3} P2 PR subtypes.

While P2X PRs have primarily been studied in connection with their implication in neurological pathology, members of this class have also been found in organisms as diverse as an amoeba, a single-celled green algae and invertebrate species. However, the homologous receptors identified in these species show a low sequence homology of less than 45% with human P2X PRs.

Despite the pharmaceutical interest in these receptors, the role of PRs in arthropod parasites, including insect vectors of disease, remains obscure today. One reason for this is lack of evidence for P2 PRs in the sequenced genome in well-investigated insect vectors, such as the African malaria mosquito *Anopheles gambiae.* In fact, P2X PRs are considered absent from insects. Whole genome sequences of blood-feeding insect vectors of disease are apparently devoid of genes coding for P2X PR homologues (Hou, Z. and Cao, J. 2016 Comparative study of the P2X gene family in animals and plants. Purinergic Signalling 12, 269-281).

Despite this lack of any molecular evidence for known PRs in insect vectors of disease, the inventors could prove the presence of a purine-sensitive cell on the mouthparts of the malaria mosquito, which responds to ATP and adenosine by increasing its action potential frequency. In addition, the inventors have established that ATP acts as a feeding stimulant for the malaria mosquito as measured by increased feeding by this mosquito species on a feeding medium to which ATP is added in solution. These findings are contrary to what is reported in the art.

Moreover, the inventors show for the first time that known P1, P2X₂, P2X₃, P2X_{2/3} antagonists of vertebrate PRs are effective inhibitors of the electrophysiological response of the purine-sensitive cell of arthropod parasites, such as mosquitos. Adding an antagonist of a P1 PR in solution with adenosine causes inhibition in action potential frequency recorded from the purine-sensitive cell compared to when adenosine is presented without the antagonist. Likewise, adding an antagonist of P2X₂, P2X₃, P2X_{2/3} PRs in solution with ATP causes inhibition in action potential frequency recorded from the purine-sensitive cell compared to when ATP is presented without the antagonist.

The inventors provide evidence of the presence of a purine-sensitive cell, which can be inhibited by PR antagonists, including known antagonists of human PRs, in the mouthpart of the malaria mosquito *Anopheles gambiae.*

Remarkably, the inventors demonstrate that by interfering with the activation of PRs, PR antagonists block the blood-feeding behaviour of hematophagous arthropod parasites, such as biting insects and ticks.

It could be shown that the same P1, P2X₂, P2X₃, P2X_{2/3} PR antagonists of vertebrate PRs inhibit feeding of two mosquito species, two biting fly species, and three tick species on a feeding medium in which either adenosine or ATP, or adenosine plus ATP is present as a feeding stimulant. PR antagonists, such as P1, P2X₂, P2X₃, P2X_{2/3} PRs antagonists, are therefore promising alternatives to existing methods to control blood-feeding arthropod parasites on humans and host animals through inhibition of their PRs.

As will be described in greater detail below, the efficacy of specific PR antagonists to inhibit parasites from feeding on host blood may vary according to the arthropod species and the type of PR a specific species expresses.

The invention concerns the use of a product comprising a purinergic receptor antagonist, wherein the antagonist inhibits the arthropod parasite from feeding on host tissue.

Products comprising PR antagonists may be directed at arthropod ectoparasites, for example a blood-feeding ectoparasite.

Products comprising PR antagonists may also be directed at arthropod endoparasite. Such products may be used therapeutically. They may also be used as a preventative measure to protect against infection by an arthropod endoparasite. The products may be used to prevent the establishment of an arthropod endoparasite in the host body.

The product may comprise only one PR antagonist compound. The product may also comprise different PR antagonist compounds. The product my comprise different compounds which are antagonists to one or more P2X PR subtypes. The product may comprise different compounds, which are antagonists to one or more of P1 PR subtypes. The product may also comprise a mixture of antagonists directed towards P1 PRs and antagonists directed against P2X PRs. Additionally, the product may comprise metal modulators, which are suitable for inhibiting PRs.

PR antagonist compounds, including PR antagonists that act selectively on either P1 or on P2 PR classes, or that act selectively on a particular PR subtype within a PR class, are known in the art. Several examples of PR antagonist compounds are mentioned in subsequent sections. It is understood that this invention is not limited to the examples of known PR antagonist compounds mentioned herein. The skilled person understands that other PR antagonists are equally suitable for the purpose of this invention, most importantly the claimed use of the product.

The products may be directed against different kinds of arthropod parasites. The product may for example be used against arthropod ectoparasites, preferably against biting insects. The product may also be used to protect against arachnids, such as ticks, for example.

Moreover, the product may be used against endoparasites that may be arthropod endoparasites. If the product is used against endoparasites, it be may be provided in form of a therapeutically active formulation.

The products should be used to protect against arthropod parasites having at least one PR and/or one purine-sensitive cell. The PR may be P1 type PR or a P2 type PR. The PR may also be a homolog to a known vertebrate PR. It may also be a new type of PR. Depending on the kind of PR the arthropod parasite expresses and displays on its cells, it may respond to nucleotides, preferably ATP or to nucleosides, preferably adenosine, as feeding stimulant. It is also possible, that the arthropod parasite responds to both feeding stimulants, to ATP and/or other nucleotides, and to adenosine and/or other nucleosides and to a combination nucleotide and nucleoside feeding stimulants.

A suitable formulation comprising PR antagonists may be administered systemically to a host. It is also possible, that the product will be formulated for a targeted delivery of the PR antagonist compounds, respectively the PR metal modulators.

Compounds that inhibit P1, P2X₂, P2X₃, P2X_{2/3} PRs of a blood-feeding arthropod parasite may be formulated using known methods and deployed to inhibit feeding by an arthropod parasite on or in a host animal. However, this invention is not limited to any specific formulation of the PR antagonist comprising product.

Depending on its intended application, the product comprising PR antagonist compounds and PR metal modulators may be provided in different forms. The product may for example be provided as a liquid composition. The liquid composition is preferably water-based, optionally containing a molecule or molecules that enhances solubility of the PR antagonist in said liquid composition. It may be a water dispersible emulsion.

A liquid product could be suitable for spraying onto surfaces, for example clothes or mosquito nets, or onto host animals and humans, or onto a habitat occupied by an arthropod parasite.

The product may also be provided in solid form, for example as a powder.

The concentration of the PR antagonists in the product should be chosen according to the inhibitory activity, as preferably determined by an inhibitory concentration IC, for example the IC₅₀ value, or an inhibitory dose ID, for example the ID₅₀ value, of each PR antagonist compound. In addition, the mode of application, for example topically or systemically, should also be considered when determining a suitable concentration of PR antagonists in the product. A liquid product for example, may comprise up to 50 wt% or up to 5 wt% of total PR antagonist compounds. Depending on their mode of use, less concentrated products may be preferable. The content of PR antagonist compounds in a less concentrated product may, for example, be no less than 10 ⁻⁹ wt% or more than 10 ⁻³ wt %.

It was furthermore shown for blood-feeding arthropod parasites that metal modulators inhibit the response of PRs to its agonists. Metal modulators are suitable for inhibiting blood-feeding behaviour in hematophagous parasites. To inhibit blood-feeding behaviour metal modulators may for example be provided in combination with PR antagonist compounds.

The invention also concerns methods for screening and selecting PR antagonist candidate compounds for their efficacy in inhibiting blood-feeding behaviour of arthropod parasites.

The methods may be based on screens of arthropod test animals. Such arthropod test animals may be arthropod parasites, such as blood-feeding ectoparasites. The arthropod test animals may also be transgenic animals. Alternatively, the method may be based on a heterologous cell systems.

The present invention provides a means to inhibit feeding by an arthropod parasite by interfering with its ability to sense nucleosides and nucleotides. This is achieved by inhibiting the arthropod parasite's P1, P2X₂, P2X₃ or P2X_{2/3} PR or any combination of said PRs using PR antagonists.

The present invention therefore provides a novel means to stop or so reduce feeding by an arthropod parasite and to prohibit it from inflicting injury to the host animal through biting, through establishing a feeding site, through injury, through loss of blood, through loss of vital body fluids, through loss of essential nutrients and/or through loss of tissue or cells by the host animal.

Reduced feeding on the host animal also reduces the capacity of an arthropod parasite to transmit pathogens to its host.

Furthermore, reduced feeding by the arthropod parasite diminishes its capacity to grow and multiply which, in turn, leads to a lower population density of the arthropod parasite in subsequent generations.

This invention provides a protection against arthropod parasites based on an entirely new mechanism targeting a molecular structure, i.e. a purinergic receptor, which has never been used for this purpose before. By providing this new protection mechanism, the invention overcomes the current acute problem of resistances against known insecticides, acaricides and parasiticides employed to control these parasites.

### Terms and definitions used herein:

Action potential: The terms action potential and spike are used synonymously herein.

Activates: As used herein the terms "activates", "activation" and "activated", mean to enhance the function. In respect of receptors and in particular of a P1, P2X₂, P2X₃ and/or P2X_{2/3} PR activate means to enhance the functioning of said receptor.

Agonist: The term "agonist" as used herein means a compound that enhances the action of a molecule or a molecular structure, for example a receptor, or a function of said molecule or molecular structure. Specifically, a "PR agonist" is a compound that activates a molecule or molecular structure which is a PR and/or which exhibits a PR function. Use of the term agonist herein comprises a plurality of agonists.

Antagonist: The term "antagonist" as used herein means a compound that inhibits, reduces, or prevents the action of another molecule or molecular structure, such as a receptor, or the function of said molecule or molecular structure. Specifically, a "PR antagonist" is a compound that inhibits, reduces, or prevents the action of another molecule, or prevents the action of a molecule or molecular structure which is a PR and/or which exhibits a PR function.

Arthropod parasite: The term arthropod parasite as used herein means a parasitic arthropod living on or within a host animal that obtains nutrients from the host animal through routes comprising an arthropod parasite's mouthparts, or by absorption or adsorption of host animal nutrients through the integument or plasma membrane of an arthropod parasite for survival, growth, and reproduction. Nutrients obtained from the host animal may comprise blood or blood cells, nutrients from within a cell, and nutrients may be obtained when an arthropod parasite feeds on a cell or cells, on tissue, on keratinous particles, on epidermal particles, on fur, on feathers, on nutrients from hair, on feather follicles, on hypodermal tissue, on tissue fluids, on sebaceous and exocrine gland secretions, on lymph, on inflammation products, on lysed tissue, on content of the gastrointestinal system, on mucous membranes, on mucus, and when feeding on blood is restricted to a particular life stage of an arthropod parasite. Accordingly, an arthropod parasite comprises such means of obtaining nutrients from the host animal. Arthropod parasites comprise arthropod ectoparasites and arthropod endoparasites. Arthropod parasites also comprise micro-predators. Arthropod parasites are important vectors of disease. Use of the term arthropod parasite herein comprises a plurality of arthropod parasites.

Blood-feeder: The term "blood-feeder" as used herein means blood-feeding arthropod parasite.

Blood-feeding insect: The term "blood-feeding insect" as used herein is synonymous with biting insect.

Biting insect: The term "biting insect" as used herein comprises mosquitoes, sandflies, tsetse flies, black flies, tabanid horse and deer flies, stable flies *Stomoxys calcitrans,* horn flies *Haematobia irritans* and other members of the genus *Haematobia,* snipe flies, midges, fleas, lice, true bugs, bed bugs and triatomine bugs. These insects can serve as vectors of diseases and infections. During blood-feeding, biting insects may transmit parasites and infectious agents into humans and host animals as for example into domesticated farm animals. Parasites transmitted by biting insects comprise, for example, malaria, trypanosomiasis, surra, leishmaniasis, onchocerciasis, filariasis and loiasis. Infectious agents transmitted by biting insects comprise, amongst others, dengue virus, yellow fever virus, Zika virus, West Nile virus, chikungunya, plague, *Bacillus anthracis,* Bluetongue virus and lumpy skin disease virus. Use of the term biting insect herein comprises a plurality of biting insects.

Arthropod ectoparasite: The term "arthropod ectoparasite" as used herein means a parasitic arthropod that survives on blood, on or in the skin or epidermis, or that feeds on outgrowths of the skin or epidermis, or that feeds on subcutaneous or intracutaneous tissue or other tissue or on any other fluids of a host animal and comprises ectoparasitic arthropods that include, amongst others, biting insects, face flies, sheep head flies, louse flies or keds of the family Hippoboscidae, bat flies of the superfamily Hippoboscoidea, sucking lice, chewing lice, biting lice, poultry lice, fleas, ticks, feather mites, follicle mites, quill mites, depluming mites, fur mites, chigger mites, chicken mite or roost mite *Dermanyssus gallinae,* northern fowl mite *Ornithonyssus sylviarum,* tropical fowl mite *Ornithonyssus bursa,* cattle ear mite, sheep itch mite, otoacariosis mite, scaly-leg mite *Knemidocoptes mutans,* the tracheal mite and varroa mite of honey bees, isopod parasites of fish and aquatic invertebrates, copepod parasites of mollusks, amphipod or scud parasites of mollusks and other aquatic invertebrates, copepods parasites on fish such as species of the family Ergasilidae or gill lice, lernaeid anchor worms of the copepod genus *Lernaea* on freshwater fish such as carp and goldfish, copepod anchor worms on marine fish, cuckoo copepods or nicothoid parasites of lobsters and spider crabs, sea lice of *Lepeophtheirus* spp. and *Caligus* spp. on salmonids, and sea louse host-animal infective free-living copepodid life stages. Ectoparasites can transmit parasites and infectious agents and can also debilitate host animals through blood-feeding, anaemia, irritability, dermatitis, loss of weight, skin necrosis, secondary infection, inoculation of toxins, paralysis, and death. The term bleed-feeding ectoparasite as used herein comprises also animals classified as blood-feeding micro-predators. Use of the term arthropod ectoparasite herein comprises a plurality of arthropod ectoparasites.

Arthropod endoparasite: The term "arthropod endoparasite" as used herein means a parasitic arthropod that lives and grows inside a host animal and comprises endoparasitic arthropods of vertebrate animals that are intracutaneous-, tissue- and cavity-dwelling organisms including, amonst others, mites of the Sarcoptidae family causing sarcoptic mange or scabies in the skin of humans and animals, mites of the nasal cavity, tracheae, lungs and of the gastric mucosa of birds and mammals, sarcoptic mites of the buccal mucosa of mammals, botfly larvae of the family Oestridae parasitizing the pharyngonasal cavity and throat of mammals, cattle grub flies or gadflies of the genus *Hypoderma* responsible for subdermal cysts or warbles on host animals, botfly larvae of the family Gasterophilidae in the gastric mucosa of horses and donkeys, fly larvae feeding on vertebrate host tissue, for example, myiasis or flesh flies of the families Calliphoridae, Muscidae and Sacrophagidae such as the New World Screwworm *Cochliomyia hominivorax,* Old World Screworm *Chrysomya bezziana* and *Wohlfahrtia magnifica,* and larvae of the botfly families Cuterebridae and Hypodermatidae. Endoparasitic arthropods of invertebrates include parasitic barnacles such as the rhizocephalan parasites or *Sacculina* spp. barnacle parasites of the reproductive system of crabs. Use of the term arthropod endoparasite herein comprises a plurality of arthropod endoparasites.

Feed: The term "feed" as used herein means obtaining nutrients by a mosquito, biting fly, tick, mite, or by any other arthropod parasite relying on host-animal fluid or host-animal tissue for survival. Accordingly, "feeding" is the process or state of bringing host-animal fluid, cells, tissue, or nutrients inside an arthropod parasite.

Feeding inhibitor: The term "feeding inhibitor" as used herein means a compound which modifies an arthropod parasite's behaviour, such that the parasite does not feed on host resources comprising blood, nutrients within a cell, a cell or cells, tissue, tissue fluids, lymph, content of the gastrointestinal system, mucus or host mucous membranes. The terms "inhibit feeding", "inhibits feeding", "inhibition of feeding", "inhibiting feeding", "inhibitor of feeding", "reduce feeding", "prevent feeding", "block feeding" and "control feeding" as used herein comprise what a feeding inhibitor for an arthropod parasite achieves. A feeding inhibitor for an arthropod parasite can be composed of one or more compounds.

Engorgement: The terms "engorgement" and "engorged" as used herein mean to swell with blood, cells, tissue, tissue fluids, lymph, or with any other body fluid or nutrient taken from a host animal during the process of feeding by an arthropod parasite. The terms "engorgement" and "engorged" as used herein also mean to swell with fluid taken from a feeding medium by an arthropod parasite during the process of feeding. Accordingly, the terms engorgement, engorged, feeding and fed are used synonymously herein.

Express: The terms "express", "expression", "expresses", "expressing" and "expressed" as used herein mean the biological process of a cell by which a protein such as a receptor protein is synthesized by transcription and translation from information stored in a gene into a polypeptide which can be further processed within the cell such as by protein folding. The polypeptides constituting receptors of relevance to this disclosure are in addition transported into the plasma membrane where they organize to confer on the cell its purine-sensitive phenotype.

Feeding-stimulant: The term "feeding-stimulant" as used herein means a compound or combination of compounds which induce feeding behaviour of an arthropod parasite. The feeding stimulants used in this invention are purinergic compounds.

Hematophagous: The terms "hematophagous" and "blood-feeding" are used synonymously herein.

Host animal: The terms "host" or "host animal" as used herein comprise animals which can carry or harbour an arthropod parasite or may be prey for a micro-predator. Hosts or host animal include, without limitation, mammals, humans and animals such as cattle, buffalo, bison, pigs, donkeys, horses, sheep, goats, camels, deer, reindeer, dogs, cats, hamsters, rabbits, birds, cage birds, canary, parrot, finch, hens, chickens, ducks, geese, turkeys, pigeons, ostrich, reptiles, turtles, fish, farmed fish, aquarium fish, salmon, caged salmon, cod, trout, carp, sole, bass, sturgeon, turbot, tilapia, arthropods, crustaceans, shrimps, crabs, lobsters, crayfish, prawns, insects, honey bees, molluscs, slugs, snails, squid, mussels, scallops, clams and oysters. Use of the term host animal herein comprises a plurality of host animals.

Inverse agonist: The term "inverse agonist" as used herein means a compound that can reduce constitutive receptor activity regulating cellular signalling in absence of an agonist.

Micro-predator: The term "micro-predator" as used herein is an hematophagous arthropod which attacks more than one host, reducing each host's fitness by at least a small amount, and is only in contact with any one host intermittently. This behaviour makes micro-predators suitable as vectors, as they can pass smaller parasites from one host to another. The term micro-predator as used herein is comprised in the term ectoparasite defined above.

Modulator: The terms "modulator", "modulate", "modulation", "modulated" and "modulating" as used herein mean a change in the activity of a target molecule or a target multi-molecular structure forming a receptor. Modulation may cause an increase or decrease in activity of said target molecule or a target multi-molecular structure, for example through changes in binding capacity, in ion flux changes in receptor molecules, ion channel opening, ion channel conductance or in other functional characteristics of receptor molecules. Accordingly, a "modulator" is a compound that interacts with another molecule or multi-molecular structure, or which alters the function of a molecule or multi-molecular structure. Use of the term modulator herein comprises a plurality of modulators.

Non-parasitic organism: The term "non-parasitic organism" as used herein comprises organisms that do not feed on a host animal to survive, for example humans, rat and the fruit fly *Drosophila melanogaster.*

Organism: The terms "animal" and "organism" are used synonymously herein.

Parasite: The term "parasite" is an organism that lives in or on an organism of another species, a host, and benefits by deriving nutrients at the other's expense.

Pharmacologically acceptable salts: Reference herein to pharmacologically acceptable salts includes solvates, hydrates and polymorph crystal forms of same.

Purinergic receptor: The term "purinergic receptor" or "PR" as used herein means a plasma-membrane molecule or molecular structure activated by, amongst others, adenosine 5'-triphosphate (ATP), related nucleotides adenine 5'-diphophate (ADP) and uridine 5'-triphosphate (UTP), and/or by adenosine 5'-monophosphate (AMP) or adenosine. A PR permits an arthropod parasite to detect and respond to nucleotides and/or nucleosides, for example, on a host animal's skin, in a host animal's blood, in a host animal's body fluids, in a host animal's tissues, in a cell of a host animal, in a host animal's tissue fluids, in a host animal's lymph, in a host animal's gastrointestinal content and in a host animal's mucous membranes. Purinergic receptors (PRs) are divided into two broad classes, P1 PRs activated by adenosine and P2 PRs activated by ATP, ADP and UTP. Use of the term PR herein includes a plurality of purinergic receptors (PRs).

Purine-sensitive cell: The term "purine-sensitive cell" as used herein means a sensory neuron expressing at least one PR that allows an arthropod parasite to detect a nucleoside such as adenosine and/or a nucleotide such as ATP in the cell's surroundings, for example, on a host animal's skin, in a host animal's blood, in a host animal's body fluids, in a host animal's tissues, in a cell of a host animal, in a host animal's tissue fluids, in a host animal's lymph, in a host animal's gastrointestinal content and in a host animal's mucous membranes. A purine-sensitive cell forms part of the purine-sensing system of an arthropod parasite.

Tick: The term "tick" as used herein means an organism belonging to the Arachnida and, like biting insects, is an arthropod that feeds on blood. Ticks are found throughout the world and are divided into Ixodidae, hard ticks, and Argasidae, soft ticks. Parasites transmitted by ticks comprise theileriosis, babesiosis, anaplasmosis and heartwater disease. Infectious agents transmitted by ticks comprise Lyme disease, bovine borreliosis, relapsing fever, tularaemia, Q-fever, Crimean-Congo haemorrhagic fever, tick-borne encephalitis and louping-ill. Use of the term tick herein comprises a plurality of tick species.

Vertebrate: The term "vertebrate" as used herein means an animal that develops and possesses a vertebral column or backbone. Vertebrates comprise, for example, humans and domesticated animals. Use of the term vertebrate herein comprises a plurality of vertebrate species.

### Short description of the drawings

Exemplar embodiments of the invention are disclosed in the description and illustrated by the drawings in which:

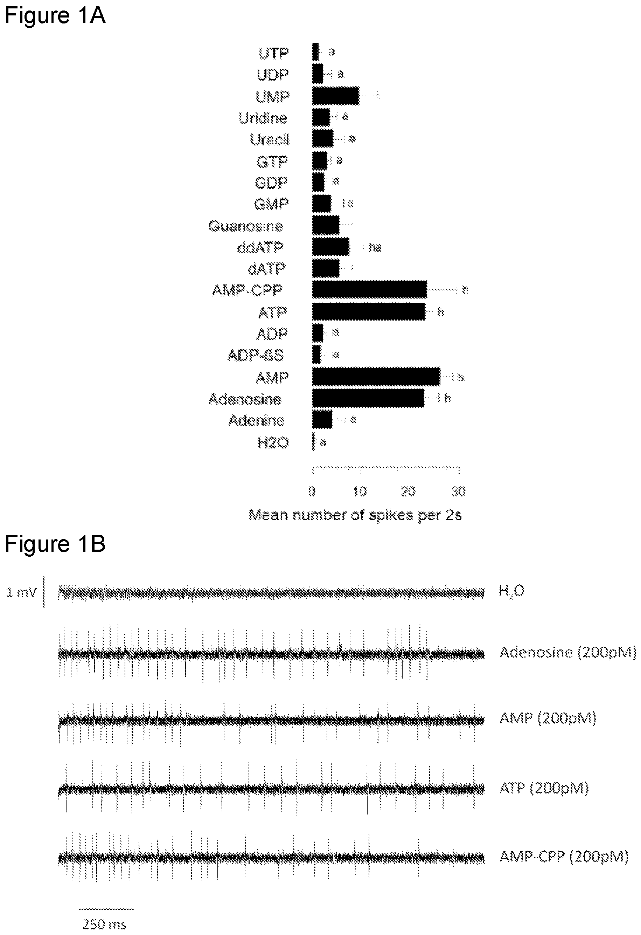

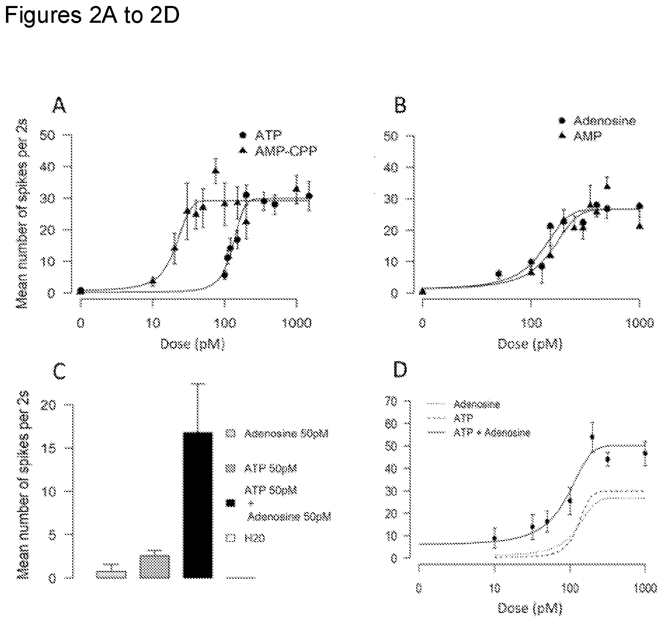

**Figure 1A** and **B:** Mean spike frequencies / 2 s (± s.e.m.) generated in response to 200 pM doses of nucleotides, nucleosides, uracil and adenine presented to *Anopheles gambiae* (*A. gambiae*) labellum sensilla **(****Figure 1A****)** and representative 2 s recordings **(****Figure 2B****).** Responses, which are significantly different to the water negative control are indicated by "h" (P<0.05) and spike frequencies significantly lower than those recorded for ATP are indicated by "a" (P<0.05). Treatment repetitions (n) are reported in Materials and Methods.
**Figure 2A** to **2D****:** Number of spikes (mean ± s.e.m.) per 2s recorded from *A. gambiae* labellum sensilla in response to increasing doses of ATP and AMP-CPP **(****Figure 2A****),** to adenosine and AMP **(****Figure 2B****),** to 50 pM of adenosine, 50 pM of ATP, and to an equimolar mixture of 50 pM ATP and adenosine with water as negative control (0 spikes) **(****Figure 2C****),** and to increasing equimolar doses of ATP admixed with adenosine (continuous line) compared to responses to increasing doses of ATP and adenosine (respectively, dashed and dotted lines, data as in A and B) **(****Figure 2D****).** Treatment repetitions (n) are reported in Materials and Methods.

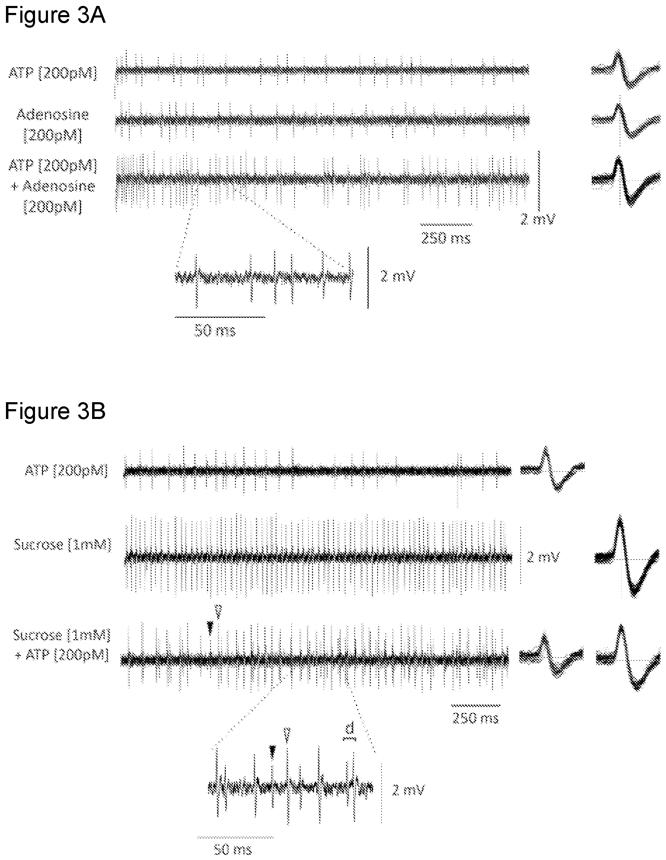

**Figure 3A****:** Two second recordings from a female *A. gambiae* labellum sensillum presented with ATP, adenosine and an equimolar 200pM mixture of ATP plus adenosine. Discrimination for action potentials of similar amplitudes and durations (on right) indicate that treatments elicited responses characterized by one spike class.
**Figure 3B****:** Typical 2 second recordings from the sensillum of Figure 3A presented with 200pM ATP, 1mM sucrose and 1mM sucrose admixed to 200pM ATP. Spike amplitudes elicited by sucrose are 1.7 times higher than those elicited by ATP or adenosine, permitting discrimination of two spike classes each distinguished by similar amplitudes and durations (on right) elicited by sucrose (open arrowhead at bottom) and by ATP (closed arrowhead at bottom). A spike doublet (d) resulting from near simultaneous firing by the sucrose-sensitive and ATP-sensitive cells is indicated. Treatment repetitions (n) are reported in Materials and Methods.

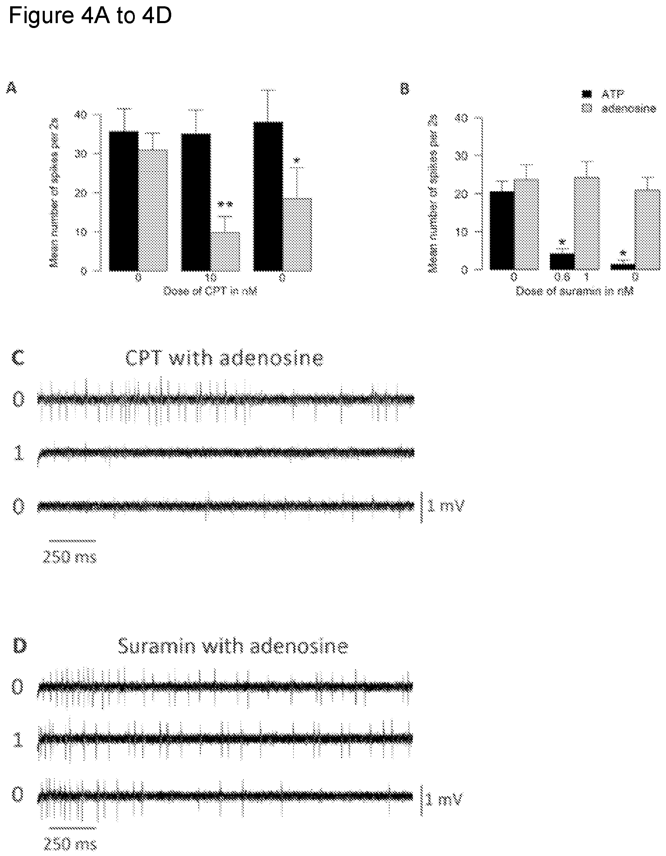

**Figures 4A** to **4D****:** The effect of CPT **(****Figure 4A****)** and suramin **(****Figure 4B****)** on mean (± s.e.m.) action potential frequencies recorded from *A. gambiae* labellum sensilla in response to adenosine and ATP each at 200 pM. The test sequence in Figures 4A and 4B was agonist, agonist admixed with antagonist, followed by agonist, presented to the sensillum at approximately 2 min intervals; doses of CPT and suramin are indicated. Antagonism lasted for a least 2 min (final response to an agonist compared to the initial response). Typical 2s spike trains from such recording sequences where 10 nM CPT **(****Figure 4C****)** and 1 nM suramin **(****Figure 4D****)** were admixed with adenosine. * P <0.05, **P <0.001 indicate significant reductions in spike frequency. Treatment repetitions (n) are reported Materials and in Methods.

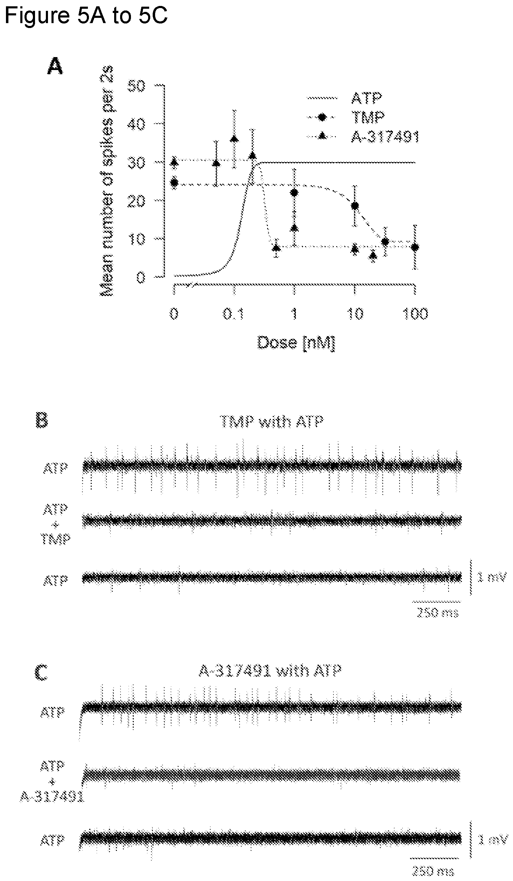

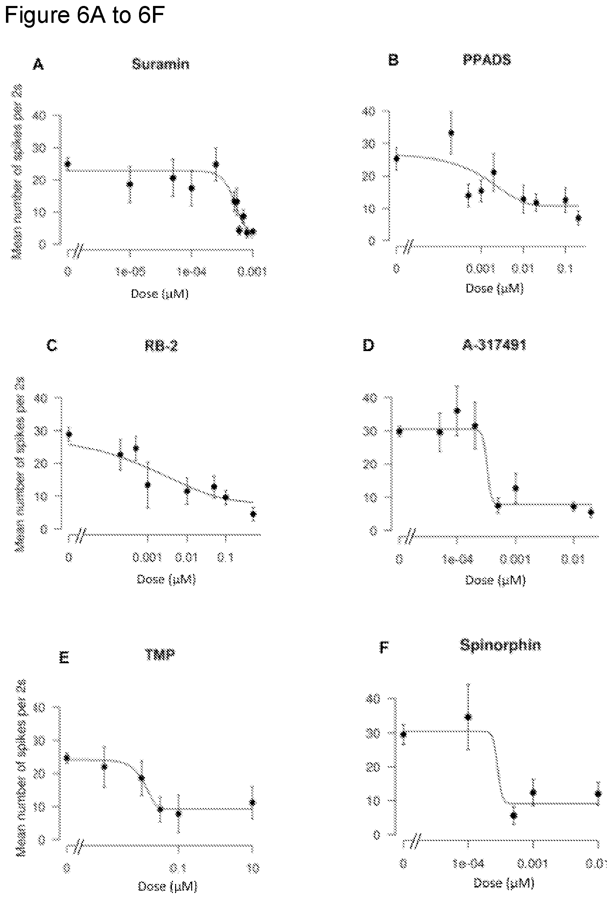

**Figure 5A****:** Number of spikes (mean ± s.e.m.) per 2s recorded from *A. gambiae* labellum sensilla in response to increasing doses of ATP (solid line; data as in Figure 2A), and to increasing doses of TMP (black dots) and A-317491 (triangles) admixed with 200 pM ATP (positive control, 0 on abscissa). Doses up to 100 nM TMP are presented for purposes of clarity, for full dose range of TMP tested see Figure 6E.
**Figures 5B** and **5C****:** Two second recordings obtained with 10 nM doses of TMP and A-317491 admixed with 200 pM ATP following the sequence ATP (top trace), ATP + antagonist and ATP, presented at approximately 2 min intervals to the sensillum. Antagonism lasted at least 2 min (spike count for the final response to ATP compared to the initial response). Treatment repetitions (n) are reported in Materials and Methods.
**Figures 6A** to **6F****:** Number of spikes (mean ± s.e.m.) per 2s recorded from *A. gambiae* labellum sensilla presented with 200 pM ATP (positive control, 0 on abscissa) and to increasing doses of the P2 PR antagonists suramin **(****Figure 6A****)** and PPADS **(****Figure 6B****),** the P2X₂ PR antagonist RB-2 **(****Figure 6C****),** the P2X₃ / P2X_{2/3} PR antagonist A-317491 **(****Figure 6D****),** and the P2X₃ PR antagonists TMP **(****Figure 6E****)** and spinorphin **(****Figure 6F****),** each dose admixed with 200 pM ATP. Treatment repetitions (n) are reported in Materials and Methods.

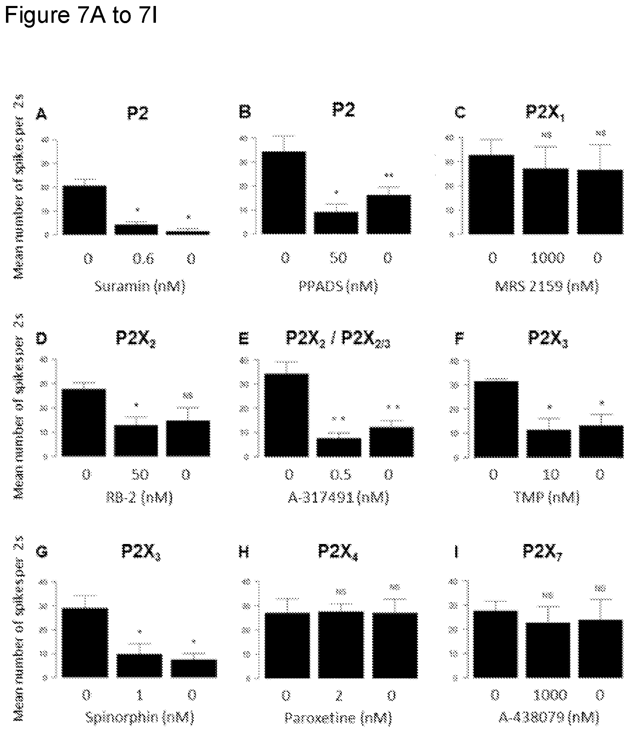

**Figure 7A** to **7I****:** Number of spikes (mean ± s.e.m.) per 2s recorded from *A. gambiae* labellum sensilla presented in sequence with 200 pM ATP, the indicated nM dose of a PR antagonist admixed with 200 pM ATP, and to 200 pM ATP: suramin **(****Figure 7A****),** PPADS **(****Figure 7B****),** MRS 2159 **(****Figure 7C****),** RB-2 **(****Figure 7D****),** A-317491 **(****Figure 7E****),** TMP **(****Figure 7F****),** spinorphin **(****Figure 7G****),** paroxetine **(****Figure 7H****)** and A-438079 **(****Figure 7I****).** Treatments were presented to sensilla at approximately 2min intervals. The dose of antagonist used approximates its estimated ID₉₅ value, whereas the dose of a substance failing to inhibit is the maximum dose tested (see Table 4). * P <0.05, ** P <0.01 compared to the initial response to ATP, NS not significant. Treatment repetitions (n) are reported in Materials and Methods.

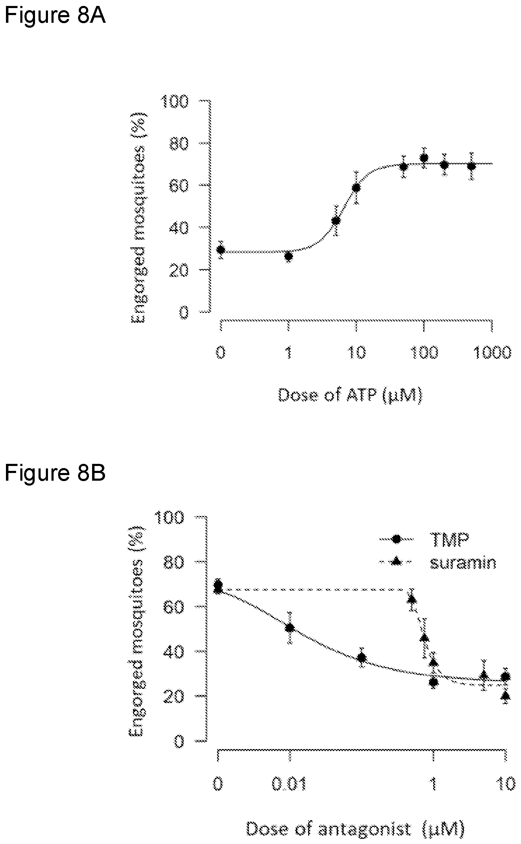

**Figure 8A** Mean percentage (± s.e.m.) *A. gambiae* feeding on increasing doses of ATP in the feeding solution.
**Figure 8B****:** Percentage *A. gambiae* feeding on the feeding solution containing 20 µM ATP (positive control, 0 on abscissa) with increasing doses of TMP (black dots) and suramin (triangles) added. Treatment repetitions (n) are reported in Materials and Methods.

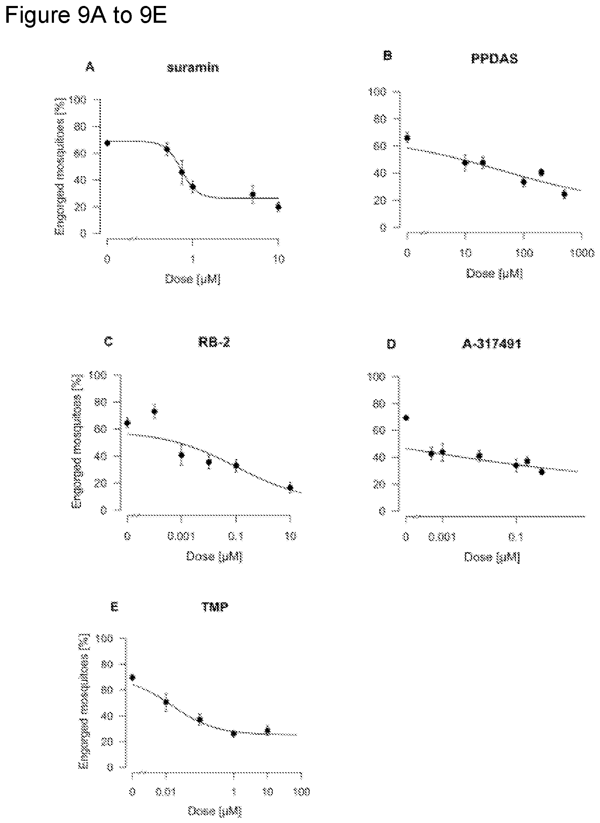

**Figure 9A** to **9E****:** Mean proportion (± s.e.m.) of *A. gambiae* feeding on a solution containing 20 µM ATP (positive control, 0 on abscissae) and on the same solution containing 20 µM ATP with increasing doses added of the P2 PR antagonists suramin **(****Figure 9A****)** and PPADS **(****Figure 9B****),** the P2X₂ PR antagonist RB-2 **(****Figure 9C****),** the P2X₃, P2X_{2/3} PR antagonist A-317491 **(****Figure 9D****),** and the P2X₃ PR antagonist TMP **(****Figure 9E****).** Treatment repetitions (n) are reported in Materials and Methods.

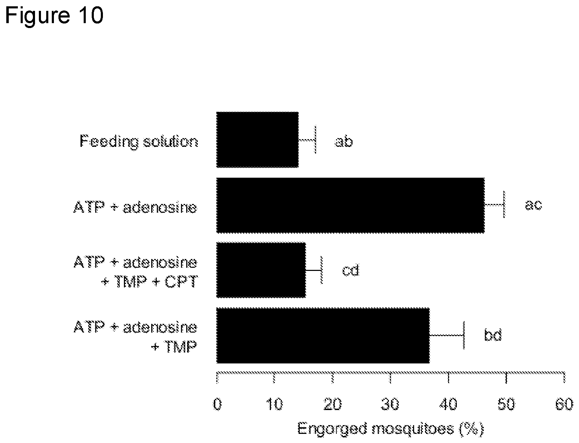

**Figure 10****:** Percentage *A. gambiae* feeding on the feeding solution (buffered NaCl/dextrose), on 20 µM ATP + 200 µM adenosine as feeding stimulant in the feeding solution, on the P1A₁ PR antagonist CPT at 20 µM + the P2X₃ PR antagonist TMP at 7.5 µM + the feeding stimulant in the feeding solution, and on TMP alone at 7.5 µM + the feeding stimulant in the feeding solution. Bars accompanied by same lower-case letter indicate a significant difference. Treatment repetitions (n) and exact P values are reported on Table 6.

### Detailed Description and embodiments of the present invention

### Basic embodiments

The present invention comprises a means to inhibit feeding by an arthropod parasite using purinergic-receptor antagonists identified for at least one P1, P2X₂, P2X₃ or P2X_{2/3} PR of said organism using the means described in the invention. The present invention also comprises methods for selecting compounds that antagonize P1, P2X₂, P2X₃, P2X_{2/3} PRs of an arthropod parasite for the purpose of using such antagonists to inhibit feeding by an arthropod parasite on or in a host animal.

A suitable PR antagonist is for example Suramin. Suramin is a symmetric molecule of formula 1: 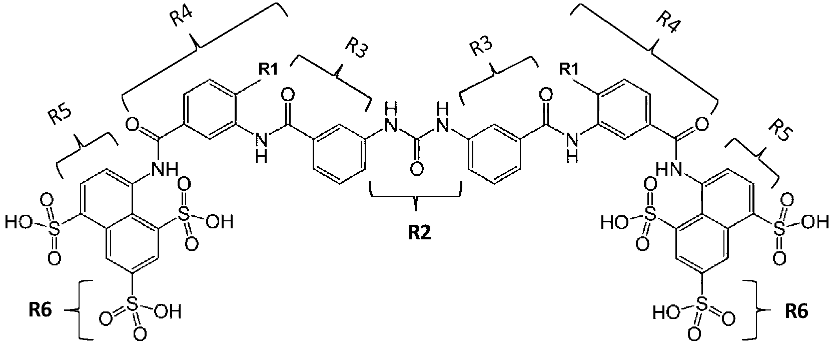 wherein R1 is -methyl in Suramin, and wherein the methyl group may be replaced in suramin derivatives which may be used as PR antagonists according to this invention, by moieties selected from a group consisting of - H, an ethyl group, an isopropyl group, a phenyl group, -F, -Cl, a methoxy group, or -CH₂OCH₃.

Derivatives of suramin, which may be used as PR antagonists according to this invention, include compounds of formula 1 wherein the carbamide moiety forming R2 is replaced by methylene, carbonyl, or carbonate.

Derivatives of suramin, which may be used as PR antagonists according to this invention also include compounds of formula 1 wherein benzene ring with amide linkers forming R3 is replaced by benzyl, benzofuran, and indole.

Derivatives of suramin, which may be used as PR antagonists according to this invention further include compounds of formula 1 wherein the amidotoluene forming R4 is replaced by thiazolidinone, thiazinanone, or thiobenzamide.

Derivatives of suramin, which may be used as PR antagonists according to this invention further include compounds of formula 1 wherein the naphthalene rings forming R5 are replaced by benzene rings, cyclohexane rings, or 1,3,4-triazine rings.

Derivatives of suramin, which may be used as PR antagonists according to this invention further include compounds of formula 1 wherein sulfonate group as in R6 is replaced by a methyl group, a methoxy group, or an amino group.

Suramin-derived P2X2, P2X3 and P2X2/3 PR antagonist also comprise the following compounds: 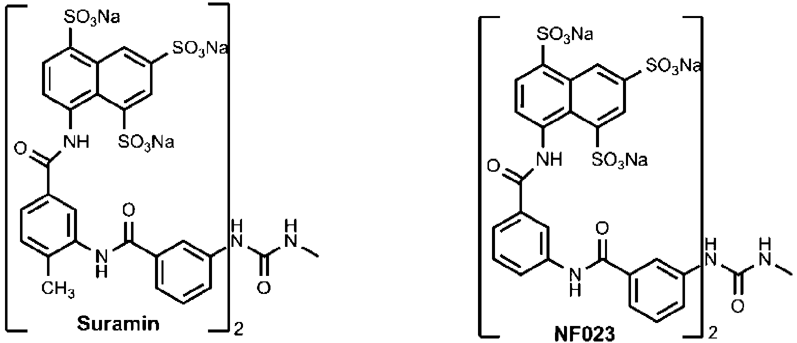 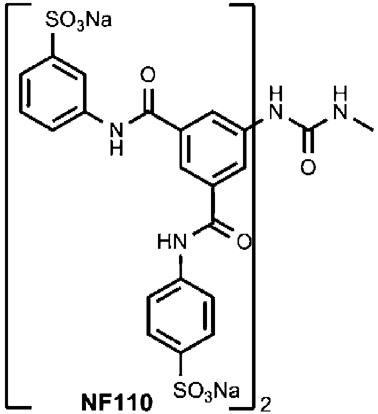 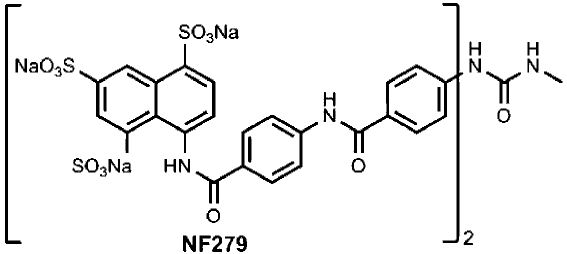 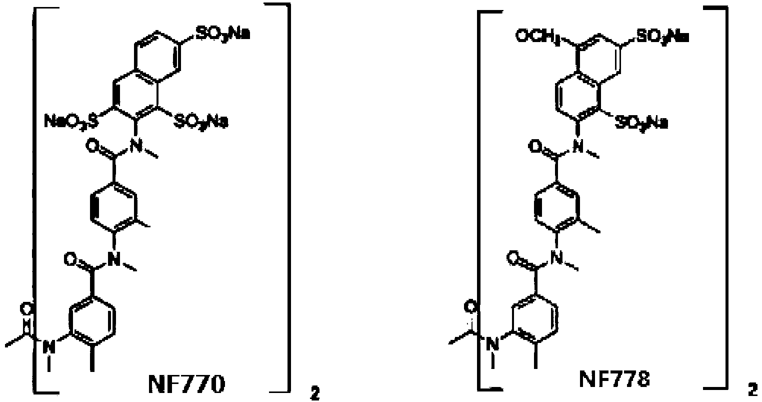 PR antagonists may also be small molecule inhibitors. P2PX2 PR antagonists may be selected from compounds comprising an anthraqinone moiety of the formula: 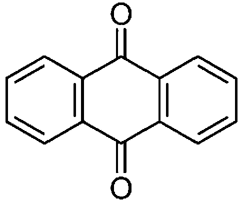 such as compound RB-2 having the formula: 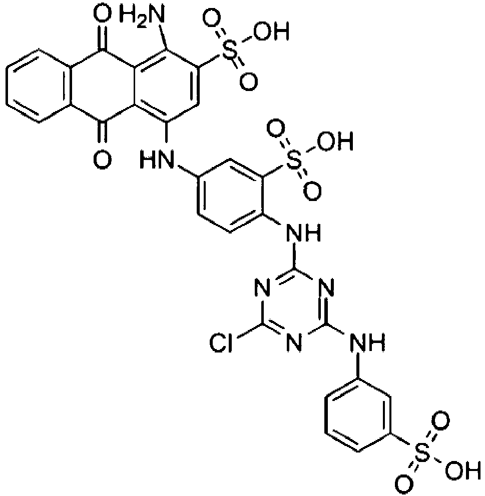 P2PX2 PR antagonists may also be selected from compounds comprising a pyridoxal phosphate moiety of the formula: 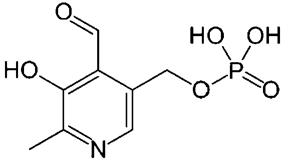 such as PPADS having the following formula:

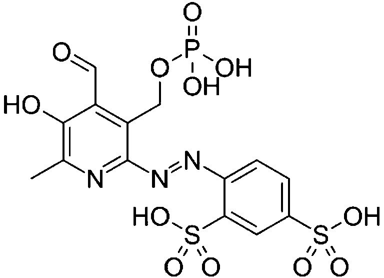

P2PX₃ and P2X_{2/3} PR antagonists may also be selected from the following compounds: A317491: 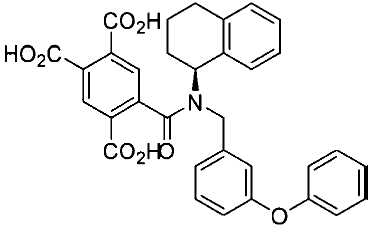 AF-906: 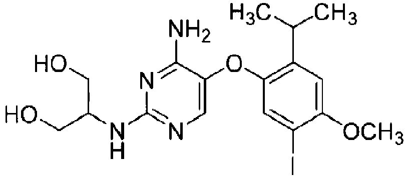 BLU-5937: 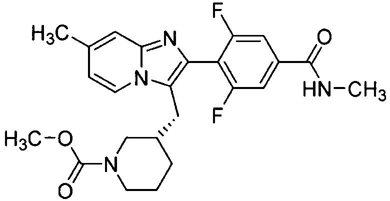 S-600918:

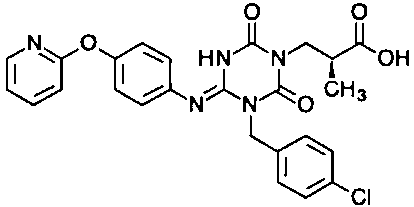

The present invention also concerns assay methods for selecting compounds capable of modulating at least one P1, P2X₂, P2X₃ or P2X_{2/3} PR of an arthropod parasite, in particular a blood-feeding arthropod parasite. Such assay methods can be physiological and/or biochemical assays and/or behavioural assays. Examples of behavioural assays are described herein in Material and Methods and in Examples, but suitable assays are not limited to the examples provided in Material and Methods and in Examples herein. Assay methods can be used to select compounds for their ability to modulate a P1, P2X₂, P2X₃ or P2X_{2/3} PR of an arthropod parasite from available libraries containing hundreds of thousands of molecules or substances.

In one embodiment of this invention a product comprising at least one PR antagonist is directed against arthropod parasites of a vertebrate host, preferably warm-blooded vertebrate host.

In another embodiment, the product is directed against parasites of an invertebrate host, such as an insect, for example a bee.

In an embodiment the product is directed against arthropod parasites. The product may be directed against arthropod endoparasites such as mange mites, botfly larvae, myisasis fly larvae, and/or against arthropod ectoparasites such as blood-feeding micro-predators, mites and ticks.

In an embodiment the product is directed against arthropod biofouling, such as against barnacles.

In an embodiment at least one P1 PR of an arthropod parasite, preferably a blood-feeding arthropod parasite, is modulated by a P1 PR antagonist or by a combination of P1 PR antagonists;

In an embodiment at least one P1 A₁ PR of an arthropod parasite, preferably a blood-feeding arthropod parasite, is modulated by a P1 A₁ PR antagonist or by a combination of P1 A₁ PR antagonists;

In an embodiment at least one P2X₂ PR of an arthropod parasite, preferably a blood-feeding arthropod parasite, is modulated by a P2X₂ PR antagonist or by a combination of P2X₂ PR antagonists.

In an embodiment at least one P2X₃ PR of an arthropod parasite, preferably a blood-feeding arthropod parasite, is modulated by a P2X₃ PR antagonist or by a combination of P2X₃ PR antagonists;

In an embodiment at least one P2X_{2/3} PR of an arthropod parasite, preferably a blood-feeding arthropod parasite, is modulated by a P2X_{2/3} PR antagonist or by a combination of P2X_{2/3} PR antagonists;

In an embodiment at least one P1, P2X₂, P2X₃ or P2X_{2/3} PR of an arthropod parasite, preferably a blood-feeding arthropod parasite, is modulated by a combination of P1, P2X₂, P2X3₃, P2X_{2/3} PR antagonists;

In an embodiment the invention comprises modulation of any combination of P1, P2X₂, P2X₃ and P2X_{2/3} PRs of an arthropod parasite, preferably a blood-feeding arthropod parasite, by a combination of P1, P2X₂, P2X₃ and P2X_{2/3} PR antagonists;

### Methods for selecting PR antagonists

The present invention comprises a method for selecting an antagonist for a P1, P2X₂, P2X₃ or P2X_{2/3} PR of an arthropod parasite by making, for example, electrophysiological recordings from a cell expressing at last one of said PRs. This is achieved when a test compound is presented in combination with an agonist such as adenosine or ATP and brought into contact in solution with the PR. A PR antagonist causes a decrease, for example, in action potential frequency recorded from the cell compared to the action potential frequency recorded from the same cell when an agonist such as adenosine or ATP is presented alone as positive control.

In an embodiment, the invention comprises an electrophysiological method for selecting an antagonist for a purine-sensitive cell of an arthropod parasite expressing at least one P1, P2X₂, P2X₃ or P2X_{2/3} PR. In a preferred embodiment the test compound is presented in solution to contact the cell at increasing doses over the 1 pM to 100 nM range in combination with a fixed dose of an agonist such as adenosine or ATP. Alternatively, 1 pM to 100 µM dose range for the test compound can be chosen for testing. Inhibition induced by an antagonist occurs within seconds as measured by the reduction in the number of action potentials compared to the number of action potentials recorded for adenosine or ATP presented alone as positive control. This is shown herein in Examples on Figure 4 A and C for the P1 antagonist 8-cyclopentyl-1,3-dimethylxanthine (CPT), and on Figure 5 for the P2X₃ PR antagonist 2,3,5,6-tetramethylpyrazine (TMP) and for the P2X₃ / P2X_{2/3} PR antagonist A-317491. This permits calculation of a dose-response function and ID₅₀ value for a PR antagonist which can be in the 300 pM to 15 nM range as shown herein in Examples on Figures 5 and 6, and on Table 4 for the P2 PR antagonists suramin and PPADS, for the P2X₂ PR antagonist RB-2, for the P2X₃ PR antagonists TMP and spinorphin, and for the P2X₃ / P2X_{2/3} PR antagonist A-317491. Nevertheless, an ID₅₀ value of an antagonist for a P1, P2X₂, P2X₃ or P2X_{2/3} PR of an arthropod parasite is not limited to a particular molar range.

The present invention comprises a method for selecting an inverse agonist for a P1, P2X₂, P2X P2X₃ or P2X_{2/3} PR of an arthropod parasite by making, for example, electrophysiological recordings from a cell expressing at last one of said PRs. This is achieved when a test compound is brought into contact in solution with the PR. An inverse agonist causes, for example, a decrease in action potential frequency recorded from the cell compared to the action potential frequency recorded from the same cell when the inverse agonist is absent from the solution.

### Use of transgenic animals

For the selection of suitable PR antagonists it may be advantageous to use transgenic animals.

In an embodiment the present invention provides for a method for selecting an antagonist for a P1, P2X₂, P2X₃ or P2X_{2/3} PR of an arthropod parasite by making, for example, recording from a cell, organ or tissue of a transgenic arthropod parasite containing an altered allele for a reporter gene that encodes and expresses for example a fluorescent protein in addition to at least one functional P1, P2X₂, P2X₃ or P2X_{2/3} PR. Presence of the encoded fluorescent protein permits recording of Ca⁺⁺ transients in the cell, organ or tissue. Selection of a PR antagonist can be achieved when a test compound is presented in combination with an agonist such as adenosine or ATP and brought into contact in solution with the cell, organ or tissue of the transgenic arthropod parasite. A PR antagonist causes, for example, a decrease in cellular activity as measured by Ca⁺⁺ transients recorded from said cell, organ or tissue compared to Ca⁺⁺ transients recorded from the same cell, organ or tissue when adenosine or ATP is presented alone as positive control. In this case, modulation by the PR antagonist comprises inhibition of functional PR activity in the transgenic arthropod parasite's cell, organ or tissue.

In an embodiment the present invention provides for a method for selecting an antagonist for a P1, P2X₂, P2X₃ or P2X_{2/3} PR of an arthropod parasite by making, for example, electrophysiological recording from a cell of a non-parasitic transgenic animal containing an altered allele for a gene that naturally encodes and expresses at least one functional P1, P2X₂, P2X₃ or P2X_{2/3} PR of an arthropod parasite. This is achieved when a test compound is presented in combination with an agonist such as adenosine or ATP and brought into contact in solution with the PR. A PR antagonist causes, for example, a decrease in action potential frequency recorded from said cell compared to the action potential frequency recorded from the same cell when adenosine or ATP is presented alone as positive control. In the present case, modulation comprises inhibition of the functional PR of the non-parasitic transgenic animal.

In an embodiment the present invention provides for a method for selecting an antagonist for a P1, P2X₂, P2X₃ or P2X_{2/3} PR of an arthropod parasite by making, for example, electrophysiological recordings from a cell of a non-parasitic transgenic animal containing an altered allele for a gene that naturally encodes and expresses at least one functional P1, P2X₂, P2X₃ or P2X_{2/3} PR of a non-parasitic organism. It is understood that the functional P1, P2X₂, P2X₃ or P2X_{2/3} PR of the non-parasitic organism responds to an agonist of at least one P1, P2X₂, P2X₃ or P2X_{2/3} PR of the arthropod parasite. Selection of an antagonist is achieved when a test compound is presented in combination with an agonist such as adenosine or ATP and brought into contact in solution with the PR. A PR antagonist causes, for example, a decrease in action potential frequency recorded from said cell compared to the action potential frequency recorded from the same cell when adenosine or ATP is presented alone as positive control. In the present case, modulation comprises inhibition of the functional PR of the transgenic animal.

In one embodiment the selection method is performed using a transgenic animal containing an altered allele for the gene that naturally encodes and expresses a functional P1, P2X₂, P2X₃ or P2X_{2/3} PR of a non-parasitic organism such as humans or a rat;

In an embodiment the selection method is performed using a transgenic animal containing an altered allele for the gene that naturally encodes and expresses a functional P1, P2X₂, P2X₃ or P2X_{2/3} PR of an arthropod parasite such as a biting insect or a tick;

In an embodiment the selection method is performed using a transgenic animal possessing an altered allele for a functional P1, P2X₂, P2X₃ or P2X_{2/3} PR with a phenotype that shows enhanced sensitivity to adenosine and/or ATP as a feeding stimulant compared to the wild-type animal. It is understood that such as transgenic animal is a non-parasitic one;

In an embodiment the selection method is performed using a transgenic animal possessing an altered allele for a non-functional P1, P2X₂, P2X₃ or P2X_{2/3} PR that renders it less sensitive to adenosine or ATP as a feeding stimulant. Such a transgenic animal is a knockout (KO) transgenic animal. Further, such a KO transgenic animal can be either an arthropod parasite or a non-parasitic animal.

### Use of heterologous system to select PR antagonists

It may furthermore be useful to base a selection method for a PR antagonist on a heterologous system.

In an embodiment the selection method is performed using a heterologous cell system, using host cells of vertebrate or invertebrate origin, containing an allele for the gene that naturally encodes and expresses at least one functional P1, P2X₂, P2X₃ or P2X_{2/3} PR of an arthropod parasite for the purpose of selecting antagonists for said PRs. The host cell in such a system expresses a nucleic acid sequence or sequences that is not part of its own genome. Host cells can have a variety of origins, for example being human in origin such as human HEK cells, or in another example being amphibian in origin such as the *Xenopus* oocyte. In such a system a functional PR is constituted when polypeptide subunits synthesized by the host cell combine in the cell membrane to form a functional PR. The PR is activated by a nucleoside such as adenosine or a nucleotide such as ATP in solution through ion flux, and/or ion channel activation and/or induction of secondary messengers. Such activation by an agonist can be used to select antagonists for P1, P2X₂, P2X₃, P2X_{2/3} PRs of an arthropod parasite through changes brought about by the antagonist in solution such as changes in ion flux when the antagonist contacts the functional PR. Such changes in ion flux can be quantified by a variety of techniques well known in the art as indicative of cellular activity, for example by measurement of changes of cell membrane potential, measurement of cell membrane currents, using radiolabelled ion flux assays, using fluorescence assays for voltage sensitive dyes, and by patch clamp electrophysiological techniques. In the case of P1 PRs such measurements that are indicative of cellular activity can also include quantification of secondary messengers such as adenylate cyclase and inositol 1,4,5-triphosphate (IP₃). Automation of such measurements permits high-throughput screening of compound libraries using said heterologous systems to select antagonists of said PRs. Alternatively, the ability of cells expressing said functional PRs of an arthropod parasite to grow in a feeding medium in presence of a test compound can be used to select antagonists for P1, P2X₂, P2X₃, P2X_{2/3} PRs of an arthropod parasite.

In an embodiment the selection method is performed using a heterologous cell system for the purpose of selecting antagonists for P1, P2X₂, P2X₃, P2X_{2/3} PRs of an arthropod parasite where host cells express a functional P1, P2X₂, P2X₃ or P2X_{2/3} PR or any combination of said PRs belonging to any organism within the Protozoa or belonging to any invertebrate or vertebrate organism. It is understood that the functional P1, P2X₂, P2X₃ or P2X_{2/3} PR or said PR combination expressed in the heterologous cell system responds to an agonist of at least one P1, P2X₂, P2X₃ or P2X_{2/3} PR of the arthropod parasite. Human astrocytoma cells expressing human and rat P2X₃ and P2X_{2/3} PRs permitted identification of A-317491 as a non-nucleotide antagonist of Ca⁺⁺ flux by ATP-gated P2X₃ and P2X_{2/3} PRs of humans and the rat as documented by Jarvis *et al.,* 2002 in "A-317491, a novel potent and selective non-nucleotide antagonist of P2X₃ and P2X_{2/3} PRs, reduces chronic inflammatory and neuropathic pain in the rat" in *PNAS* 99(26), doi:10.1073/pnas.252537299.

As described herein on Figures 5, 6 and 7 and Table 4 of Examples, A-317491 inhibits the malaria mosquito PR at an ID₅₀ of 320 pM in the electrophysiological assay and, as described herein on Figure 9 and Table 7 of Examples, A-317491 inhibits feeding by the malaria mosquito at an ID₅₀ of 240 pM. Furthermore, as described herein on Tables 8, 9 and 10 of Examples, A-317491 inhibits feeding by the Asian mosquito vector of malaria, by biting flies and by ticks.

### Use of ex vivo and in vivo systems to select PR antagonists

It is also possible, to perform the method of selecting PR antagonists using an *ex vivo* or an *in vivo* system.

In an embodiment the method of selecting antagonists for P1, P2X₂, P2X₃, P2X_{2/3} PRs of an arthropod parasite is performed employing cell lines, organoids and tissue originating from arthropod parasites or originating from any organism within the Protozoa or from any invertebrate or vertebrate organism that expresses native P1, P2X₂, P2X₃ and P2X_{2/3} PRs or expresses any combination of said PRs. Alternatively, transformed cell lines, organoids and tissue from said organisms expressing P1, P2X₂, P2X₃ and P2X_{2/3} PRs may be used to select antagonists for P1, P2X₂, P2X₃, P2X_{2/3} PRs of an arthropod parasite. The ability of cells expressing said native PRs or of said transformed cells expressing PRs to grow in a feeding medium in presence of a test compound can be used to select antagonists for P1, P2X₂, P2X₃, P2X_{2/3} PRs of an arthropod parasite. Notably, sensory cells of dorsal root ganglia and nodose ganglia of vertebrate animals express P1 PRs that respond to adenosine and P2X₂, P2X₃ and P2X_{2/3} PRs that respond to ATP and accordingly provide an appropriate medium in which to select antagonists for P1, P2X₂, P2X₃, P2X_{2/3} PRs of an arthropod parasite.

In an embodiment the present invention provides for a method for selecting an antagonist for a P1, P2X₂, P2X₃ or P2X_{2/3} PR of an arthropod parasite by making, for example, recording *ex vivo* from a cell, organ or tissue of a transgenic arthropod parasite containing an altered allele for a gene that encodes and expresses for example a fluorescent protein in addition to at least one functional P1, P2X₂, P2X₃ or P2X_{2/3} PR. Examples of an arthropod-parasite organ could be the labellum of a mosquito or the hypostome of a tick. Presence of the encoded fluorescent protein in the arthropod parasite permits recording of Ca⁺⁺ transients in the cell, organ or tissue indicative of activation of said PRs in presence of an agonist such as adenosine or ATP. Selection of a PR antagonist can be achieved when a test compound is presented in combination with an agonist such as adenosine or ATP and brought into contact in solution with the cell, organ or tissue of the transgenic arthropod parasite. A PR antagonist causes, for example, a decrease in cellular activity as measured by Ca⁺⁺ transients recorded from said cell, organ or tissue compared to the Ca⁺⁺ transient activity recorded from the same cell, organ or tissue when adenosine or ATP is presented alone as positive control. In this case, modulation by the PR antagonist comprises inhibition of functional PR activity in the transgenic arthropod parasite's cell, organ or tissue.

In an embodiment the selection method is performed by selecting antagonists for P1, P2X₂, P2X₃, P2X_{2/3} PRs of an arthropod parasite by using for example electrophysiological recordings of action potentials, recording cell membrane potentials, measurement of cell membrane currents, use of ion flux assays, use of fluorescence assays for voltage sensitive dyes and fluorescent proteins, patch clamp electrophysiological recording techniques or cell imaging techniques that measure cellular activity of any cell, tissue or organ of any organism within the Protozoa or of any invertebrate, including arthropod parasites, or of any vertebrate organism expressing a P1, P2X₂, P2X₃ or P2X_{2/3} PR or expressing any combination of said PRs that responds to an agonist of at least one P1, P2X₂, P2X₃ or P2X_{2/3} PR of the arthropod parasite.

### Methods of selecting feeding inhibitors

In an embodiment the present invention provides for a method of selecting antagonists of P1, P2X₂, P2X₃, P2X_{2/3} PRs that inhibit feeding by an arthropod parasite through inhibition of at least one of its P1, P2X₂, P2X₃, P2X_{2/3} PRs.

In an embodiment the present invention provides for selecting a compound that inhibits feeding by an arthropod parasite possessing at least one P1, P2X₂, P2X₃ or P2X_{2/3} PR. This is achieved by measuring feeding by the arthropod parasite on a feeding medium to which a test compound plus a feeding stimulant such as adenosine or ATP is added in solution. A compound that inhibits feeding causes a reduction in feeding by the arthropod parasite compared to its feeding, for example, on the positive control where only the feeding stimulant is added to the feeding medium. In this case, modulation comprises inhibition by the antagonist of the PR that interferes with the ability of the arthropod parasite to sense the feeding stimulant in the feeding medium.

In an embodiment, a compound that inhibits feeding can be selected using an arthropod parasite expressing at least one P1, P2X₂, P2X₃ or P2X_{2/3} PR. In a preferred embodiment the test compound is presented in solution to the arthropod parasite at increasing doses over the 10 nM to 10 µM range as shown herein on Figures 8 B of Examples for TMP and suramin when added to a solution containing a fixed dose of ATP. Alternatively, a 1 pM to 1 mM dose range of a test compound can be chosen for testing. Inhibition of feeding induced by a test compound is measured within a period of a few minutes to over an hour, depending on the arthropod parasite in question, and is expressed, for example, relative to the number of the arthropod parasites feeding on the positive control where only the feeding stimulant is added to the feeding medium. This permits calculation of a dose-response function and ID₅₀ value for a feeding inhibitor which can be in the 200 pM to 800 nM range as shown herein on Figures 8 and 9 and on Table 7 of Examples for the P2 PR antagonists suramin and PPADS, for the P2X₂ PR antagonist RB-2, for the P2X₃ PR antagonists TMP and spinorphin, and for the P2X₃ / P2X_{2/3} PR antagonist A-317491. Nevertheless, the ID₅₀ value for a compound that inhibits feeding by an arthropod parasite is not limited to a particular molar range.

An embodiment of the present invention comprises selecting a compound that inhibits feeding by an arthropod parasite possessing at least one P1, P2X₂, P2X₃ or P2X_{2/3} PR where a test compound is presented on or anywhere around a substrate such as a membrane or surface of a reservoir held at a temperature at or near 37°C on which the arthropod parasite has the ability to feed on a feeding medium. This is achieved by measuring feeding by the arthropod parasite on the feeding medium to which a feeding stimulant such as adenosine or ATP is added in solution and where the test compound is present on the substrate over the feeding medium. Inhibition of feeding induced by a test compound is measured within a period of a few minutes to over an hour, depending on the arthropod parasite in question. A compound that inhibits feeding causes a reduction in feeding by the arthropod parasite compared to its feeding on the positive control where, for example, an untreated membrane is placed over the feeding medium incorporating the feeding stimulant. This is shown herein on Tables 10 and 11 of Examples where the P2X3 antagonist TMP applied onto the feeding membrane inhibits feeding by ticks and biting flies. In this case, modulation comprises inhibition by the antagonist of the PR that interferes with the arthropod parasite's ability of the feed through the membrane. Feeding rates of the arthropod in the presence and in the absence of at least one test compound are compared. The comparison allows for selection of compounds which are suitable feeding inhibitors.

An embodiment of the present invention comprises selecting a compound that inhibits feeding by an arthropod parasite possessing at least one P1, P2X₂, P2X₃ or P2X_{2/3} PR where a test compound is presented on or anywhere around a substrate or warm body held at a temperature at or near 37°C. Once an arthropod parasite, such as a mosquito, has contacted said substrate or warm body the arthropod parasite starts probing the surface with its mouthparts leading to biting attempts. Such probing and biting activity on said substrate or warm body is typical of mosquito behaviour once it has landed on a host animal. This biting behaviour induced on a warm body has been exploited to test repellents for arthropod parasites as described in Kröber et al., 2010, "An in vitro assay for testing mosquito repellents employing a warm body and carbon dioxide as a behavioral activator", Journal of the American Mosquito Control Association 26, 381-386; doi.org/10.2987/10-6044.1. Selection of a compound that inhibits feeding is made by measuring the biting rate by the arthropod parasite on the warm body onto which the test compound is applied. Inhibition of the biting rate induced by the test compound is measured within a period of a few minutes to over an hour, depending on the arthropod parasite in question. A compound that inhibits feeding causes a reduction in the biting rate by the arthropod parasite compared to its biting rate on the positive control where, for example, an untreated warm body is presented. In this case, modulation comprises inhibition by the antagonist of the PR that interferes with the arthropod parasite's ability of bite. Biting rates of the arthropod in the presence and in the absence of at least one test compound are compared. The comparison allows for selection of compounds which are suitable feeding inhibitors.

The present invention comprises a method for selecting an antagonist for a P1, P2X₂, P2X₃ or P2X_{2/3} PR of an arthropod parasite by recording, for example, a feeding electrogram from said parasite expressing at last one of said PRs. The feeding electrogram technique is used to measure the feeding rate by the arthropod parasite on a feeding medium to which a test compound plus a feeding stimulant such as adenosine or ATP is added in solution. The feeding electrogram permits recording of changes in potential, measured in mV, across the mouthparts and action potential frequencies generated by cells of the arthropod parasite's mouthparts. A regular feeding rate is sustained, for example, by sucking bouts induced by activation of pharyngeal muscles, and salivation induced by closing of the pre-pharyngeal valve accompanied by lowering of the salivarium floor. Use of the feeding electrogram to measure the feeding rate of an arthropod parasite is described in Lösel et al., 1992, "Feeding electrogram studies on the African cattle brown ear tick Rhipicephalus appendiculatus: evidence for an antifeeding effect of tick resistant serum", Physiological Entomology 17, 342-350; doi.org/10.1111/i.1365-3032.1992.tb01032.x. The arthropod parasite can feed on said medium with the test compound plus the feeding stimulant added either across a membrane, or directly on said medium with the test compound and the feeding stimulant added that is held, for example, in a micro reservoir such as a glass capillary. Inhibition of feeding induced by the test compound is measured within a period of a few minutes to over an hour, depending on the arthropod parasite in question. Both the membrane-feeding and glass-capillary feeding methods are described herein in Materials and Methods. Feeding rates measured from the arthropod parasite in the presence and in the absence of at least one test compound are compared to detect, for example, a decrease in the frequency of sucking events caused by the test compound. The comparison allows for selection of compounds which are suitable feeding inhibitors. Sucking and salivation events that indicate the feeding rate of an arthropod parasite can also be measured by macro-video photography as described in Lösel et al., 1992, *ibid.*

An embodiment of the present invention comprises selecting a compound that inhibits feeding by an arthropod parasite through use of a non-parasitic transgenic animal containing cells with an altered allele for a gene that naturally encode and express at least one functional P1, P2X₂, P2X₃ or P2X_{2/3} PR of an arthropod parasite. This is achieved when feeding by the transgenic animal is lower on a feeding medium to which a feeding stimulant such as adenosine or ATP plus a test compound is added in solution than feeding by the same transgenic animal on the feeding medium that serves as positive control to which only the feeding stimulant is added. In this case, modulation comprises inhibition by the feeding inhibitor of the functional PR that interferes with the ability of the transgenic animal to sense the feeding stimulant in the feeding medium.

In an embodiment the present invention provides for selecting a compound that inhibits feeding by an arthropod parasite using of a non-parasitic transgenic animal possessing an altered allele for a gene that naturally encodes and expresses at least one functional P1, P2X₂, P2X₃ or P2X_{2/3} PR of a non-parasitic organism. It is understood that the functional P1, P2X₂, P2X₃ or P2X_{2/3} PR of the non-parasitic organism responds to an agonist of at least one P1, P2X₂, P2X₃ or P2X_{2/3} PR of the arthropod parasite. Selection of an antagonist is achieved when feeding by the transgenic animal is lower on a feeding medium to which a feeding stimulant such as adenosine or ATP plus a test compound is added in solution than feeding by the same transgenic animal on the feeding medium that serves as positive control to which only the feeding stimulant is added. In this case, modulation comprises inhibition by the feeding inhibitor of the functional PR that interferes with the ability of the transgenic animal to sense the feeding stimulant in the feeding medium.

### Embodiments for feeding inhibitors

In an embodiment the present invention comprises an antagonist of at least one P1, P2X₂, P2X₃ or P2X_{2/3} PR of an arthropod parasite, for example a blood-feeding arthropod parasite, such as a biting insect, as a feeding inhibitor for said organism.

In an embodiment the present invention comprises an antagonist of any combination of P1, P2X₂, P2X₃ and P2X_{2/3} PRs of an arthropod parasite, for example a blood-feeding arthropod parasite, such as a biting insect, as a feeding inhibitor for said organism.

In an embodiment the present invention comprises an antagonist of at least one P1 PR of an arthropod parasite, for example a blood-feeding arthropod parasite, such as a biting insect, as a feeding inhibitor for said organism.

In an embodiment the present invention comprises an antagonist of at least one P1 A₁ PR of an arthropod parasite, for example a blood-feeding arthropod parasite, such as a biting insect, as a feeding inhibitor for said organism.

In an embodiment the present invention comprises an antagonist of at least one P2X₂ PR of an arthropod parasite, for example a blood-feeding arthropod parasite, such as a biting insect, as a feeding inhibitor for said organism.

In an embodiment the present invention comprises an antagonist of at least one P2X₃ PR of an arthropod parasite, for example a blood-feeding arthropod parasite, such as a biting insect, as a feeding inhibitor for said organism.

In an embodiment the present invention comprises an antagonist of at least one P2X_{2/3} PR of an arthropod parasite, for example a blood-feeding arthropod parasite, such as a biting insect, as a feeding inhibitor for said organism.

In an embodiment the present invention comprises an antagonist of any combination of P1, P2X₂, P2X₃ and P2X_{2/3} PRs of an arthropod parasite, for example a blood-feeding arthropod parasite, such as a biting insect, as a feeding inhibitor for said organism.

For the purposes of this invention it is understood that inhibition of P1, P2X₂, P2X₃, P2X_{2/3} PRs by an antagonist can be achieved in different ways, for example, by binding to the ion channel pore thus blocking opening of the ion channel by the agonist, by blocking the ion channel pore in the open state, by acting as a competitive agonist for binding by the agonist with the PR, or by binding to a non-agonist allosteric site on the PR to prevent its activation.

In an embodiment this invention comprises suramin (antrypol, a polysulfonated naphthylamine), PPADS (pyridoxal-phosphate-6-azophenyl-2,4-disulfonate), RB-2 (an anthraquinone-sulfonic acid), spinorphin (a heptapeptide), 2,3,5,6-tetramethylpyrazine (TMP) or A-317491 (a tricarboxylate) as an antagonist for P2X₂, P2X₃, P2X_{2/3} PRs of an arthropod parasite, for example a blood-feeding arthropod parasite, such as a biting insect, that respond to ATP or AMP-CCP as an agonist, as disclosed herein on Figures 4 B and D, 5, 6 and 7 and on Table 4 of Examples. The invention also comprises any combination of said PR antagonists and pharmacologically admissible salts of said PR antagonists for an arthropod parasite. An antagonist for P2X₂, P2X₃, P2X_{2/3} PRs of an arthropod parasite may also comprise compounds selected from P2X₂, P2X₃, P2X_{2/3} PR antagonists listed herein.

In an embodiment this invention comprises suramin, PPADS, RB-2, spinorphin, TMP or A-317491 as a feeding inhibitor for an arthropod parasite, for example a blood-feeding arthropod parasite, such as a biting insect, where said organism responds to ATP or AMP-CPP as a feeding stimulant, as disclosed herein on Figures 8 B, 9 and 10, and on Tables 7, 8, 9, and 10 of Examples. The invention also comprises any combination of said feeding inhibitors and pharmacologically admissible salts of said feeding inhibitors for an arthropod parasite. A feeding inhibitor for an arthropod parasite may also comprise compounds selected from P2X₂, P2X₃, P2X_{2/3} PR antagonists listed herein.

In an embodiment this invention comprises 1,3-dimethyxanthine (theophylline) or 8-cyclopentyl-1,3-dimethylxanthine (8-cyclopentyltheophylline or CPT) as an antagonist of a P1 PR of an arthropod parasite that respond to adenosine or AMP as an agonist, as herein disclosed on Figure 4 A and C and in the corresponding text in Examples. The invention also comprises a combination of said antagonists and pharmacologically admissible salts of said antagonists. An antagonist for P1 PRs of an arthropod parasite may also comprise compounds selected from P1 PR antagonists listed herein.

In an embodiment this invention comprises 8-cyclopentyl-1,3-dimethylxanthine as a feeding inhibitor for an arthropod parasite, for example a blood-feeding arthropod parasite, such as a biting insect, that responds to adenosine as a feeding stimulant, as herein disclosed on Table 6 of Examples. The invention also comprises a pharmacologically admissible salt of 8-cyclopentyl-1,3-dimethylxanthine as a feeding inhibitor for an arthropod parasite, for example a blood-feeding arthropod parasite, such as a biting insect. A feeding inhibitor for an arthropod parasite may also comprise compounds selected from P1 PR antagonists listed herein.

In an embodiment this invention comprises the P1A₁ PR antagonist 8-cyclopentyl-1,3-dimethylxanthine combined with the P2X₃ PR antagonist 2,3,5,6-tetramethylpyrazine as a feeding inhibitor for an arthropod parasite, for example a blood-feeding arthropod parasite, such as a biting insect, that responds to adenosine with ATP as a feeding stimulant, as herein disclosed on Figure 10 and Table 6 of Examples. A feeding inhibitor for an arthropod parasite may also comprise combinations of compounds selected from P1 PR antagonists listed herein.

In an embodiment this invention comprises combinations of PR antagonists. A combination of said PR antagonists may comprise suramin with 8-cyclopentyl-1,3-dimethylxanthine. A combination of said PR antagonists may comprise suramin with 1,3-dimethylxanthine. A combination of said PR antagonists may comprise suramin with 2,3,5,6-tetramethylpyrazine. A combination of said PR antagonists may comprise suramin with a tricarboxylate such as A-317491. A combination of said PR antagonists may comprise suramin with spinorphin. A combination of said PR antagonists may comprise PPADS with 8-cyclopentyl-1,3-dimethylxanthine. A combination of said PR antagonists may comprise PPADS with 1,3-dimehtylxanthine. A combination of said PR antagonists may comprise RB-2 with 8-cyclopentyl-1,3-dimethylxanthine. A combination of said PR antagonists may comprise RB-2 with 1,3-dimehtylxanthine. A combination of said PR antagonists may comprise 2,3,5,6-tetramethylpyrazine with 1,3-dimehtylxanthine. A combination of said PR antagonists may comprise A317491 with 8-cyclopentyl-1,3-dimethylxanthine. A combination of said PR antagonists may comprise A317491 with 1,3-dimehtylxanthine. A combination of said PR antagonists may comprise spinorphin with 8-cyclopentyl-1,3-dimehtylxanthine. A combination of said PR antagonists may comprise spinorphin with 1,3-dimehtylxanthine. A combination of said PR antagonists may comprise RB-2 with 2,3,5,6-tetramethylpyrazine. A combination of said PR antagonists may comprise s RB-2 with A317491. A combination of said PR antagonists may comprise RB-2 with spinorphin. A combination of said PR antagonists may comprise PPADS with 2,3,5,6-tetramethylpyrazine. A combination of said PR antagonists may comprise PPADS with A317491. A combination of said PR antagonists may comprise PPADS with spinorphin. A combination of said PR antagonists may comprise s 2,3,5,6-tetramethylpyrazine with spinorphin. A combination of said PR antagonists may comprise suramin with 2,3,5,6-tetramethylpyrazine and A-317491. A combination of said PR antagonists may comprise suramin with 8-cyclopentyl-1,3-dimethylxanthine and 2,3,5,6-tetramethylpyrazine. A combination of said PR antagonists may comprise suramin with 8-cyclopentyl-1,3-dimethylxanthine and A317491. A combination of said PR antagonists may comprise suramin with 1,3-dimethylxanthine and A317491. A combination of said PR antagonists may comprise suramin with 1,3-dimethylxanthine and 2,3,5,6-tetramethylpyrazine. A combination of said PR antagonists may comprise RB-2 with 2,3,5,6-tetramethylpyrazine and A-317491. A combination of said PR antagonists may comprise RB-2 with 8-cyclopentyl-1,3-dimethylxanthine and 2,3,5,6-tetramethylpyrazine. A combination of said PR antagonists may comprise RB-2 with 1,3-dimethylxanthine and 2,3,5,6-tetramethylpyrazine. A combination of said PR antagonists may comprise RB2 with 8-cyclopentyl-1,3-dimethylxanthine and A317491. A combination of said PR antagonists may comprise 2,3,5,6-tetramethylpyrazine with A-317491.

In an embodiment of the present invention a feeding inhibitor for an arthropod parasite, for example a blood-feeding arthropod parasite, such as a biting insect, comprises an antagonist of P1, P2X₂, P2X₃ or P2X_{2/3} PRs or an antagonist for any combination of said PRs belonging to any invertebrate or vertebrate organism.

In an embodiment of the present invention a feeding inhibitor for an arthropod parasite, for example a blood-feeding arthropod parasite, such as a biting insect, comprises antagonists of P1, P2X₂, P2X₃ or P2X_{2/3} PRs of said arthropod parasites as disclosed herein, combined with a compound or compounds selected from P1 PR antagonists listed herein, such as DPCPX, and/or combined with a compound or compounds selected from P2X₂, P2X₃ or P2X_{2/3} PR antagonists listed herein, such as AF-353.

In an embodiment of this invention a feeding inhibitor for an arthropod parasite, for example a blood-feeding arthropod parasite, such as a biting insect, comprises one or more compounds selected from a group consisting of: pyridoxalphosphate-6-azophenyl-2',5'-disulfonic acid (iso-PPADS); 2-[[2-hydroxy-5-(hydroxymethyl)-2-oxo-4H-[1,3,2]dioxaphosphinino[4,5-c]pyridin-8-yl]diazenyl]benzene-1,4-disulfonic acid (MRS 2220); MRS 2160; [3-[[4-formyl-5-hydroxy-6-methyl-3-(phosphonooxymethyl)pyridin-2-yl]diazenyl]-5-[[hydroxy(oxido)phosphoryl]methyl]phenyl]methyl-hydroxyphosphinate (MRS 2257); P¹P⁵-di-(inosine-5')pentaphosphate (Ip₅I); aurintricarboxylic acid; 2',3'-O-benzyldiene-ATP; 3'-deoxy-3'-(3,5- dimethoxybenzamido)-ATP (DT-0111); 4-[(2-amino-3,5-dibromophenyl)methylamino]cyclohexan-1-ol (ambroxol); 5-O-[2-[benzyl(methyl)amino]ethyl] 3-O-methyl 2,6-dimethyl-4-(3-nitrophenyl)-1,4-dihydropyridine-3,5-dicarboxylate (nicardipine).

In an embodiment of this invention a feeding inhibitor for an arthropod parasite, for example a blood-feeding arthropod parasite, such as a biting insect, comprises one or more compounds selected from a group consisting of: polysulfonated aromatic compound; sulfonamide tethered (hetero)aryl ethylidene compound; diaminopyrimidine compound; biaryl benzamide compound; teatrahydropyrido-pymiridine compound; pyrrolopyrimidin-7-one; pyrrolinone; minodronic acid; benzamide compound containing piperazine, tetrazole, thiazole, imidazole, triazole or pyrazole. The invention also comprises a pharmacologically admissible salt of said compounds.

In an embodiment of this invention a feeding inhibitor for an arthropod parasite, for example a blood-feeding arthropod parasite, such as a biting insect, comprises one or more compounds selected from a group consisting of: imidazopyridine; piperidino-aminopyrimidyl; benzamide; pyrrolidino-aminopyrimidyl; benzoimidazole; phenoxy-diaminopyrimidine; benzyl-diaminopyrimidine; carboxamide; benzoxazole; pyrrol; oxo-triazine; pyrimidinone. The invention also comprises a pharmacologically admissible salt of said compounds.

In an embodiment of this invention a feeding inhibitor for an arthropod parasite, for example a blood-feeding arthropod parasite, such as a biting insect, comprises one or more compounds selected from a group consisting of: diaminopyrimidine; arylamide; pyrazole; oxazole; pyridine; pyrimidine; pyrazolo-pyrimidine; imidazo-pyridine. The invention also comprises a pharmacologically admissible salt of said compounds.

In an embodiment of this invention a feeding inhibitor for an arthropod parasite, for example a blood-feeding arthropod parasite, such as a biting insect, comprises one or more compounds selected from a group consisting of: 2'(3')-*O-*(2,4,6-trinitrophenyl)-adenosine 5'-triphosphate (TNP-ATP); 2',3'-O-(2,4,6-trinitrophenyl)-ADP (TNP-ADP); 2',3'-O-(2,4,6-trinitrophenyl)-AMP (TNP-AMP); 2',3'-O-(2,4,6-trinitrophenyl)-GTP (TNP-GTP); 5-[[4,5-dimethoxy-2-(1-methylethyl)phenyl]methyl]-2,4-pyrimidinediamine (RO-3); 2',3'-O-cyclohexylideneATP; 2',3'-O-cyclohexylidene-α,β-methylene ATP; 5-(5-iodo-2-isopropyl-4-methoxy-phenoxy)-pyrimidine-2,4-diamine (AF-353 or RO-4); tetrasodium 4-amino-6-[[4-[4-[(8-amino-1-hydroxy-5,7-disulfonatonaphthalen-2-yl)diazenyl]-3-methylphenyl]-2-methylphenyl]diazenyl]-5-hydroxynaphthalene-1,3-disulfonate (Evans Blue); sodium 1-amino-9,10-dihydro-9,10-dioxo-4-(phenylamino)-2-anthracenesulfonate (Acid Blue 25); 5-(2,4-diamminopyrimidin-5-yloxy)-4-isopropyl-2-methoxybenzenesulfonamide (AF-219, MK-7264 or gefapixant); 9-(5-iodo-2-isopropyl-4-methoxybenzyl)-*N*⁶-methyl-9*H*-purine-2,6-diamine; 2-(R)-(4-fluoro-2-methylphenyl)-4-(5)-((8aS)-6- oxo-hexahydro-pyrrolo[1,2-a]-pyrazin-2-yl)-piperidine-1-carboxylic acid [I-(R)-(3,5-bis-trifluoromethyl-phenyl)- ethyl]-methylamide (orvepitant); 5-(4-methoxy-5-methylsulfonyl-2-propan-2-ylphenoxy)pyrimidine-2,4-diamine (AF-130); 5-(5-isobutyltetrazol-1-yl)- 4'-methylbiphenyl-3-carboxylic acid ((S)-2-hydroxy-1-methylethyl)amide; N-[1(R)-(5-fluoropyridin2-yl)ethyl]-3-(5-methylpyridin-2-yl)-5-[5(S)-(2-pyridyl)-4,5-dihydroisoxazol-3-yl]benzamide (MK-3901); 3-(5-methylthiazol-2-yl)-5-(((R)-tetrahydrofuran-3-yl)oxy)-*N*-((R)- 1-(2-(trifluoromethyl)pyrimidin-5-yl)ethyl)benzamide (eliapixant or BAY-1817080); methyl (S)-3-((2-(2,6- difluoro-4-(methylcarbamoyl)phenyl)-7-methylimidazo [1,2-a] pyridin-3-yl)methyl)piperidine-1-carboxylate (BLU-5937); (2S)-3-[(4E)-3-[(4-chlorophenyl)methyl]-2,6-dioxo-4-({4-[(pyridin-2-yl)oxy]phenyl}imino)-1,3,5-triazinan-1-yl]-2-methylpropanoic acid (sivopixant or S-600918); 5-cyclohexyl-3-hydroxy-1-(4-(isoxazol-4-yl)phenyl)- 4-(4-methoxybenzoyl)-1,5-dihydro-2*H*-pyrrol-2-one; biphenyl and phenyl-pyridine amides; oxazolone and pyrrolidinone-substituted arylamides; amino quinazoline; fused heterocyclics; triazole-substituted arylamide; oxalamide; indole arylamide; indazole arylamide; benzimidazole arylamide. The invention also comprises a pharmacologically admissible salt of said compounds.

In an embodiment of this invention a feeding inhibitor for an arthropod parasite, for example a blood-feeding arthropod parasite, such as a biting insect, comprises one or more compounds selected from a group consisting of: 8-[[3-[[3-[(4,6,8-trisulfonaphthalen-1-yl)carbamoyl]phenyl]carbamoylamino]benzoyl]amino]naphthalene-1,3,5-trisulfonic acid (NF023); tetrasodium 4-[[3-[[3,5-bis[(4-sulfonatophenyl)carbamoyl]phenyl]carbamoylamino]-5-[(4-sulfonatophenyl)carbamoyl]benzoyl]amino]benzenesulfonate (NF110); hexasodium 8-[[4-[[4-[[4-[[4-[(4,6,8-trisulfonatonaphthalen-1-yl)carbamoyl]phenyl]carbamoyl]phenyl]carbamoylamino]benzoyl]amino]benzoyl]amin o]naphthalene-1,3,5-trisulfonate (NF279); 5-methoxy-3-[[3-[[3-[[3-[[5-[(8-methoxy-3,6-disulfonaphthalen-2-yl)carbamoyl]-2-methylphenyl]carbamoyl]phenyl]carbamoylamino]benzoyl]amino]-4-methylbenzoyl]amino]naphthalene-2,7-disulfonic acid (NF770); 2-[[4-Methyl-5-[[3-[[3-[[2-methyl-5-[(1,3,7-trisulfonaphthalen-2-yl)carbamoyl]phenyl]carbamoyl]phenyl]carbamoylamino]cyclohexa-2,5-diene-1-carbonyl]amino]cyclohexa-2,4-diene-1-carbonyl]amino]naphthalene-1,3,7-trisulfonic acid (NF778); 1-amino-4-[3-[(4,6-dichloro-1,3,5-triazin-2-yl)amino]anilino]-9,10-dioxoanthracene-2-sulfonic acid (PSB-10211); 1-amino-4-[3-(4,6-dichloro[1,3,5]triazine-2-ylamino)-4-sulfophenylamino]-9,10-dioxo-9,10-dihydroanthracene-2-sulfonate (PSB-1011); 2-[[4-amino-5-(5-iodo-4-methoxy-2-propan-2-ylphenoxy)pyrimidin-2-yl]amino]propane-1,3-diol (AF-906 or RO-51); N-[(2R)-1-(4-acetylpiperazin-1-yl)propan-2-yl]-1-methyl-3-phenylthieno[2,3-c]pyrazole-5-carboxamide (RO-85); 5-[5-Methyl-2-(1-methylethyl)phenoxy]-2,4-pyrimidinediamine (TC-P 262); 8-cyclopentyl-1,3-dipropylxanthine (DPCPX): 1,3-dipropyl-8-[2-(5,6-epoxy)norbornyl] xanthine (ENX); (S-1,3-dipropyl-8[2-(5,6- epoxynorbornyl)]xanthine (CVT124); (5*R*)-5-[[9-methyl-8-[methyl(propan-2-yl)amino]purin-6-yl]amino]bicyclo[2.2.1]heptan-2-ol (WRC-0571); [2,8-bis(trifluoromethyl)quinolin-4-yl]-piperidin-2-ylmethanol (mefloquine); *trans*-4-[(2-Phenyl-7*H*-pyrrolo[2,3-d]pyrimidin-4-yl)amino]cyclohexanol (SLV 320); 8-(Hexahydro-2,5-methanopentalen-3a(1*H*)-yl)-3,7-dihydro-1,3-dipropyl-1*H*-purine-2,6-dione (KW 3902); *N*-(2-Aminoethyl)-2-[4-(2,3,6,7-tetrahydro-2,6-dioxo-1,3-dipropyl-1*H*-purin-8-yl)phenoxy]-acetamide dihydrochloride (XAC); 3-[[5-(2,3-dichlorophenyl)tetrazol-1-yl]methyl]pyridine (A-438079). The invention also comprises a pharmacologically admissible salt of said compounds.

In an embodiment this invention comprises a peptide, a polypeptide or a protein, and a pharmacologically admissible salts thereof, as a feeding inhibitor for an arthropod parasite, for example a blood-feeding arthropod parasite, such as a biting insect. Purotoxin-1, a 35 amino acid peptide of the wolf spider, antagonizes rat and human P2X₃ PRs at nanomolar doses. Spinorphin is an endogenous antinociceptive heptapeptide and a known antagonist of human P2X₃ PRs.

As herein disclosed in Figures 6 and 7 and in Table 4 of Examples, spinorphin serves as an antagonist of a purine-sensitive PR of the malaria mosquito at sub-nanomolar doses, and spinorphin inhibits feeding by the malaria mosquito at 100 nM as disclosed herein on Table 7 of Examples. A feeding inhibitor for an arthropod parasite may also comprise compounds structurally related to spinorphin, such as heptapeptides AVVYPWT, LAVYPWT, LVAYPWT, LVVAPWT and the cyclic peptide LWYPWT that serve as selective P2X₃ PR antagonists.

In an embodiment this invention comprises a natural product and pharmacologically admissible salts thereof as a feeding inhibitor for an arthropod parasite, for example a blood-feeding arthropod parasite, such as a biting insect. The isoflavone puerarin and polyphenols emodin and resveratrol are reported as P2X₃ PR antagonists. Theophylline, a constituent of tea, coffee and chocolate, is a non-selective P1 PR antagonist and as disclosed herein in Examples, theophylline antagonizes the response of the malaria mosquito P1 PR to adenosine. 2,3,5,6-Tetramethylpyrazine (TMP), synonym to ligustrazine, from *Ligusticum wallichii,* employed as an analgesic in Chinese medicine, is a P2X₃ PR antagonist. As disclosed herein on Figures 5, 6 and 7 and on Table 4 of Examples, TMP antagonizes a PR of the malaria mosquito at an ID₅₀ of 12 nM. Furthermore, and as disclosed herein on Figures 8 and 9 and on Table 7 of Examples, TMP inhibits feeding by the malaria mosquito at an ID₅₀ of 15 nM. In addition, TMP inhibits feeding by the Asian mosquito vector of malaria and by the horn fly as disclosed herein on Tables 8 and 10 of Examples. As disclosed herein on Tables 9 and 10 of Examples, TMP inhibits feeding when applied onto a membrane through which a biting fly and tick species feeds on a feeding medium with ATP added as feeding stimulant.

In an embodiment this invention comprises an antibody as a feeding inhibitor for an arthropod parasite, for example a blood-feeding arthropod parasite, such as a biting insect,. A means of using vascular endothelial growth factor (VEGF) antibody to inhibit the expression of the P2X₂ and P2X₃ PRs has been described, and application of monoclonal antibodies produced against human P2X₃ PRs induce significant inhibition of human P2X₃ and P2X_{2/3} PRs.

### Embodiments for metal modulators of P2X₂, P2X₃, P2X_{2/3} PRs

In an embodiment the product used for the protection against arthropod parasites comprises metal ions as modulators of P2X₂, P2X₃, P2X_{2/3} PRs of an arthropod parasite further comprising PR antagonist compounds. Metals such as copper, magnesium, iron, nickel and zinc modulate P2X₂ and P2X₃ PR function. As disclosed herein in Table 5 of Examples, Zn⁺⁺ significantly increases the response to ATP by the malaria mosquito purine-sensitive cell at 100 pM but not at double that dose, and 200 pM Mg⁺⁺ inhibits the response of the malaria mosquito purine-sensitive cell to ATP.

In an embodiment the present invention comprises a product for inhibiting feeding by an arthropod parasite using at least one metal ion modulator of its P2X₂, P2X₃, P2X_{2/3} PRs

In an embodiment the present invention comprises a product for inhibiting feeding by an arthropod parasite using at least one metal ion modulator of its P2X₂, P2X₃, P2X_{2/3} PRs further comprising PR antagonist compounds.

Embodiments for inhibiting feeding by an arthropod parasite , preferably a blood-feeding arthropod parasite, using an antagonist for at least one of its PRs as a feeding inhibitor

In an embodiment the present invention comprises a product for inhibiting feeding by an arthropod parasite, preferably a blood-feeding arthropod parasite, comprising an antagonist of at least one of its PRs.

In an embodiment the invention comprises a product for inhibiting feeding by an arthropod parasite, preferably a blood-feeding arthropod parasite, comprising an antagonist for at least one of its P1, P2X₂, P2X₃ or P2X_{2/3} PRs or for any combination of said PRs where said antagonist is selected by means of assay methods described in Materials and Methods and in Examples herein, or selected using other suitable assay methods described herein. Compounds structurally related to the selected antagonist, isomers thereof, esters thereof, prodrugs thereof and pharmaceutically admissible salts thereof are herein included.

In an embodiment, the invention comprises a product for inhibiting feeding by an arthropod parasite, preferably a blood-feeding arthropod parasite, comprising an antagonist for at least one of its P1 PRs.

In an embodiment, the invention comprises a product for inhibiting feeding by an arthropod parasite, preferably a blood-feeding arthropod parasite, comprising an antagonist for at least one its P1A₁ PRs.

In an embodiment, the invention comprises a product for inhibiting feeding by an arthropod parasite, preferably a blood-feeding arthropod parasite, comprising an antagonist for at least one of its P2X₂ PRs.

In an embodiment, the invention comprises a product for inhibiting feeding by an arthropod parasite, preferably a blood-feeding arthropod parasite, comprising an antagonist for at least one of its P2X₃ PRs.

In an embodiment, the invention comprises a product for inhibiting feeding by an arthropod parasite, preferably a blood-feeding arthropod parasite, comprising an antagonist for at least one its P2X_{2/3} PRs.

In an embodiment, the invention comprises a product for inhibiting feeding by an arthropod parasite, preferably a blood-feeding arthropod parasite, comprising an antagonist for any combination of its P1, P2X₂, P2X₃ and P2X_{2/3} PRs.

In an embodiment the invention comprises a product for inhibiting feeding by an arthropod parasite, preferably a blood-feeding arthropod parasite, through inhibition of at least one of its P1, P2X₂, P2X₃ or P2X_{2/3} PRs by at least one P1, P2X₂, P2X₃, P2X_{2/3} PR antagonist.

In an embodiment the invention comprises a product for inhibiting feeding by an arthropod parasite, preferably a blood-feeding arthropod parasite, comprising an antagonist for at least one of its P2X₂, P2X₃, P2X_{2/3} PRs accompanied by an antagonist for another PR of the arthropod parasite. For example, feeding inhibition can be achieved employing an antagonist for at least one P2X₂, P2X₃, P2X_{2/3} PR combined with an antagonist for at least one P1 PR of the arthropod parasite as disclosed herein on Figure 10 and Table 6 of Examples where the P1 PR antagonist 8-cyclopentyl-1,3-dimethylxanthine combined with the P2X₃ PR antagonist 2,3,5,6-tetramethylpyrazine serves to inhibit feeding by the malaria mosquito.

In an embodiment the invention comprises a product for inhibiting feeding by an arthropod parasite, preferably a blood-feeding arthropod parasite, using at least one metal modulator of P2X₂, P2X₃ or P2X_{2/3} PRs further comprising at least one P1, P2X₂, P2X₃, P2X_{2/3} PR antagonist.

In an embodiment the invention comprises a product for inhibiting feeding by an arthropod parasite, preferably a blood-feeding arthropod parasite, comprising an antagonist for at least one of its P1, P2X₂, P2X₃, P2X_{2/3} PRs and further comprising at least one metal modulator of P2X₂, P2X₃ or PX_{2/3} PRs.

In an embodiment the invention comprises a product for inhibiting feeding by an arthropod parasite, preferably an arthropod parasite, comprising an antagonist for at least one of its P1, P2X₂, P2X₃ or P2X_{2/3} PRs where said antagonist can be modified through derivatization, conjugation, or esterification to facilitate, among others, stability, miscibility, resistance to light exposure and for target delivery to the arthropod parasite.

In an embodiment the invention comprises inhibiting feeding by an arthropod parasite, preferably a blood-feeding arthropod parasite, using a feeding inhibitor comprising an antagonist for at least one of its P1, P2X₂, P2X₃ or P2X_{2/3} PRs where said inhibitor is applied onto a host animal for the prevention and/or treatment of an infestation by the arthropod parasite, preferably a blood-feeding arthropod parasite. This control method aims to inhibit the arthropod parasite, preferably a blood-feeding arthropod parasite, from feeding on the host animal. For example, in the case of a flying biting insect such as a mosquito or a biting fly, this can lead to the organism flying away in a starved state from a host animal.

In an embodiment the invention comprises inhibiting feeding by an arthropod parasite using a product comprising a PR antagonist where said product affects an endosymbiont of the arthropod parasite, thereby adversely affecting development, reproduction and survival of the arthropod parasite.

In an embodiment the invention comprises inhibiting feeding by an arthropod parasite using a PR antagonist where said antagonist is applied onto a host animal for the prevention and/or treatment of an infestation by the arthropod parasite. Said control method aims to inhibit host recognition, biting, entering the host animal's body, establishing a feeding site, feeding on host animal blood or on body fluids or on body tissues by the arthropod parasite, and/or inhibit the arthropod parasite from absorbing nutrients from the host animal through its integument or plasma membrane so as to prevent the arthropod parasite's survival, growth and reproduction. Examination of an adequate number of host animals for presence of the arthropod parasite made at regular intervals following application of the antagonist, such as is made by following a test protocol, allows assessment of the PR antagonist as a feeding inhibitor to control the arthropod parasite infestation, and permits establishment of an effective dose of the feeding inhibitor by a person skilled in the art.

In an embodiment the invention comprises inhibiting feeding by an arthropod parasite, preferably a blood-feeding arthropod parasite, using a PR antagonist where said antagonist is applied at an effective dose for the prevention and/or treatment of an infestation by the arthropod parasite. An effective dose is the amount of a feeding inhibitor that achieves the desired effect of inhibiting feeding by the arthropod parasite, preferably a blood-feeding arthropod parasite.

Establishing an effective dose of a PR antagonist that achieves the desired effect of inhibiting feeding by an arthropod parasite on a host animal considers several variables comprising, in case of arthropod parasites for example, the life stage and species of the arthropod parasite in question, the population density of the arthropod parasite, the type of compound, formulation of the compound, the method of application of said formulation and the required duration of the effect. Accordingly, the effective dose of a feeding inhibitor in a formulation or in any use form of the feeding inhibitor may vary over a wide range from 10 ⁻⁹ wt% to 50 wt%, preferably from 10 ⁻⁷ wt% to 5 wt% of the feeding inhibitor as a proportion of the formulation or of any use form of the feeding inhibitor. Details of suitable formulation methods for active ingredients used in the control of parasites are known in the art. A person skilled in the art of controlling an arthropod parasite infestation can therefore determine the amount of a compound of this invention that is required as a feeding inhibitor for the prevention and/or treatment of the infestation.

In an embodiment the invention comprises inhibiting feeding by an arthropod parasite for the purpose of preventing and/or treating an infestation by said parasite on a host animal using a product comprising a PR antagonist where said product is applied topically onto a host animal's pelage or skin, and/or onto a neck collar, a saddle, a cover for a host animal such as a horse cover or fly rug, and/or onto a fly screen or any other protective screen or netting for a host animal, and/or onto a fly mask, cap or blinkers for a host animal, and/or onto a harness, bridle, or any means of restraining a host animal.

In an embodiment the invention comprises inhibiting feeding by an arthropod parasite for the purpose of preventing and/or treating an infestation by said parasite on a host animal using a product comprising a PR antagonist where said product is applied in the form of a spray onto a host animal's pelage or skin, and/or onto a neck collar, a saddle, a cover for a host animal, such as a horse cover or fly rug, and/or onto a fly screen or any other protective screen or netting for a host animal, and/or onto a fly mask, cap or blinkers for a host animal, and/or onto a harness, bridle, or any means of restraining a host animal.

In an embodiment the invention comprises inhibiting feeding by an arthropod ectoparasite for the purpose of preventing and/or treating an infestation by said parasite on a host animal using a product comprising a PR antagonist where said product is applied topically in the form of a solution or in the form of a spray onto a predilected biting or feeding site or onto a preferred egg deposition site of the arthropod parasite on a host animal.

In an embodiment the invention comprises inhibiting feeding by an arthropod parasite for the purpose of preventing and/or treating an infestation by said parasite on a host animal using a product containing PR antagonist where said product that may optionally contain a surfactant is applied, for example, in the form of a solution, emulsion, shampoo, cream, gel, lotion, ointment, by microencapsulation, as a nanoparticle formulation such as a nanoemulsion, as a sustained-release formulation, or as an ear tag preparation, or by applying any other suitable pour-on or pharmaceutical preparation, or by applying a solution or by applying a device onto the host animal's pelage or skin, and/or onto a neck collar, a saddle, a cover for a host animal, such as a horse cover or fly rug, and/or onto a fly screen or any other protective screen or netting for a host animal, and/or onto a fly mask, cap or blinkers for a host animal, and/or onto a harness, bridle, or any means of restraining a host animal.

In an embodiment the invention comprises inhibiting feeding by an arthropod parasite for the purpose of preventing and/or treating an infestation by said parasite using a product comprising a PR antagonist where said product is applied at an effective dose onto clothing of a person by use of a spray or by immersing a person's clothing in an appropriate solution containing said feeding inhibitor.

In an embodiment the invention comprises inhibiting feeding by an arthropod parasite for the purpose of preventing and/or treating an infestation by said parasite using a product comprising a PR antagonist where said product is applied at an effective dose onto a bed net by use of a spray or by immersing the bed net in an appropriate solution containing said PR antagonist.

In an embodiment the invention comprises inhibiting feeding by an arthropod parasite for the purpose of preventing and/or treating an infestation by said parasite using a product comprising a PR antagonist where said product is applied topically in the form of a solution onto the arthropod parasite.

In an embodiment the invention comprises inhibiting feeding by an arthropod parasite for the purpose of preventing and/or treating an infestation by said parasite using a product comprising a PR antagonist where said product is applied in the form of the spray onto the arthropod parasite.

In an embodiment the invention comprises inhibiting feeding by an arthropod parasite for the purpose of preventing and/or treating an infestation by said parasite using a product comprising a PR antagonist where said product is applied topically in the form of a solution or in the form of a spray onto any life stage of the arthropod parasite.

In an embodiment the invention comprises inhibiting feeding by an arthropod parasite for the purpose of preventing and/or treating an infestation by said parasite using a product comprising a PR antagonist where said product is incorporated at an appropriate dose into an attractive bait, for example a sugar bait, fed on by the arthropod ectoparasite.

In an embodiment the invention comprises inhibiting feeding by an arthropod parasite for the purpose of preventing and/or treating an infestation by said parasite using a product comprising a PR antagonist where said product that may optionally contain a surfactant is applied in the form of a spray or solution, or applied using a slow-release device, or applied by microencapsulation, or applied in a sustained-release long-lasting formulation onto and/or into, for example, any facility for a host animal comprising a refuge, and/or a tent, and/or a dwelling, and /or a barn, and /or a stable, and /or a perch, and/or a cage, and/or an animal rearing or housing facility or any part thereof, and/or a breeding site of an arthropod parasite, and/or a swarm occupied by an arthropod parasite, and/or a terrestrial zone, and/or an aquatic zone, and/or a biotope, and/or a natural or man-made habitat occupied by the arthropod parasite or by any life stage of said parasite. Details of suitable preparation methods for P1, P2X₂, P2X₃ or P2X_{2/3} antagonist application are found in Knowles D.A. 1998, "Chemistry and Technology of Agrochemical Formulations", Springer Dordrecht.

In an embodiment the invention comprises inhibiting feeding by an arthropod parasite for the purpose of preventing and/or treating an infestation by said parasite using a product comprising a PR antagonist where said product optionally comprises an insecticide, insecticide synergist, acaricide, parasiticide, repellent or a chemosterilant, each at its effective dose, in the form of a solution or a spray, in the form of a shampoo, or from a slow-release device, or by microencapsulation, or in a sustained-release long-lasting formulation for topical application onto a host animal's pelage or skin, onto any life stage of the arthropod parasite, and/or for application onto a neck collar, a saddle, a cover for a host animal such as a horse cover or fly rug, and/or onto a fly screen or any other protective screen or netting for a host animal, and/or onto a fly mask, cap or blinkers for a host animal, and/or onto a harness or bridle, or any means of restraining a host animal.

In an embodiment the invention comprises inhibiting feeding by an arthropod parasite for the purpose of preventing and/or treating an infestation by said parasite using a product comprising a PR antagonist where said product optionally comprises an insecticide, insecticide synergist, acaricide, parasiticide, repellent or a chemosterilant, each at its effective dose, in the form of a solution or a spray, or from a slow-release device, or by microencapsulation, or in a sustained-release long-lasting formulation for application onto or into any facility for a host animal comprising a refuge, a tent, a dwelling, a barn, a stable, a perch, a cage, an animal rearing or housing facility or any part thereof, a breeding site of the arthropod parasite, a swarm occupied by the arthropod parasite, a terrestrial zone, an aquatic zone, a biotope, or a natural or man-made habitat occupied by the arthropod parasite or occupied by any life stage of the arthropod parasite. An effective dose of an insecticide, insecticide synergist, acaricide, parasiticide or a chemosterilant is one which permits controlling the arthropod parasite infestation.

In an embodiment the invention comprises inhibiting feeding by an arthropod parasite for the purpose of preventing and/or treating an infestation by said parasite using a product comprising a PR antagonist where said product optionally comprises an insecticide, insecticide synergist, acaricide, repellent or parasiticide or a chemosterilant, each at its effective dose, for application onto a person's clothing and/or onto a bed net in the form of a spray or by immersion of the clothing or bed net in an appropriate solution containing the combination of said product and the insecticide, insecticide synergist, acaricide, parasiticide or chemosterilant.

### Embodiments for inhibiting feeding by an arthropod parasite with a therapeutically effective does of a PR antagonist

In an embodiment the invention comprises inhibiting feeding by an arthropod endoparasite using a product comprising a PR antagonist where said product is administered as a pharmaceutical formulation onto or internally to a host animal for the prevention and/or treatment of an arthropod endoparasite infestation . This control method aims to inhibit the arthropod endoparasite from recognizing the host, entering the host animal's body, establishing a feeding site, inhibit the arthropod endoparasite from feeding on host animal blood or on body fluids or on body tissues, and/or inhibit the arthropod endoparasite from absorbing or adsorbing nutrients from the host animal through its integument or plasma membrane so as to prevent the arthropod endoparasite's survival, growth and reproduction. Examination of an adequate number of host animals for presence of the arthropod endoparasite made at regular intervals following administration of the PR antagonist, such as is made by following a test protocol, allows assessment of a compound of this invention as a feeding inhibitor and permits a person skilled in the art to determine a therapeutically effective dose of the PR antagonist comprised in said product for the prevention and/or treatment of the arthropod endoparasite infestation.

In an embodiment the invention comprises inhibiting feeding by an arthropod endoparasite or an arthropod ectoparasite for the prevention and/or treatment of an infestation by said parasite using a product comprising a PR antagonist where said product is administered at a therapeutically effective dose onto or internally to a host animal. The product, that may optionally contain a surfactant, can be administered as a pharmaceutically acceptable formulation suitable for parenteral, oral, buccal, enteral, rectal, vaginal, nasal, intramuscular, intravenous, intraarterial, intrathecal, subcutaneous, and transdermal delivery, or by any suitable means for systemic delivery of said pharmaceutical formulation to the host animal. Establishing a therapeutically effective dose for the PR antagonist that achieves the desired effect of inhibiting feeding by the arthropod endoparasite or an arthropod ectoparasite considers several variables, comprising the type of compound, the method of administration of the formulated compound onto and/or internally to the host animal, the pharmacokinetics of the compound, the pharmacodynamics of the compound, the species of endoparasite being targeted, the severity of a endoparasite's burthen on the host animal, the age and health of the host animal and the duration of protection against the endoparasite that is desired.

The therapeutically effective dose of a PR antagonist that achieves the desired effect of inhibiting feeding by an arthropod endoparasite or an arthropod ectoparasite to be used in a pharmaceutically acceptable formulation, or in a sustained-release formulation or in any use form of the feeding inhibitor may vary over a wide range from 0.000000001 wt% to 50 wt%, preferably between 0.0000001 wt% to 5 wt% by weight of the PR antagonist as a proportion of the pharmaceutical formulation or of any use form of the feeding inhibitor. A person skilled in the art of controlling endoparasitic and ectoparasitic arthropods can determine the amount of a compound or compounds of this invention that is required to inhibit feeding by the endoparasite for the prevention and/or treatment of the infestation by said parasite.

In an embodiment the invention comprises inhibiting feeding by an arthropod parasite using a product comprising a PR antagonist where said product is purposely synthesized to inhibit P1, P2X₂, P2X₃ or P2X_{2/3} PRs of both humans and animals, formulation of said compound as a pharmaceutical preparation and administration of said compound at a therapeutically effective dose for the prevention and/or treatment of an infestation by an arthropod parasite. Details of pharmaceutically suitable preparation methods for P1, P2X₂, P2X₃ or P2X_{2/3} PR antagonist administration onto or internally to a host animal are provided in Felton L. 2013, in "Remington: Essentials of Pharmaceutics", Pharmaceutical Press London.

In an embodiment the invention comprises inhibiting feeding by an arthropod endoparasite or an arthropod ectoparasite for the prevention and/or treatment of an infestation by said parasite using a product comprising a PR antagonist where said product optionally comprises an insecticide, insecticide synergist, acaricide, parasiticide, endectocide or a chemosterilant, each at its therapeutically effective dose, for administration onto or internally to a host animal as a pharmaceutical formulation, or as a sustained-release formulation or in any use form suitable for parenteral, oral, buccal, enteral, rectal, vaginal, nasal, intramuscular, intravenous, intraarterial, intrathecal, subcutaneous, and transdermal delivery, or by any suitable means for systemic delivery said pharmaceutical formulation to the host animal. A therapeutically effective dose of an insecticide, insecticide synergist, acaricide, parasiticide or a chemosterilant is one which permits controlling the arthropod endoparasite.

### Materials and Methods

### Mosquitoes, ticks and biting flies:

*Anopheles gambiae* Giles (Diptera, Culicidae) 16cSS-strain colony derived from wild-caught adults in Lagos, Nigeria in 1974. The *Anopheles stephensi mysorensis* Liston (Diptera, Culicidae) colony originated from the Swiss Tropical Institute, Basel. Colonies were maintained under standardized conditions. A 10% sucrose solution and water were supplied *ad libitum* on cotton for adults. Females were deprived of sucrose the night before electrophysiological recordings.

N3 or N4 nymphs of the eyeless tampan, *Ornithodorus moubata* Murray (Ixodida, Argasidae) , were from an in-house culture of individuals collected in Ulanga District, Tanzania and originally maintained at the Swiss Tropical Institute, Basel. *Ixodes ricinus* Linnaeus (Ixodida, Ixodidae) and *Amblyomma hebraeum* Koch (Ixodida, Ixodidae) were from colonies at Novartis, Centre de Recherche Santé Animale SA, St Aubin, Switzerland. *Glossina pallidipes* Austen (Diptera, Glossinidae) pupae were obtained from the International Atomic Energy Agency (IAEA) Siebersdorf Laboratories, Austria. Adult tsetse were sexed at emergence and maintained separately in cotton-netting cages (1-mm mesh; 25 x 15 x 15 cm, tg^{®} tubular bandage, Lohmann & Rauscher, St Gallen, Switzerland) in an environmental cabinet at 24°C, 80% relative humidity (RH) under 10 h photophase:14 h scotophase with 1 hour light ramps in the first and last hour of the photophase. Pupae of the horn fly, *Hoematobic irritans* Linnaeus (Diptera, Muscidae), obtained from a rearing at BAYER Animal Health GmbH, Monheim, Germany were maintained in cages as tsetse using 0.5-mm mesh netting and provided with water-soaked cotton on the cage roof from emergence.

### Chemicals:

Compounds used for the characterisation of purine-sensitive sensory cells in *A. gambiae* are listed in Tables 1 and 2. Compounds were dissolved in nanopure water and serially diluted. Solutions were kept at -20°C for less than a week and defrosted before experiments. D-(+)-sucrose (purity >99.5%) was from Sigma-Aldrich, Switzerland.

### Electrophysiology:

The tip recording method was used to record action potentials (spikes) from sensory cells in sensilla on *A. gambiae* labellum lobes. Contrary to other labellum sensilla, the six most distally placed trichoid type 1 (T1) 8v sensilla do not house watersensitive neurones in *A. gambiae,* allowing water to be used as a negative control in recordings.

Sensilla were exposed to test compounds and PR antagonists for ca. 2 - 4 s and spikes were counted in the first 2 s of recordings. Up to 4 test compounds were tested on a sensillum to evaluate potential agonists, each presented at 200pM. To study responses as a function of dose for agonists ATP, adenosine, AMP, AMP-CPP and ATP + adenosine, up to 7 recordings were made at increasing doses of agonist from the same sensillum. Unless otherwise stated, recordings were made throughout from 4 to 16 sensilla from individual 4 - 6-day-old females per dose of agonist, antagonist and metal ion modulator. For example, a response curve established over 6 doses of an antagonist using 8 sensilla per dose involved recording from a sensillum of 48 separate mosquitoes. A given dose of a PR antagonist or metal ion modulator was only presented once to a sensillum. Two-second recordings were imported as ASCII files into DataView software for analysis of spike amplitude. Differences in spike amplitude generated by stimulation of one 8v sensillum/female with 1 mM sucrose (n =294 spikes from 6 females) 200 pM adenosine (n =207 spikes from 7 females) and 200pM ATP (n =192 spikes from 7 females) were compared by measuring the spike amplitude in mV between the highest point of depolarisation and the lowest point of hyperpolarisation.

### Feeding assays:

Experiments with *A. gambiae* and *A. stephensi* were made in a climate chamber at 28°C and 80% RH during the last 6 hours of scotophase. As a sugar meal can interfere with the blood meal by reducing the number of females that feed, the sucrose solution supplied in rearing cages to mosquitoes employed for feeding experiments was reduced to 5%. This did not affect mosquito survival but induced higher appetence. The 5% sucrose solution was presented to mosquitoes from emergence on four 1 cm diam. cotton dental rolls (Hartmann AG, Neuhausen, Switzerland) inserted in 10 ml upturned glass vials placed in holes at each corner of the rearing cage ceiling. Cotton rolls were turned daily in vials and renewed every two days. Mosquitoes were not fed blood and were deprived of sucrose 17 to 20 hours before experiments. Thirty min before a feeding experiment, between 10 - 25, 6-day-old females that responded to activation by human breath were removed with a mouth aspirator from the rearing cage wall and placed in polystyrol cylinders with the floor cut out (10 cm dm x 15 cm high; Semadeni, Ostermundigen, Switzerland) that served as feeding cages. Each cage was covered with mosquito netting at one end and a soft polyurethane lid at the other.

Mosquitoes were fed for 30 min using a feeding system (Hemotek^{®}, Hemotek Ltd, Blackburn, UK) consisting of 3.5 ml stainless steel reservoirs 37 mm in diameter covered with a 60 - 90 µm thick silicone-impregnated membrane (Kröber and Guerin, 2007 in "Feeding assays for hard ticks", Trends in parasitology 23, 445-449; doi 10.1016/j.pt.2007.07.010). Feeding reservoirs reached a temperature of 37°C within 10 min and were placed on the netting of feeding cages. Mosquitoes were activated using a 200 ms pulse of pure CO₂ (volume 6.25 ml) from a pressurised cylinder (Carbagas, Switzerland) using a solenoid valve that caused the CO₂ level in the feeding cage to rise to at least 1,500 ppm. Between 3 and 4 sets of 10 feeding cages held in a custom-built Ertacetal^{®} POM frame were tested per day, depending of the number of female mosquitoes available from the colony.

All compounds were tested in a mosquito feeding solution (FS) consisting of 25 mM dextrose, 25 mM NaCl buffered with 8.93 mM NaHCO₃ adjusted to pH 7.4. Feeding responses *of A. gambiae* and *A. stephensi* to ATP as a function of dose were tested with between 7 to 10 feeding cages per dose. Each feeding experiment with an antagonist systematically included a treatment containing the FS as negative control along with a treatment containing the FS with 20 µM ATP added as positive control for *A. gambiae* and with 160 µM ATP added as positive control for *A. stephensi.* These positive and negative controls permitted to monitor any effect of experimental day on feeding responses as a function of dose for antagonists that were recorded over several days. Each dose of PR antagonist was tested in the FS with 20 µM ATP for *A*. *gambiae* and with 160 µM ATP for *A*. *stephensi.* Four to 10 cages each with 10 to 29 mosquitoes were tested per antagonist dose. The experimental procedure for testing antagonists as feeding inhibitors was set up using suramin as test compound for *A*. *gambiae,* hence 43 positive controls for this compound. The number of feeding cages used to test other compounds and their mixtures as feeding stimulants or as feeding inhibitors is listed on Table 6. The percentage of fed mosquitoes was estimated by visual inspection of abdominal distention that is indicative of engorgement.

**Table 1. Substances tested for characterization of A. gambiae PRs**

| **Substance** | **Synonym/ abbreviation** | **CAS Number** | **Purity** |
|---|---|---|---|
| 6-Aminopurine **1** | Adenine | 73-24-5 | >98% |
| 9-β-D-Ribofuranosyladenine **2** | Adenosine | 58-61-7 | >99% |
| Adenosine 5'-monophosphate **3** | AMP | 61-19-8 | 99% |
| Adenosine 5'-diphosphate **4** | ADP | 58-64-0 | 97% |
| Adenosine 5'-O-(2-thiodiphosphate) **5** | ADP-β-S | 73536-95-5 | >80% |
| Adenosine 5'-triphosphate **6** | ATP | 34369-07-8 | 99% |
| α,β-Methyleneadenosine-5'-triphosphate **7*** | AMP-CPP | 1343364-54-4 | >98% |
| 2'-Deoxyadenosine 5'-triphosphate **8** | dATP | 1927-31-7 | >99% |
| 2',3'-Dideoxyadenosine-5'-triphosphate **9** | ddATP | 178451-61-1 | ≥98% |
| 9-β-D-Ribofuranosyl-guanine **10** | Guanosine | 118-00-3 | >99% |
| Guanosine 5'-monophosphate **11** | GMP | 5550-12-9479 | ≥99% |
| Guanosine 5'-diphosphate **12** | GDP | 43139-22-6 | ≥96% |
| Guanosine 5'-triphosphate **13** | GTP | 36051-31-7 | ≥95% |
| 2,4(1H,3H)-Pyrimidinedione **14** | Uracil | 66-22-8 | >99% |
| 1-β-D-Ribofuranosyluracil **15** | Uridine | 58-96-8 | >99% |
| Uridine-5'-monophosphate **16** | UMP | 3387-36-8 | 98% |
| Uridine-5'-diphosphate **17** | UDP | 27821-45-0 | >96% |
| Uridine 5'-triphosphate **18** | UTP | 116295-90-0 | >80% |

| | | | |
|---|---|---|---|
| Substances were purchased from Sigma-Aldrich, Schnelldorf, Germany and *Tocris Bioscience, Bristol, UK. | | | |

*I. ricinus* adults were prefed for 3-4 days and *A. hebraeum* adults for 10 days on a membrane over bovine blood (Kröber T. and Guerin P.M., 2007, *supra).* Partly fed *I. ricinus* and *A. hebraeum,* freshly detached from the membrane, were tightly fixed by the scutum and alloscutum to double-sided adhesive tape (Tesafix^{®} 4964, Beiersdorf, Hamburg, Germany) on a glass slide and secured with plasticine. Test compounds were fed to these partly-fed female ticks using borosilicate capillary tubes (Clark GC120-10, 0.69 mm i.d., 1.2 mm o.d., 50 mm long; Clark Electromedical Instruments, Pangbourne, Reading, UK) with drawn out tips adapted to fit around the mouthparts of individual ticks. Feeding capillaries were filled with a solution of 100 mM NaCl, 8.9 mM NaHCO₃ with 1000 µM ATP added, hereafter termed tick feeding solution (TFS) that served as positive control in each feeding experiment. PR antagonists were added to the TFS and pH of the solution was adjusted to 7.2 -7.8. Capillary-fed ticks were kept in a plastic box on a warm plate at 34°C and 80 % RH using wet paper. After 1 h, the amount of solution imbibed by a tick was indicated by the drop of the meniscus in the capillary and measured using Vernier callipers and converted to mg. Numbers of ticks (n) tested per treatment are provided in Table 9.

*O. moubata* nymphs were fed for 25 min through a membrane (50-75 µm thick) as for hard ticks in glass feeding unit on TFS containing 1000 µM ATP as positive control in each feeding experiment. PR antagonists were added to the TFS as for hard ticks. One compound, TMP was applied onto the membrane in aqueous solution (8 µg TMP in 70 µl) in small drops using a micropipette. Individual nymphs were marked on the dorsum with nail varnish in distinguishable colours and weighed before and after the experiment. Numbers of ticks (n) tested per treatment are provided in Table 9.

Flies of both species were transferred 30 min before feeding experiments to feeding cages as used for mosquitoes at 24°C, 80% RH in an environmental cabinet. Teneral tsetse were fed at 2 to 4 days old during the photophase with between 7 and 13 flies per feeding cage, covered with a black cloth. Horn flies were tested at 1 and 2 days old in the photophase with between 6 and 21 flies per cage. Both fly species were fed using small Hemotek feeding reservoirs (29 mm diameter, 1 ml) equipped with a membrane as for mosquitoes. Horn flies were fed for 15 min with feeding reservoirs placed on the cage. For tsetse, cages were closed with black mosquito netting at the bottom to allow flies to feed through the membrane placed under the cage. Feeding time (1.5 to 6 min) for tsetse was adjusted as a function of age to achieve at least 30 % feeding on the positive control included in each feeding experiment. The positive control for both fly species contained 150 mM NaCl, 8.9 mM NaHCO₃ with 100 µM ATP added, hereafter termed fly feeding solution (FFS). The pH of the FFS was adjusted as for ticks. The effect of TMP on feeding by *G. pallidipes* was also tested at 16 µg TMP/cm² dissolved in 140 µl hexane and applied homogeneously to the membrane surface using a micropipette. Hexane was used to treat the membrane with TMP to avoid the test compound being dispersed by fly wing-fanning. Flies of both species were activated just prior to feeding on a treatment by exhalation into the cage. Tsetse that had fed were identified with swollen abdomens and counted at the end of a feeding experiment. Since it was difficult to ascertain by eye whether a horn fly had fed on a treatment, 10 µl/ml of blue food colorant (No. 4866908, Trawosa AG, St.Gallen, Switzerland) was added. This facilitated the detection of ingested liquid when the abdomen of flies, killed by freezing, was squashed onto filter paper. Only experiments in which the feeding rate by flies was >30% on the positive control were included in analyses. Numbers of flies tested per treatment (n) are provided in Table 10.

### Data analysis

Significance level for all tests was set at *P* =0.05. Asterisks on bar graphs represent significance levels at * *P* <0.05, ** *P* <0.01, and *** *P* <0.001; NS not significant.

**Table 2. P1 PR and P2 PR antagonists tested for characterization of A. gambiae PRs**

| **Purinergic receptor antagonis t** | **Name/ abbreviation** | **Purin-ergic receptor subtype** | **CAS number/ other identifier** | **Purity** |
|---|---|---|---|---|
| 8-[[4-methyl-3-[[3-[[3-[[2-methyl-5-[(4,6,8-trisulfonaphthalen-1-yl) carbamoyl]phenyl]carbam oyl]phenyl] carbamoylamino]benzoyl] amino]benzoyl] amino]naphthalene-1,3,5-trisulfonic acid **19*** | Suramin | P2 | 145-63-1 | ≥99% |
| pyridoxal phosphate-6-azophenyl-2',4'- disulfonic acid **20** | PPADS | P2 | 149017-66-3 | ≥98% |
| [2-[(4-carboxyphenyl)diazenyl]-4-formyl-6-methyl-5-oxidopyridin-3-yl]methyl phosphate **21** | MRS 2159 | P2X₁, (P2X₇) | PubChem CID 135484644 | >98% |
| 1-amino-4-[[4-[[4-chloro-6-[[3 (or 4)-sulfophenyl]amino]-1,3,5-triazin-2-yl]amino]-3-sulfophenyl]amino]-9,10-dihydro-9,10-dioxo-2-anthracenesulfonic acid **22**** | Reactive Blue-2, RB-2 | P2X₂, | 12236-82-7 | 94% |
| 5-[(3-phenoxyphenyl)methyl-[(1S)-1,2,3,4-tetrahydronaphthalen-1-yl]carbamoyl]benzene-1,2,4-tricarboxylic acid **23** | A-317491 | P2X₃, P2X_{2/3} | 475205-49-3 | >98% |
| 2,3,5,6-tetramethylpyrazine | TMP | P2X₃ | 1124-11-4 | >98% |
| L-leucyl-L-valyl-L-valyl-L-tyrosly-L-prolyl-L-tryptophyl-L-threonine **24*** | Spinorphin | P2X₃ | 137201-62-8 | 98% |
| (3*S*,4*R*)-3-(1,3-benzodioxol-5-yloxymethyl)-4-(4-fluorophenyl)piperidine **25** | Paroxetine | P2X₄ | 61869-08-7 | >98% |
| 3-[[5-(2,3-dichlorophenyl)tetrazol-1-yl]methyl]pyridine **26** | A-438079 | P2X₇ | 899507-36-9 | >98% |
| 1,3-dimethylxanthine **27** | Theophylline | P1 | 58-55-9 | >99% |
| 8-cyclopentyl-1,3-dimethylxanthine **28** | 8-Cyclopentylth eophylline /CPT | P1A₁ | 35873-49-5 | > 98% |

| | | | | |
|---|---|---|---|---|
| Substances were purchased from Sigma-Aldrich Schnelldorf, Germany; * Tocris Bioscience, Bristol, UK; and **Chemical Point (Deisenhofen, Germany) | | | | |

Spike counts elicited by 200pM doses of nucleotides, nucleosides, purines, pyrimidines and water were log(x + 1.25)-transformed (optimal constant numerically established using a maximum likelihood procedure) and analysed using a linear mixed effects model (function 'Ime' in *"nlme"* R package) with mosquito number as random effect. To model inhomogeneous variance in residuals (near normal distribution, *P =* 0.045, Shapiro-Wilk test, W=0.98604), the variance function 'varldent' in the *"nlme"* package that allows for different variance per treatment was used. Statistical differences were identified between treatments using post-hoc-Tukey test with contrasts adjusted by Holm's family wise error compensation (FWEC). Spike counts elicited by ATP, AMP, adenosine and their binary mixtures were analysed using a generalised linear model (LM) of log (x + 10) transformed data and differences were identified using post-hoc-Tukey test with Holm's FWEC.

Log transformed spike amplitudes generated in response to ATP, adenosine and sucrose were compared using linear mixed effects models using the 'Ime' function of the *"nlme"* R package, with spike amplitude as the dependent variable, treatment as the fixed factor and mosquito number within treatment as random effect. Residuals were normally distributed (Shapiro-Wilk test *P* > 0.0563) and their variances homogeneous (Bartlett test *P* >0.9799); *P* - values were corrected using Holm's FWEC.

Pairwise comparisons *of A. gambiae* electrophysiological (EP) responses to ATP and adenosine alone and combined with a given dose of PR antagonist, EP responses to ATP alone and combined with a given dose of a metal modulator, and EP responses to ATP prior to and after exposure of a sensillum to a given dose of a PR antagonist were made with a one-tailed Wilcoxon signed-rank test for data showing a non-normal distribution, with a paired Student's t-test for normally distributed data showing homogeneous variance and with a Welch's t-test for normally distributed data showing non-homogeneous variance. Effects on feeding of adding ATP, AMP-CPP, adenosine, ATP + adenosine, AMP and ADP-βS, CPT with ATP or adenosine, TMP with ATP + adenosine, TMP + CPT with ATP + adenosine to the FS for *A*. *gambiae,* and of adding PR antagonists with ATP to the FS for *A. stephensi* were compared using one-tailed Mann-Whitney U-test or Welch's t-test, depending on data distribution; Holm's FWEC was applied where treatment comparisons involved the same positive control. Percent feeding inhibition induced by PR antagonists in *A. stephensi* was calculated using Abbott's formula.

Dose-response models fitted to mosquito data were generated using the 'drm' command in the *"drc"* R package (version 2.5-12). Best fitting models (goodness of fit) and effects of dose of a compound were assessed using, respectively, 'modelFit' and 'noEffect' functions in the same R package. A 3-parameter logistic model (L3) was fitted to spike-counts recorded to increasing doses of adenosine, AMP, ATP and AMP-CPP. Spike numbers recorded in response to 200 pM ATP with increasing doses of PR antagonists added were analysed using a 4-parameter log-logistic model (LL.4) for A-317491 and spinorphin, using a 4-parameter Weibull model (W1.4) for PPADS, RB-2 and TMP, and using a 3-parameter log-logistic model (LL.3) for suramin. An L3 model was fitted to proportions of females feeding on increasing doses of ATP. Feeding rates on 20 µM ATP with increasing doses of a PR antagonist added were analysed using a 4-parameter log-logistic model (LL.4) with the median of the positive control (20 µM ATP) fixed as the upper asymptote. Response dynamics in 3-parameter models are related to the slope b, the upper asymptote, and inflexion point of the model e (the ED₅₀ value), whereas 4 parameter models also include the lower asymptote parameter. The upper asymptote d in EP responses represents the maximum response, which is insurmountable, i.e., the response in spike count does not rise regardless of how much the dose of agonist increases.

Effective ED₅₀ and ED₉₅ doses of agonists and inhibitory ID₅₀ and ID₉₅ doses of antagonists were estimated using the 'ED function' of the *"drc"* package. An ED*ₓ* value is the effective dose of a compound at x percent of the total response encompassed between the upper and lower asymptotes estimated by the *"drc"* model. Statistical differences between ED₅₀ values of compounds were compared using the 'EDcomp' and 'comParm' functions in the *"drc"* package. In EP experiments an ID*ₓ* value is the inhibitory dose of an antagonist inducing x percent of its maximum effect on the response to 200 pM ATP. Reduction in spike count in the response to 200 pM ATP induced by a PR antagonist at a dose approximating its ID₉₅ value was compared with the response to the corresponding positive control (200 pM ATP) presented before and after the antagonist treatment using one-tailed Wilcoxon rank-sum test, Students t-test or Welch's t-test. In feeding experiments an ID*ₓ* value is the dose of an antagonist reducing the feeding rate by x percent on a solution of a phagostimulant relative to the upper and lower limits of the *drc* estimated for the phagostimulant presented alone. The effect of a PR antagonist at a dose approximating its ID₅₀ value on feeding by *A. gambiae* on 20 µM ATP was compared to the positive control using the Mann Witney U-test or Student *t*-test, depending on the data distribution; *P -* values were corrected using Holm's FWEC for paroxetine and A-438079 that shared the same positive control.

Uptake of TFS containing PR antagonists, converted to mg/h for hard ticks and weight gains in mg/25 min for soft ticks, was compared to TFS uptake in the corresponding positive controls. For soft ticks, 3.5 mg was added to all measurements to compensate for negative values when ticks did not feed and lost weight through evaporative water loss. For tsetse and horn flies the percentage flies that fed per antagonist treatment served as the criterion for inhibition of feeding. As horn fly sexes emerged within 24 h, both sexes were assumed to show the same degree of appetence. In tsetse, males emerged first so a gender-related appetence could not be excluded. No sex-related feeding differences in feeding were found for testse (GLM, *P* >0.05) so data for the sexes were pooled. Comparisons of percentage fed flies were made using a Bayesian GLM - the most appropriate model considering the zeroinflated distributions shown by the data sets. As measurements of reduced feeding on TFS containing PR antagonists by hard ticks and weight gain by soft ticks did not fit to a particular distribution type, values were compared with corresponding positive controls using the Kruskal-Wallis test for soft tick data, the one-tailed Wilcoxon test for *A. hebraeum* and the one-tailed Mann-Whitney U tests for *I. ricinus. P -* values were corrected using Holm's FWEC in multiple comparisons of data for each tick and fly species.

### Examples

### Evidence for P1 and P2X PRs on malaria mosquito mouthparts

The labellum, used for probing substrates on which the malaria mosquito feeds, is located at the tip of the mouthparts, and bears long (30 - 35 µm) terminalpore trichoid sensilla housing four chemosensory cells. One of these cells responds to sugars for nectar feeding. The inventors set out to establish whether other cells respond to stimuli and identified a cell which is particularly sensitive to ATP (detection threshold <100 pM). ATP is present in blood. The inventors therefore investigated if the capacity to sense the presence of ATP is associated with blood-feeding behaviour in the mosquito using a series of electrophysiological (EP) assays.

EP responses to a set of pertinent nucleotides, nucleosides, purines and pyrimidines **(1 - 18,** Table 1) each at 200 pM indicate how labellum sensory cells show strongest responses to adenine-containing ATP, AMP-CPP, adenosine and AMP, all at 23 to 26 spikes/2s (Figure 1A and B). ADP, a P2Y PR agonist, showed no activity, suggesting that biological activity of nucleotides is limited to one or three phosphates. Loss of OH groups on the ribose moiety, as in dATP and ddATP, strongly reduced activity. This led to prioritising P2X₂, P2X₃ and P2X_{2/3} PR agonists ATP and AMP-CPP, and P1A₁ PR agonists adenosine and AMP as best stimulants.

Action potential frequencies generated as a function of dose for adenosine, AMP and ATP indicate an increase in spike frequency from 100pM reaching an unsurmountable asymptote, typical of PRs, at 250 pM where approximately 30 spikes/2 s were recorded for all three compounds (Figure 2A and B). No difference was found between dose-response models (*drc*) generated for adenosine, AMP and ATP (all *P* >0.2) with similar ED₅₀ (~120 pM) and ED₉₅ values (~200 pM, *P* >0.05 in all cases). AMP-CPP (α,β-methylene ATP), a stronger P2X PR agonist than ATP (Coddou et al., 2011 in "Activation and Regulation of Purinergic P2X Receptor Channels", Pharmacological Reviews 63, 641-683; doi 10.1124/pr.110.003129), proved the most potent agonist. This nonhydrolysable ATP phosphonic analogue, with a methylene group substituting for oxygen between the α- and β-phosphates, shifted the EP response curve to lower doses with a steep increase in spike frequency from 10 pM to an asymptote at 80 pM (ED₉₅ of 50 pM) and an ED₅₀ at 20 pM, significantly lower than for adenosine, AMP and ATP (all *P* < 0.001; Figure 2A and B). The *drc -* slope computed for AMP-CPP was steeper compared to AMP and adenosine (*P* < 2E-16), but no different from that estimated for ATP (*P* = 0.1563), suggesting different kinetics in the binding of AMP and adenosine to a P1 PR than by AMP-CPP and ATP to a P2 PR. Superior activity of AMP-CPP and failure by the P2Y PR agonists ADP, ADP-βS and uracil nucleotides to elicit responses (Figure 1A) suggested presence of a P2X PR activated by ATP and AMP-CPP accompanied by a P1A₁ PR responding to adenosine and AMP on *A*. *gambiae* mouthparts.

Whereas ATP and adenosine alone at 50 pM hardly elicited a response, respectively 2.5 ± 0.7/2s and 1.25 ± 0.62/2s, an equimolar mixture containing 50 pM of each agonist elicited a spike frequency of 16.28 ± 4.68/2s, higher than to 100 pM of either compound presented alone (Figure 2C and D). Equimolar amounts of the two agonists were necessary since adding either 20 pM adenosine to 200pM ATP or 20 pM ATP to 200 pM adenosine did not significantly enhance the response. Presenting adenosine admixed with ATP at increasing equimolar doses to the sensillum shifted the ED₅₀ value to 86.09 ± 10.2 pM, significantly lower than for either adenosine or ATP alone (Figure 2D). Despite this, the *drc* models and ED₉₅ values estimated for ATP, adenosine and their combination are not different. The insurmountable asymptote reached for adenosine, AMP, ATP and AMP-CPP show no difference, suggesting presence of PRs that saturate in the same dose range. Yet adding either 200pM adenosine or 200pM AMP to 200pM ATP (approximately asymptote doses of each) elicited spike frequencies higher than either agonist presented alone, whereas adenosine combined with AMP had not such effect (Figure 2C and D, and Table 3). Taken together, these data indicate a P1A₁ PR activated by adenosine and AMP, and a P2X PR activated by ATP and AMP-CPP on *A. gambiae* mouthparts.

### One sensory cell with two PR types on malaria mosquito mouthparts

The inventors next addressed the question of where the PRs reside in labellum sensilla *of A. gambiae* females may be located. Action potentials recorded in response to purinergic compounds and sucrose were typically biphasic (Fig. 3A and B). Spike amplitudes elicited by ATP (2.49 mV ± 0.030, mean ± s.e.m.) and adenosine (2.57 mV ± 0.031) were the same whereas sucrose systematically elicited higher amplitude spikes (4.44 mV ± 0.047; Figure 3A and B). Firing of sensory cells with distinct receptor types in response to stimulation by two agonists generate doublets from near simultaneous firing by the cells. No doublets were found in responses recorded for ATP combined with adenosine whereas responses to ATP combined with sucrose did (Figure 3A and B). This suggests that ATP and adenosine affect PRs on the same sensory cell within mouthpart sensilla of the malaria mosquito.

**Table 3. Comparisons of mean spike frequencies (± s.e.m.) recorded from A. gambiae T1 labellum sensilla in response to stimulation with adenosine, AMP and ATP alone and combined.**

| **Agonist(s) *** | | **AMP** | **ATP** | **Adenosine + AMP** | **Adenosine + ATP** | **AMP + ATP** |
|---|---|---|---|---|---|---|
| | **Mean spike frequency per 2s (± SEM)** | **28.5 8** | **33.04** | **24.55** | **53.8** | **44.6** |
| | | **(± 4.63 )** | **(± 3.18)** | **(±2.9)** | **(± 6.59)** | **(± 5.02)** |
| **Adenosine** | 27.14 (± 2.23) | 1 | 0.9087 | 1 | **0.001** | **0.0316** |
| **AMP** | 28.58 (± 4.63) | - | 1 | 1 | **0.0033** | 0.0559 |
| **ATP** | 33.04 (± 3.18) | - | - | 0.8033 | **0.0254** | 0.3804 |
| **Adenosine + AMP** | 24.55 (±2.9) | - | - | - | **0.0025** | **0.0372** |
| **Adenosine** + **ATP** | 53.8 (± 6.59) | - | - | - | - | 1 |

| | | | | | | |
|---|---|---|---|---|---|---|
| * Each compound was tested at 200 pM alone and in binary mixtures. Treatments with spike frequencies that are significantly different have *P* - values in bold. Recording were made from between 9 and 25 sensilla per compound and binary mixtures of compounds. | | | | | | |

### Malaria mosquito PR responses are inhibited by P1 PR and P2 PR antagonists

Xanthines selectively block adenosine-sensitive PRs. One µM of the non-selective P1 PR antagonist theophylline (**27**, Table 2) reduced the EP response elicited by 200 pM adenosine by almost half from 30.23 ± 3.02 spikes/2s to 17.63 ± 4.48 for the adenosine theophylline combination (paired *t*-test, *P* = 0.0259). As ATP complexes with theophylline in solution the inventors used the high affinity and selective P1A₁ PR antagonist and theophylline-related compound 8-cyclopentyl-1,3-dimethylxanthine (CPT, **28,** Table 2) as an alternative. CPT inhibited the EP response to 200pM adenosine but not that to ATP (Figure 4A and C). Conversely, 1nM suramin (**19**, Table 2), a non-selective P2 PR antagonist, inhibited the response to ATP but not to adenosine (*P >* 0.05; Figure 4B and D). Inhibition persisted for at least 2min in both cases (Figure 4 A and B, and Figure 7 A). Use of these PR inhibitors confirmed presence of P1A₁ and P2X PRs on *A. gambiae* mouthparts.

The P2X PR expressed by the *A. gambiae* mouthpart sensory cell was further characterised using non-selective P2 PR and selective P2X PR antagonists (**19** - **26,** Table 2), some tested over several doses (Figures 5 and 6). PPADS (P2 PR antagonist) inhibited the response to ATP at an ID₅₀ of 1.42 nM, close to that of suramin (0.55 nM; Table 4). EP responses to ATP were inhibited by the P2X₂ PR antagonist RB-2, the P2X₃ PR antagonists TMP and spinorphin, and by the P2X₃ / P2X_{2/3} PR antagonist A-317491 but not by antagonists for other P2X PRs (Figures 5, 6 and 7, Table 4). The most effective antagonists were spinorphin and A-317491 (matching ID₉₅ values at ca. 0.4 nM; Table 1), suggesting a P2X₃/ P2X_{2/3} PR on *A. gambiae* mouthpart sensilla. P2, P2X₃ and P2X_{2/3} PR antagonists, but not the P2X₂ PR antagonist RB-2, induced persistent antagonism to ATP presented to the sensillum approximately 2 min later (Figures 5 and 7). Response curves for these antagonists, except that for suramin, indicate resistance to inhibition, characteristic of PRs, when spike counts reach ca. 10/2 s (Figures 5 and 6).

**Table 4. Neuropharmacological effects of P2 PR antagonists on responses of A. gambiae mouthpart ATP-sensitive receptor.**

| **PR antagonist** | **ID₅₀ in nM¹** | **ID₉₅ in nM¹** | **Antagonist dose [nM]** | **Mean no. of spikes per 2s (± s.e.m.)** | | **p-values** |
|---|---|---|---|---|---|---|
| | **(± s.e.m.)** | **(± s.e.m.)** | | **ATP [200pM]** | **ATP + antagonist²** | |
| **Suramin (P2)** | 0.55 ± 0.06 | 1.15 ± 0.46 | 0.6 | 20.60 ± 2.73 | 4.20 ± 1.32 | *0.0011* |
| **PPADS (P2)** | 1.42 ± 1.2 | 12.66 ± 24.05 | 50 | 21.63 ± 3.09 | 8.13 ± 2.69 | ***0.0253*** |
| **MRS 2159 (P2X₁)** | No block (1 µM) | | 1000 | 32.71 ± 6.54 | 27.14 ± 8.95 | *0.5339* |
| **RB-2 (P2X₂)** | 1.04 ± 1.11 | 113.02 ± 247.77 | 50 | 27.67 ± 2.63 | 12.83 ± 3.35 | ***0.0038*** |
| **A-317491 (P2X₃, P2X_{2/3})** | 0.32 ± 0.33 | 0.41 ± 0.51 | 0,5 | 34.17 ± 4.95 | 7.5 ± 2.29 | ***0.0033*** |
| **TMP(P2X₃)** | 12.17 ± 6.61 | 27.50 ± 46.49 | 10 | 31.40 ± 1.17 | 11.2 ± 4.86 | ***0.0203*** |
| **Spinorphin (P2X₃)** | 0.34 ± 0.47 | 0.43 ± 0.36 | 1 | 29. ± 5.26 | 9.8 ± 4.43 | ***0.0426*** |
| **Paroxetine (P2X₄)** | No block (2 µM) | | 2000 | 27 ± 6.01 | 27.6 ± 3.23 | 1.0 |
| **A-438079 (P2X₇)** | No block (1 µM) | | 1000 | 27. 57± 4.16 | 22.57 ± 6.77 | *0.843* |

| | | | | | | |
|---|---|---|---|---|---|---|
| ¹ ID₅₀ and ID₉₅ values in nM (unless otherwise stated) for PR antagonists were estimated by testing them at increasing doses admixed to 200 pM ATP that served as positive control (see Figures 5 and 6). The maximum dose tested is provided when an antagonist failed to inhibit. ² Mean spike frequencies provided for 200 pM ATP + antagonist is for an antagonist dose near its estimated ID₉₅ value (column 4); *P* - values indicating significant reductions in spike frequency compared to 200 pM ATP are indicated in bold. Treatment repetitions (n) are reported in Materials and Methods. | | | | | | |

### Malaria mosquito P2X PR responses are modulated by metal ions

Essential trace metals like Zn²⁺ and macrometals like Mg²⁺ modulate vertebrate P2X PRs via extracellular binding sites (Coddou et al., *supra).* The crystal structure and EP responses of AmP2XR from the Gulf Coast tick *Amblyomma maculatum* indicate it functions as an ATP-gated cation channel with two extracellular metal-binding sites, M1 and M2 (Kasuya et al., 2016 in "Structural Insights into Divalent Cation Modulations of ATP-Gated P2X Receptor Channels", Cell Reports 14, 932-44; doi 10.1016/i.celrep.2015.12.087). The M1 site located at the trimer interface permits allosteric potentiation by Zn²⁺ of *Am*P2XR responses to ATP. LsP2X of the pond snail *Lymnaea stagnalis* with a similar M1 site is likewise potentiated by Zn²⁺. The inventors established through EP recordings from *A. gambiae* labellum sensilla that 200pM Zn²⁺ admixed with 100pM ATP induced a 5-fold increase in spike frequency but 200pM Zn²⁺ had no such effect at the asymptote dose of 200 pM ATP (Table 5). The M2 site on AmP2XR is associated with the ATP binding site and subject to modulation by Mg²⁺, reducing ATP receptor affinity at higher Mg²⁺ levels (Kasuya et al., *supra).* The inventors found that adding 200pM Mg²⁺ to 200pM ATP reduced spike frequency recorded from *A. gambiae* labellum sensilla by half compared to 200pM ATP alone (Table 5). These findings suggest the P2X PR *of A. gambiae* shares characteristics with vertebrate, tick, and snail P2X PRs.

**Table 5. Effects of metals ions on electrophysiological responses of A. gambiae labellum ATP-sensitive receptor.**

| **Treatment** | **Mean no. of spikes per 2 s (± s.e.m.) *** | ***P*-value** |
|---|---|---|
| 100 pM ATP | 3 ± 1.67 | *P* = 0.008 compared to 100 pM ATP |
| 200 pM Zn²⁺ + 100 pM ATP | 15.4 ± 3.72 | |
| 200 pM ATP | 24.8 ± 4.84 | *P* = 0.89 compared to 200 pM ATP |
| 200pM Zn²⁺ + 200 pM ATP | 24.0 ± 3.41 | |
| 200 pM ATP | 28.12 ± 3.5 | *P* = 0.0049 compared to 200 pM ATP |
| 200 pM Mg²⁺ + 200 pM ATP | 12 ± 4.13 | |

| | | |
|---|---|---|
| * Treatment repetitions (n) are reported in Materials and Methods. | | |

### Feeding by biting insects and ticks is inhibited by P1 PR and P2 PR antagonists

Considering the sensitivity shown by *A*. *gambiae* peripheral neurones for adenosine, adenine nucleotides and PR antagonists, the inventors set out to test the effects of these compounds on feeding by mosquitoes, biting flies and ticks. Adding ATP to the feeding solution induced dose dependent feeding from 1 µM ATP with an ED₅₀ at 6.54 µM and an asymptote at 45 µM where 70.25 % mosquitoes fed (the ED₉₅ at 22.5 µM for 68.15 % feeding was rounded to 20 µM for feeding assays; Figure 8A and Table 6). AMP-CPP at 20 µM proved a better feeding stimulant, eliciting 84.35 % feeding though not significantly different to ATP (Table 6). AMP and adenosine each presented at 20 µM elicited increased feeding albeit to a significantly lower extent than 20 µM ATP, whereas 1mM adenosine induced an equivalent level of feeding as on 20 µM ATP. No effect on feeding higher than on 20 µM ATP was recorded when 20 µM ATP was admixed with 200 µM adenosine (Table 6). The increase in feeding induced by the P2Y PR agonist ADP-βS was not significantly different to that of the feeding solution (Table 6).

**Table 6. Proportion of A. gambiae feeding on the feeding solution, on nucleotides and adenosine in the feeding solution, and on the P1A₁ PR antagonist CPT and the P2X₃ PR antagonist TMP admixed with ATP and adenosine in the feeding solution. Different experimental series are separated by a blank line.**

| **Treatment** | **% Engorged mosquitoes** | **n*** | **P-value** | | |
|---|---|---|---|---|---|
| | | | **compared to FS **** | **Compared to** | |
| Feeding solution (FS **) | 24.1 ± 1.6 | 71 | -- | -- | -- |
| 20 µM ATP | 67.5 ± 2.1 | 39 | 4.08E-16 | -- | -- |
| 20 µM AMP-CPP | 84.3 ± 6.8 | 4 | 4.33E-12 | ATP 20 µM | 0.0956 |
| 20 µM Adenosine | 39.1 ± 5.7 | 10 | 0.0089 | ATP 20 µM | 6.11E-04 |
| 1 mM Adenosine | 64.4 ± 3.2 | 17 | 1.70E-17 | ATP 20 µM | 1 |
| 20 µM ATP + 200 µM Adenosine | 60.1 ± 4.5 | 4 | 7.53E-06 | ATP 20 µM | 0.4831 |
| 20 µM AMP | 38.3 ± 5.1 | 10 | 0.0110 | ATP 20 µM | 3.40E-04 |
| 20 µM ADP-βS | 37.8 ± 4.1 | 4 | 0.0956 | ATP 20 µM | 0.0191 |
| 20 µM ATP + 10 µM CPT | 65.4 ± 10.2 | 5 | 1.08E-07 | ATP 20 µM | 1 |
| 1 mM Adenosine + 10 µM CPT | 48.7 ± 7.0 | 6 | 7.02E-05 | Adenosine 1 mM | 0.0146 |
| Feeding solution (FS **) | 14.1 ± 2.6 | 8 | -- | -- | -- |
| 20 µM ATP + 200 µM Adenosine | 46.2 ± 3.4 | 18 | 1.20E-05 | -- | -- |
| 20 µM ATP + 200 µM Adenosine + 7.5 µM TMP + 20 µM CPT | 15.3 ± 2.8 | 16 | 0.3961 | 20 µM ATP + 200 µM Adenosine | 2.32E-07 |
| 20 µM ATP + 200 µM Adenosine + 7.5 µM TMP | 36.7 ± 6.0 | 11 | 0.0117 | 20 µM ATP + 200 µM Adenosine | 0.1499 |
| | | | | 20 µM ATP + 200 µM Adenosine + 7.5 µM TMP + 20 µM CPT | 0.0116 |

| | | | | | |
|---|---|---|---|---|---|
| * n =number of cages for each experimental situation;** FS =feeding solution (buffered NaCl/dextrose). | | | | | |

PR antagonists identified by EP recordings could act on PRs to inhibit feeding. 10 µM CPT, the selective P1A₁ PR antagonist, significantly inhibited A. *gambiae* feeding on 1 mM adenosine but not on 20 µM ATP added to the feeding solution (Table 6). Testing P2 PR antagonists over several doses shows how suramin, PPADS, RB-2, TMP, spinorphin and A-317491 inhibited feeding on a solution containing the feeding stimulant ATP (Figures 8B and 9, Table 7). *A. gambiae* feeding proved most sensitive to TMP (ID₅₀ 15 nM) and A-317491 (ID₅₀ 0.24 nM; Figures 8B and 9, Table 7). Except for the P2X₇ PR antagonist A-438079, antagonists for other P2X PR subtypes were less effective (Table 7).

**Table 7. Effect of P2 PR antagonists on A. gambiae feeding on a feeding solution containing ATP.**

| **PR antagonist** | **ID₅₀ in nM (± s.e.m) *** | **ID₉₅ in µM (± s.e.m.) *** | **% fed mosquitoes (mean ± s.e.m.)** | | | **P-value** |
|---|---|---|---|---|---|---|
| | | | **ATP [20µM]** | **Antagonist dose tested** | **ATP + antagonist** | |
| Suramin (P2) | 735 ± 56 | 1.36 ± 0.30 | 67.6 ± 1.92 | 1 µM | 34.86 ± 4.49 | **0.00001** |
| PPADS (P2) | 42.84 ± 111.58 µM | # | 66.12 ± 3.67 | 100 µM | 33.39 ± 3.49 | **0.0001** |
| MRS 2159 (P2X₁) | No feeding inhibition (20 µM) | | 65.96 ± 3.1 | 20 µM | 79.19 ± 6.43 | 0.0633 |
| RB-2 (P2X₂) | 100.55 ± 92.48 | # | 64.49 ± 3.56 | 0.1 µM | 32.88 ± 4.53 | **0.0004** |
| A-317491 (P2X₃ / P2X_{2/3}) | 0.24 ± 1.36 | # | 69.12 ± 1.59 | 1nM | 43.71 ± 6.5 | **0.0005** |
| TMP(P2X₃) | 15.47 ± 10.58 | 1.28 ± 3.00 | 69.68 ± 2.52 | 10 nM | 50.47 ± 6.80 | **0.0084** |
| Spinorphin (P2X₃) | (100 nM, 27.31%) | | 64.2 ± 3.74 | 100 nM | 46.62 ± 6.79 | **0.0469** |
| Paroxetine (P2X₄) | No feeding inhibition (20 µM) | | 70.2 ± 5.7 | 20 µM | 62.72 ± 6.63 | 0.1624 |
| A-438079 (P2X₇) | (20 µM, 30.74%) | | 70.2 ± 5.7 | 20 µM | 48.65 ± 2.7 | **0.0005** |

| | | | | | | |
|---|---|---|---|---|---|---|
| * ID₅₀ values in nM and ID₉₅ values µM (unless otherwise stated) for antagonists were estimated by testing their effects at increasing doses on *A. gambiae* feeding on 20µM ATP (positive control) in the feeding solution (cf. Figures 8 and 9). # The log logistic LL.4 model did not permit a reliable estimate of the ID₉₅ for PPDAS, RB-2 and A-317491. Data for the maximum dose tested is provided when an antagonist failed to inhibit feeding. For spinorphin and A438079 (not tested over several doses) the % reduction in feeding is provided for the dose indicated. The four columns on the right present, respectively, the proportion of mosquitoes feeding on 20 µM ATP (cf. Table 6), the dose of antagonist tested nearest the ID₅₀ value, the proportion of mosquitoes feeding on that dose of antagonist admixed with 20 µM ATP, and the probability level for the comparison of the two proportions (significant values are in bold). Treatment repetitions (n) are reported in Materials and Methods. | | | | | | |

20 µM CPT with 7.5 µM TMP added to the feeding solution inhibited the feeding stimulant effect of the 20 µM ATP plus 200 µM adenosine combination to a level no different to that of the feeding solution, and this combination of P1A₁ PR and P2X₃ PR antagonists proved a better feeding inhibitor for the 20 µM ATP plus 200 µM adenosine combination in the feeding solution than 7.5 µM TMP alone (Figure 10, Table 6). It is noteworthy how even the highest doses of PR antagonists tested only reduced feeding to levels corresponding to that on the feeding solution tested as negative control, allowing the inventors to conclude that the action of PR antagonists is on purine-sensitive cells.

Another anopheline mosquito, *A. stephensi,* also responded as a function of dose to ATP as a feeding stimulant with an ED₉₅ value at 160 µM ATP (Table 8). The P2 PR antagonists suramin and PPADS, the P2X₃ PR antagonist TMP and the P2X₃/ P2X_{2/3} PR antagonist A-317491 all inhibited feeding by *A. stephensi* on the feeding solution containing ATP in a pattern like that recorded for *A. gambiae* where the P2X₃ P2X_{2/3} PR antagonists TMP and A-317491 again induced the strongest inhibition (Table 8).

**Table 8. Effect of P2 PR antagonists on A. stephensi feeding on a feeding solution containing ATP.**

| **Treatment** | **ATP** | **Suramin (P2)** | **PPADS (P2)** | **A-317491 (P2X₃ / P2X_{2/3})** | **TMP (P2X₃)** |
|---|---|---|---|---|---|
| Dose | 160 µM | 80 µM | 800 µM | 0.08 µM | 0.8 µM |
| **% Fed females (mean ± s.e.m.)** | 72.96 ± 5.9 | 30.04 ± 7.75 | 37.29 ± 3.75 | 29.68 ± 7.14 | 26.67 ± 2.98 |
| **% Inhibition** | - | 58.83 | 48.89 | 59.32 | 63.45 |
| ***P*-value** * | - | **0.0060** | **0.0066** | **0.0060** | **0.0027** |

| | | | | | |
|---|---|---|---|---|---|
| * Treatment effects were compared to the positive control (feeding solution with 160 µM ATP added). Treatment repetitions (n) are reported in Materials and Methods. | | | | | |

In feeding experiments, suramin inhibited feeding on a solution containing ATP by two hard tick spp. *Ixodes ricinus* and *Amblyomma hebraeum* and by the soft tick *Ornithodorus moubata,* and A-317491 inhibited feeding by *I. ricinus* and O. *moubata* (Table 9). Suramin and A-317401 likewise inhibited feeding by the tsetse fly *Glossina pallidipes* and horn fly *Haematobia irritans,* and TMP inhibited feeding by the horn fly on solutions containing ATP (Table 10). TMP in solution failed to inhibit feeding by the soft tick and tsetse, yet when applied onto the feeding membrane it significantly inhibited feeding by both blood feeders (Tables 9 and 10), suggesting that antagonism of peripheral PRs is sufficient to inhibit feeding.

**Table 9. Effect of P2 PR antagonists on feeding by three tick species on a feeding solution containing ATP.**

| **Solution containing ATP** | **Life stage** | **Feedin g metho d** | **Treatments** | **Fluid intake or weight gain** | | **n (numb er of ticks)** | ***P-*value ^{#}** |
|---|---|---|---|---|---|---|---|
| | | | | **Median (mg) ⁺** | **Range** | | |
| Ixodes ricinus | females | glass capillary | TFS * | 2.6 | 1-8 | 16 | - |
| | | | TFS + 500 µM Surami n | 1.9 | 1- 8 | 14 | **0.0213** |
| | | | TFS + 1 µM A-317491 | 1.5 | 1-3 | 12 | 0.0122 |
| Amblyomma hebraeum | females | glass capillary | TFS | 11.5 | 3-28 | 10 | -- |
| | | | TFS + 500 µM Surami n | 4.5 | 2-14 | 10 | **0.0225** |
| | | | TFS | 15 | 1-46 | 11 | -- |
| | | | TFS + 1 µM A-317491 | 6.7 | 1-99 | 10 | 0.1363 |
| Ornithodorus moubata | N3, N4 | Silicone membrane | TFS | 0.3 | 1-63 | 231 | -- |
| | | | TFS + 500 µM Surami n | 0 | 3-6 | 40 | **0** |
| | | | TFS + 1 µM A-317491 | 0.1 | 4-52 | 21 | 0.7436 |
| | | | **TFS** + **100** µM A-317491 | 0 | 4-22 | 20 | **0.0183** |
| | | | TFS + 100 µM TMP | 0.1 | 4-29 | 77 | 0.1702 |
| | | | TTFS + 8 µg/cm² TMP on membrane | 0 | 4-10 | 19 | **0.0024** |

| | | | | | | | |
|---|---|---|---|---|---|---|---|
| * TFS: tick feeding solution containing 100 mM NaCl + 8.9 mM NaHCO3 + 1000 µM ATP as positive control; + Feeding solution intake in mg/h for hard ticks and weight gain in mg/25 min for soft ticks; # Within species, treatments effects significantly different to the TFS are in bold. | | | | | | | |

**Table 10. Effect of P2 PR antagonists on feeding by two biting fly species on a feeding solution containing ATP.**

| **Fly species** | **Treatment** | **% Fed** | | **n (number of flies)** | **p-value ^{#}** |
|---|---|---|---|---|---|
| | | **Median** | **Range** | | |
| *Glossina pallidipes* | FFS * | 63.6 | 30-100 | 41 | - |
| | FFS + 50 µM Suramin | 45.5 | 0-91 | 17 | **0.0009** |
| | FFS + 1 µM A-317491 | 22.5 | 0-90 | 20 | **0.0015** |
| | FFS + 150 µM TMP | 66.7 | 40-92 | 13 | 0.8551 |
| | FFS + 16 µg/cm² TMP on membrane | 40 | 8-70 | 9 | **0.0261** |
| *Haematobia irritans* | FFS | 66.7 | 38-100 | 31 | - |
| | FFS + 50 µM Suramin | 61.5 | 31-100 | 17 | 0.1888 |
| | FFS + 100 µM Suramin | 46.2 | 9-67 | 15 | **0.0020** |
| | FFS + 10 µM A-317491 | 58.3 | 25-86 | 16 | 0.1790 |
| | FFS + 100 µM A-317491 | 41.5 | 27-78 | 7 | **0.0193** |
| | FFS + 50 µM TMP | 50 | 0-80 | 11 | **0.0114** |
| | FFS + 100 µM TMP | 37.5 | 18-38 | 3 | **0.0162** |
| | FFS + 500 µM TMP | 0 | 0 | 8 | **0.0024** |

| | | | | | |
|---|---|---|---|---|---|
| * FFS: fly feeding solution containing 150 mM NaCl + 8.9 mM NaHCO3 + 100 µM ATP as positive control; # Within species, treatment effects significantly different to the FFS are in bold. | | | | | |

Evidence is presented herein for P1A₁ and P2X₃/P2X_{2/3} PRs on mouthparts of blood-feeding arthropods. The P2X PR is particularly sensitive to selective P2X₃ / P2X_{2/3} PR antagonists A-319471, spinorphin and TMP that inhibit feeding by all blood-feeding insects and ticks tested herein. The characteristics of the P2X₃ / P2X_{2/3} PRs described by the inventors show similarities to vertebrate homomeric P2X₃ PRs and heteromeric P2X_{2/3} PRs found on unmyelinated C-fibres and on thinly myelinated Aδ-sensory fibres that transmit pain. Such nociceptive fibres and their first relay neurons in dorsal root ganglia (DRG) show high expression levels of P2X₃ PRs (Burnstock and Verkhratsky, 2012 in "Purinergic signalling and the nervous system", Springer-Verlag Berlin Heidelberg; doi 10.1007/978-3-642-28863-0). In contrast to P2X₃ PRs, slowly desensitising P2X_{2/3} PRs maintain currents in response to repeated stimulation (Coddou et al., 2011, *supra).* The inventors recorded little adaptation in response to repeated presentation of ATP *to A. gambiae* mouthpart sensilla at intervals varying from tens of seconds to 6 minutes. Vertebrate P2X₃ and P2X_{2/3} PRs show high affinity for AMP-CPP and ATP, at <1 nM for the latter, and are readily antagonised by A-317491 (K_{d} 0.9 nM; Jarvis et al., 2002, *supra).* The antinocicepive spinorphin, an endogenous heptapeptide inhibitor of encephalin-degrading enzyme, antagonises human P2X₃ PR at an ID₅₀ of 8.3 pM.

During blood feeding, ATP and ADP are hydrolysed by *A*. *gambiae* salivary apyrase to prevent platelet aggregation for wound healing. This results in adenosine being presented to mouthpart PRs. The inventors report how equimolar combinations of ATP and adenosine elicit spike frequencies significantly higher than either agonist presented singly. This shifts the threshold to lower doses of the agonist mix and the response surpasses the unsurmountable asymptote reached at higher doses of either agonist alone, leading to a wider dose range over which the PRs respond. Mouthpart PRs of *A. gambiae* are eminently equipped to detect physiologically operating levels of both ATP and adenosine, and as disclosed herein, can be readily inhibited by PR antagonists.

Even though the inventors tested and confirmed the usefulness of PR antagonists in the protection against arthropod parasites using only a number of arthropod parasite species, this invention is not limited to the arthropod species tested herein.

## Claims

1. A product comprising one or more purinergic receptor (PR) antagonist for use in prevention and/or treatment of parasitic infestation of a human and/or animal host by an arthropod parasite by exposing the arthropod parasite to one or more PR antagonist, or by contacting the arthropod parasite with one or more PR antagonist.

2. Product comprising one or more purinergic receptor (PR) antagonist for use in prevention and/or treatment of parasitic infestation of a human and/or animal host according to claim 1, wherein the one or more PR antagonist inhibits the arthropod parasite from feeding on host tissue, including blood.

3. Product for use in prevention and/or treatment of parasitic infestation of a human and/or animal host by an arthropod parasite according to claim 1 or 2, wherein the product comprises at least one of
(i) a P1 PR antagonist, or
(ii) a P2X PR antagonist selected from a group consisting of P2X₂, P2X₃, or a P2X_{2/3} PR antagonist,
(iii) or a combination thereof.

4. Product for use in prevention and/or treatment of parasitic infestation of a human and/or animal host by an arthropod parasite according to any of claims 1 to 3, wherein the one or more P1 PR antagonist is a xanthine, such as 1,3-dimethylxanthine or 8-cyclopentyl-1,3-dimethylxanthine.

5. Product for use in prevention and/or treatment of parasitic infestation of a human and/or animal host by an arthropod parasite according to any of claims 1 to 4, wherein the one or more P2X₂, P2X₃, or P2X_{2/3} PR antagonist is selected from a group consisting of a polysulfonated naphthylamine such as suramin, pyridoxal phosphate-6-azophenyl-2,4-disulfonate ("PPADS"), an anthraquinone-sulfonic acid compound such as 1-amino-4-[[4-[[4-chloro-6-[[3 (or 4)-sulfophenyl]amino]-1,3,5-triazin-2-yl]amino]-3-sulfophenyl]amino]-9,10-dihydro-9,10-dioxo-2-anthracenesulfonic acid ("Reactive Blue-2"), a tricarboxylate such as 5-[(3-phenoxyphenyl)methyl-[(1S)-1,2,3,4-tetrahydronaphthalen-1-yl]carbamoyl]benzene-1,2,4-tricarboxylic acid ("A-317491"), a pyrazine alkaloid such as 2,3,5,6-tetramethylpyrazine, and a heptapeptide such as spinorphin.

6. Product for use in prevention and/or treatment of parasitic infestation of a human and/or animal host by an arthropod parasite according to any of claims 1 to 5, comprising suramin and 8-cyclopentyl-1,3-dimethylxanthine, or suramin and 2,3,5,6-tetramethylpyrazine, or suramin and 1,3-dimehtylxanthine, or suramin and a tricarboxylate such as 5-[(3-phenoxyphenyl)methyl-[(1S)-1,2,3,4-tetrahydronaphthalen-1-yl]carbamoyl]benzene-1,2,4-tricarboxylic acid ("A-317491"), or suramin and spinorphin, or comprising suramin and 2,3,5,6-tetramethylpyrazine and 8-cyclopentyl-1,3-dimethylxanthine, or suramin and 2,3,5,6-tetramethylpyrazine and 1,3-dimethylxanthine, or suramin and spinorphin and 8-cyclopentyl-1,3-dimethylxanthine, or suramin and spinorphin and 1,3-dimethylxanthine, or suramin and 2,3,5,6-tetramethylpyrazine and A-317491, or comprising 2,3,5,6-tetramethylpyrazine and 8-cyclopentyl-1,3-dimethylxanthine, or 2,3,5,6-tetramethylpyrazine and 1,3-dimethylxanthine, or 2,3,5,6-tetramethylpyrazine and A-317491, or comprising pyridoxal phosphate-6-azophenyl-2,4-disulfonate and 8-cyclopentyl-1,3-dimethylxanthine, or pyridoxal phosphate-6-azophenyl-2,4-disulfonate and 1,3-dimehtylxanthine, or comprising Reactive Blue-2 and 8-cyclopentyl-1,3-dimethylxanthine, or Reactive Blue-2 and 1,3-dimethylxanthine, or comprising A317491 and 8-cyclopentyl-1,3-dimethylxanthine, or A317491 and 1,3-dimethylxanthine, or comprising spinorphin and 8-cyclopentyl-1,3-dimethylxanthine, or spinorphin and 1,3-dimethylxanthine.

7. Product for use in prevention and/or treatment of parasitic infestation of a human and/or animal host by an arthropod parasite according to any of claims 1 to 6, further comprising at least one PR metal modulator.

8. Product for use in prevention and/or treatment of parasitic infestation of a human and/or animal host by an arthropod parasite according to any of claims 1 to 7, wherein the product is directed against blood-feeding arthropod ectoparasites, for example against biting insects such as mosquitoes, sandflies, tsetse flies, black flies, tabanid horse and deer flies, stable flies, horn flies, midges, lice, bed bugs, triatomine bugs, fleas, and/or against mites or ticks, such as species of the group of the Ixodidae or the Argasidae, or against crustacean ectoparasites.

9. Product for use in prevention and/or treatment of parasitic infestation of a human and/or animal host by an arthropod parasite according to any of claims 1 to 8, wherein the product is directed against arthropod endoparasites, such as an endoparasitic mite, a crustacean endoparasite, endoparasitic larvae of the botfly families Cuterebridae and Hypodermatidae, endoparasitic gadflies, endoparasitic flies of the genus *Hypoderma,* or endoparasitic flies of the families Calliphoridae, Muscidae and Sacrophagidae.

10. Product for use in prevention and/or treatment of parasitic infestation of a human and/or animal host by an arthropod parasite according to any of claims 1 to 9, wherein the product further comprises an insecticide, insecticide synergist, an acaricide, an endectocide, a parasiticide, a repellent, a chemosterilant, and/or a surfactant.

11. Product for use in prevention and/or treatment of parasitic infestation of a human and/or animal host by an arthropod parasite according to any of claims 1 to 10, preventing or reducing the spread of diseases transmitted by arthropod ectoparasite disease vectors, such as
- diseases transmitted by biting insects, for example malaria, trypanosomiasis, surra, leishmaniasis, onchocerciasis, filariasis, dengue fever, yellow fever, chikungunya, plague, lumpy skin disease, and diseases caused by Zika virus, West Nile virus, *Bacillus anthracis,* and Bluetongue virus, and/or
- diseases transmitted by ticks, for example theileriosis, babesiosis, anaplasmosis, heartwater disease, Lyme borreliosis, bovine borreliosis, relapsing fever, tularaemia, Q-fever, Crimean-Congo haemorrhagic fever, tickborne encephalitis and louping-ill,
by exposing the arthropod disease vector to one or more PR antagonist, or by contacting an arthropod parasite with one or more PR antagonist.

12. Product for use in prevention and/or treatment of parasitic infestation of a human and/or animal host by an arthropod parasite according to any of claims 1 to 11, wherein the product is provided as a powder or of a liquid composition, preferably a water dispersible emulsion or a water-based solution.

13. Product for use in prevention and/or treatment of parasitic infestation of a human and/or animal host by an arthropod parasite according to any of claims 1 to 12, wherein the product is suitable for topical application onto humans and/or host animals, for application onto any solid surface, for spraying and/or for soaking of a fabric, such as clothing, a bed net, or a cover for a host animal.

14. A pharmaceutical composition comprising the product for use in prevention and/or treatment of parasitic infestation of a human and/or animal host by an arthropod parasite according to any of claims 1 to 13.

15. A screening method for selecting an antagonist for a PR of an arthropod parasite comprising:
- Arranging at least one arthropod test animal such as to expose at least one animal cell expressing one or more PRs, or at least one portion of said test animal comprising one or more purine-sensitive cells for electrophysiological measurement, or arranging a tissue or an organoid of vertebrate or invertebrate origin expressing one or more PR to determine cellular activity,
- Stimulating the one or more PRs carried on the animal cell, on the animal portion, or on the tissue or organoid of vertebrate or invertebrate origin comprising the one or more PR by presenting a fixed dose of at least one PR agonist,
- Determining the response of the animal cell, of the animal portion comprising the one or more PR, or of the tissue or organoid of vertebrate or invertebrate origin comprising the one or more PR over a defined period during stimulation, for example by determining the action potential frequency,
- Presenting a test compound in combination with the at least one PR agonist to the one or more PR carried on the animal cell, on the animal portion, or on the tissue or organoid of vertebrate or invertebrate origin expressing the one or more PR,
- Determining the response of the one or more PR carried on the animal cell, on the animal portion, or on the tissue or organoid of vertebrate or invertebrate origin over a defined period during presentation of the test compound together with the at least one PR agonist, for example by determining the action potential frequency,
- Comparing the response of the animal cell, of the at least one animal portion comprising the one or more PR, or of the tissue or organoid of vertebrate of invertebrate origin comprising the one or more PR in absence of the test compound to the response of the animal cell, of the animal portion, or of the tissue or organoid of vertebrate or invertebrate origin when presented with the test compound,
- Determining the suitability of the test compound for use as a PR antagonist based on the result of this comparison.

16. A screening method for determining the ability of a PR antagonist to prevent infestation of a human and/or animal host by an arthropod parasite comprising:
- Arranging at least one arthropod test animal such as to expose at least one animal cell expressing one or more PRs or at least one portion of said arthropod test animal comprising one or more purine-sensitive cells to a feeding medium containing a fixed dose of at least one PR agonist as feeding stimulant, or to expose the at least one animal cell or the at least one portion of said arthropod test animal to a substrate such as a membrane or micro reservoir placed over or anywhere around a feeding medium containing a fixed dose of at least one PR agonist as feeding stimulant,
- Determining the feeding rate of said arthropod test animal over a defined period in presence of the at least one PR agonist in the feeding medium, or determining the feeding rate over a defined period of said arthropod test animal exposed to least one test compound applied onto the membrane or to the micro reservoir placed over or anywhere around the feeding medium,
- Presenting at least one test compound in combination with the at least one PR agonist as feeding stimulant to said animal cell or to said at least one portion of said arthropod test animal in the feeding medium, or presenting at least one test compound applied onto the membrane or onto the micro reservoir placed over or anywhere around the feeding medium,
- Determining the feeding rate of said arthropod test animal over a defined period in presence of the at least one PR agonist and the at least one test compound in the feeding medium, or determining the feeding rate of said arthropod test animal over a defined period in presence of the at least one test compound applied onto the membrane or onto the micro reservoir placed over or anywhere around the feeding medium,
- Comparing the feeding rates of said arthropod test animal in the presence and in the absence of the at least one test compound;
- Determining the suitability of the at least one test compound for use as a feeding inhibitor based on the result of this comparison.

## Patentansprüche

1. Ein Produkt umfassend einen oder mehrere purinergen Rezeptor (PR)-Antagonisten zur Verwendung bei der Vorbeugung und/oder der Behandlung eines Parasitenbefalls eines menschlichen und/oder tierischen Wirtes durch einen parasitären Arthropoden, indem der parasitäre Arthropode einem oder mehreren PR-Antagonisten ausgesetzt oder indem der parasitäre Arthropode mit einem oder mehreren PR-Antagonisten in Kontakt gebracht wird.

2. Das Produkt umfassend einen oder mehrere purinergen Rezeptor (PR)-Antagonisten zur Verwendung bei der Vorbeugung und/oder Behandlung eines Parasitenbefalls eines menschlichen und/oder tierischen Wirtes nach Anspruch 1, wobei der eine oder mehrere PR-Antagonist den parasitären Arthropoden daran hindert, sich von Wirtsgewebe, einschließlich Blut, zu ernähren.

3. Das Produkt zur Verwendung bei der Vorbeugung und/oder Behandlung eines Parasitenbefalls eines menschlichen und/oder tierischen Wirtes nach Anspruch 1 oder 2, wobei das Produkt mindestens eines der folgenden Stoffe aufweist:
(i) einen P1 PR-Antagonisten oder
(ii) einen P2X PR-Antagonisten, ausgewählt aus einer Gruppe bestehend aus P2X₂, P2X₃ oder P2X_{2/3} PR-Antagonisten,
(iii) oder eine Kombination davon.

4. Das Produkt zur Verwendung bei der Vorbeugung und/oder Behandlung eines Parasitenbefalls eines menschlichen und/oder tierischen Wirtes nach einem der Ansprüche 1 bis 3, wobei einer oder mehrere der P1 PR-Antagonisten ein Xanthin, wie beispielsweise 1,3-Dimethylxanthin oder 8-Cyclopentyl-1,3-dimethylxanthin, ist.

5. Das Produkt zur Verwendung bei der Vorbeugung und/oder Behandlung eines Parasitenbefalls eines menschlichen und/oder tierischen Wirtes nach einem der Ansprüche 1 bis 4, wobei der eine oder mehrere P2X₂, P2X₃ oder P2X_{2/3} PR-Antagonisten ausgewählt ist/sind aus einer Gruppe bestehend aus einem polysulfonierten Naphthylamin, wie zum Beispiel Suramin, Pyridoxalphosphat-6-azophenyl-2,4-disulfonat ("PPADS"), einer Anthrachinonsulfonsäureverbindung wie 1-Amino-4-[[4-[[4-chlor-6-[[3 (oder 4)-sulfophenyl]amino]-1,3,5-triazin-2-yl]amino]-3-sulfophenyl]amino]-9,10-dihydro-9,10-dioxo-2-anthracensulfonsäure ("Reactive Blue-2"), ein Tricarboxylat wie 5-[(3-Phenoxyphenyl)methyl-[(1S)-1,2,3,4-tetrahydronaphthalin-1-yl]carbamoyl]benzol-1,2,4-tricarbonsäure (" A-317491"), einem Pyrazin-Alkaloid wie 2,3,5,6-Tetramethylpyrazin und einem Heptapeptid, wie zum Beispiel Spinorphin.

6. Das Produkt zur Verwendung bei der Vorbeugung und/oder Behandlung eines Parasitenbefalls eines menschlichen und/oder tierischen Wirtes nach einem der Ansprüche 1 bis 5, umfassend Suramin und 8-Cyclopentyl-1,3-dimethylxanthin, oder Suramin und 2,3,5,6-Tetramethylpyrazin, oder Suramin und 1,3-Dimethylxanthin, oder Suramin und ein Tricarboxylat wie 5-[(3-Phenoxyphenyl)methyl-[(1S)-1,2,3,4-tetrahydronaphthalin-1-yl] carbamoyl]benzol-1,2,4-tricarbonsäure ("A-317491"), oder Suramin und Spinorphin, oder umfassend aus Suramin und 2,3,5,6-Tetramethylpyrazin und 8-Cyclopentyl-1,3-dimethylxanthin, oder Suramin und 2,3,5,6-Tetramethylpyrazin und 1,3-Dimethylxanthin, oder Suramin und Spinorphin und 8-Cyclopentyl-1,3-dimethylxanthin, oder Suramin und Spinorphin und 1,3-Dimethylxanthin, oder Suramin und 2,3,5,6-Tetramethylpyrazin und A-317491, oder umfassend 2,3,5,6-Tetramethylpyrazin und 8-Cyclopentyl-1,3-dimethylxanthin, oder 2,3,5,6-Tetramethylpyrazin und 1,3-Dimethylxanthin, oder 2,3,5,6-Tetramethylpyrazin und A-317491, oder umfassend Pyridoxalphosphat-6-azophenyl-2,4-disulfonat und 8-Cyclopentyl-1,3-dimethylxanthin, oder Pyridoxal Phosphat-6-azophenyl-2,4-disulfonat und 1,3-Dimethylxanthin, oder umfassend Reactive Blue-2 und 8-Cyclopentyl-1,3-dimethylxanthin, oder Reactive Blue-2 und 1,3-Dimethylxanthin, oder umfassend A317491 und 8-Cyclopentyl-1,3-dimethylxanthin, oder A317491 und 1,3-Dimethylxanthin, oder umfassend Spinorphin und 8-Cyclopentyl-1,3-dimethylxanthin, oder Spinorphin und 1,3-Dimethylxanthin.

7. Das Produkt zur Verwendung bei der Vorbeugung und/oder Behandlung eines Parasitenbefalls eines menschlichen und/oder tierischen Wirtes nach einem der Ansprüche 1 bis 6, ferner umfassend mindestens einem metallischen PR-Modulator.

8. Das Produkt zur Verwendung bei der Vorbeugung und/oder Behandlung eines Parasitenbefalls eines menschlichen und/oder tierischen Wirtes nach einem der Ansprüche 1 bis 7, wobei das Produkt gegen blutsaugende Arthropoden-Ektoparasiten gerichtet ist, beispielsweise gegen stechende Insekten wie Mücken, Sandmücken, Tsetsefliegen, Kriebelmücken und Bremsen, Pferde- und Hirschfliegen, Stallfliegen, Hornfliegen, Zuckmücken, Läuse, Bettwanzen, Raubwanzen, Flöhe und/oder gegen Milben oder Zecken, wie zum Beispiel Arten der Familie Ixodidae oder der Familie Argasidae, oder gegen Krebstier-Ektoparasiten.

9. Das Produkt zur Verwendung bei der Vorbeugung und/oder Behandlung eines Parasitenbefalls eines menschlichen und/oder tierischen Wirtes nacheinem der Ansprüche 1 bis 8, wobei das Produkt gegen Arthropoden-Endoparasiten gerichtet ist, wie zum Beispiel eine endoparasitäre Milbe, ein Krebstier-Endoparasit, endoparasitäre Larven der Dasselfliegenfamilien Cuterebridae und Hypodermatidae, endoparasitäre Bremsen, endoparasitäre Fliegen der Gattung *Hypoderma* oder endoparasitäre Fliegen der Familien Calliphoridae, Muscidae und Sacrophagidae.

10. Das Produkt zur Verwendung bei der Vorbeugung und/oder Behandlung eines Parasitenbefalls eines menschlichen und/oder tierischen Wirtes nach einem der Ansprüche 1 bis 9, wobei das Produkt ferner ein Insektizid, ein Synergist eines Insektizides , ein Akarizid, ein Endektozid, ein Parasitizid, ein Repellent, ein Chemosterilans und/oder ein Tensid umfasst.

11. Das Produkt zur Verwendung bei der Vorbeugung und/oder Behandlung eines Parasitenbefalls eines menschlichen und/oder tierischen Wirtes nach einem der Ansprüche 1 bis 10, das die Ausbreitung von Krankheiten, die durch arthropode Ektoparasiten als Krankheitsüberträger übertragen werden, wie zum Beispiel
- von stechenden Insekten übertragene Krankheiten, beispielsweise Malaria, Trypanosomiasis, Surra, Leishmaniose, Onchozerkose, Filariose, Denguefieber, Gelbfieber, Chikungunya, Pest, Lumpy-Skin-Krankheit, sowie Krankheiten, die durch das Zika-Virus, das West-Nil-Virus, *Bacillus anthracis* und das Blauzungenvirus verursacht werden, und/oder
- durch Zecken übertragene Krankheiten, beispielsweise Theileriose, Babesiose, Anaplasmose, Herzwasserkrankheit, Lyme-Borreliose, Rinderborreliose, Rückfallfieber, Tularämie, Q-Fieber, Krim-Kongo-Hämorrhagisches Fieber, Zeckenenzephalitis und Louping-ill,
verhindert oder verringert, indem der Arthropoden-Krankheitsvektor einem oder mehreren PR-Antagonisten ausgesetzt wird, oder indem bei dem ein parasitärer Arthropode mit einem oder mehreren PR-Antagonisten in Kontakt gebracht wird.

12. Das Produkt zur Verwendung bei der Vorbeugung und/oder Behandlung eines Parasitenbefalls eines menschlichen und/oder tierischen Wirtes nach einem der Ansprüche 1 bis 11, wobei das Produkt als Pulver oder in Form einer flüssigen Zusammensetzung, vorzugsweise in einer wasserdispergierbaren Emulsion oder als wässrige Lösung vorliegt.

13. Das Produkt zur Verwendung bei der Vorbeugung und/oder Behandlung eines Parasitenbefalls eines menschlichen und/oder tierischen Wirtes nach einem der Ansprüche 1 bis 12, wobei das Produkt zur topischen Anwendung auf Menschen und/oder Wirtstiere, zur Anwendung auf festen Oberflächen, zum Sprühen und/oder zum Tränken eines Gewebes, wie zum Beispiel Kleidung, eines Moskitonetzes oder einer Abdeckung für ein Wirtstier, geeignet ist.

14. Eine pharmazeutische Zusammensetzung, die Das Produkt zur Verwendung bei der Vorbeugung und/oder Behandlung eines Parasitenbefalls eines menschlichen und/oder tierischen Wirtes nacheinem der Ansprüche 1 bis 13 umfasst.

15. Ein Screeningverfahren zur Auswahl eines Antagonisten für einen PR eines parasitären Arthropoden, folgende Schritte umfassend:
- Anordnen mindestens eines Arthropoden-Versuchstiers, so dass mindestens eine tierische Zelle, einen oder mehrere PRn exprimiert, oder mindestens ein Teil des Versuchstieres, der eine oder mehrere Purin-sensitive Zellen umfasst und für elektrophysiologische Messungen zugänglich ist, oder das Vorbereiten eines Gewebes oder Organoids aus Wirbeltieren oder Wirbellosen, das einen oder mehrere PRn exprimiert, um die Zellaktivität zu bestimmen,
- Stimulieren des einen oder mehrerer PRn auf einer tierischen Zelle, oder auf einem Teil des Tieres, auf dem Gewebe oder dem Organoid eines Wirbeltieres oder Wirbellosen, das den einen oder die mehreren PRn aufweist, durch die Verabreichung einer definierten Dosis mindestens eines PR-Agonisten,
- Bestimmen der Reaktion der tierischen Zelle oder des Teils des Tieres, der den einen oder die mehreren PRn trägt, oder des Gewebes oder Organoids von Wirbeltieren oder Wirbellosen, das den einen oder die mehreren PRn trägt, über einen definierten Zeitraum während der Stimulation, beispielsweise durch bestimmen der Aktionspotenzialfrequenz,
- Kontaktieren des einer tierischen Zelle auf dem der eine oder mehrere PRn vorhanden ist/sind oder einem Teil eines Tieres, einem Gewebe oder Organoid tierischen Ursprungs, in dem der eine oder die mehreren PRn exprimiert werden, mit einer Testsubstanz in Kombination mit dem einen oder mehreren PR-Agonisten,
- Bestimmen der Reaktion des einen oder der mehreren PRn, die sich auf der tierischen Zelle befinden, oder die sich auf dem Teil des Tieres oder dem Gewebe oder Organoid von Wirbeltieren oder Wirbellosen befinden, über einen definierten Zeitraum während der Zugabe der Testsubstanz in Kombination mit mindestens einem PR-Agonisten, beispielsweise durch bestimmen der Aktionspotenzialfrequenz,
- Vergleichen der Reaktion der Tierzelle oder des Teils des Tieres mit dem einen oder den mehreren PRn, oder des Gewebes oder Organoids von Wirbeltieren oder Wirbellosen, das den einen oder die mehreren PRn enthält, in Abwesenheit der Testsubstanz mit der Reaktion der Tierzelle oder des Teils des Tieres, des Gewebes oder Organoids aus Wirbeltieren oder Wirbellosen, in Anwesenheit der Testsubstanz,
- Bestimmen der Eignung der Testsubstanz zur Verwendung als PR-Antagonist auf der Grundlage des Ergebnisses dieses Vergleichs.

16. Ein Screeningverfahren zur Bestimmung der Fähigkeit eines PR-Antagonisten, den Befall eines menschlichen und/oder tierischen Wirts durch einen parasitären Arthropoden zu verhindern, bestehend aus:
- Anordnen von mindestens einem Arthropoden-Versuchstier derart, dass mindestens eine tierische Zelle, die einen oder mehrere PRn exprimiert, oder mindestens ein Teil des Arthropoden-Versuchstieres, das einen oder mehrere Purinsensitiven Zellen aufweist, einem Fütterungsmedium ausgesetzt wird, das eine festgelegte Dosis von mindestens einem PR-Agonisten als Fütterungsstimulans enthält, oder dass die mindestens eine tierische Zelle oder der mindestens eine Teil des besagten Arthropoden-Versuchstiers einem Substrat, wie zum Beispiel einer Membran oder einem Mikroreservoir, ausgesetzt wird, das über oder an irgendeiner Stelle um das Fütterungsmedium angeordnet ist, welches eine festgelegten Dosis mindestens eines PR-Agonisten als Fütterungsstimulans enthält,
- Bestimmen der Fressrate des Arthropoden-Versuchstieres über einen definierten Zeitraum in Gegenwart von mindestens einem PR-Agonisten im Fütterungsmedium, oder bestimmen der Fressrate des besagten Arthropoden-Versuchstieres über einen definierten Zeitraum in Gegenwart von mindestens einer Testsubstanz, die auf die Membran oder auf einem Mikro-Reservoir aufgebracht ist, welches über oder an irgendeiner Stelle um das Fütterungsmedium angeordnet ist,
- In Kontakt bringen von mindestens einer Testsubstanz in Kombination mit dem mindestens einem PR-Agonisten als Fütterungsstimulans mit der besagten tierischen Zelle oder dem mindestens einen Teil des Arthropoden-Versuchstieres im Fütterungsmedium, oder die in Kontakt bringen mindestens einer Testsubstanz, die auf die Membran oder auf einem Mikroreservoir aufgebracht ist, welches über oder an irgendeiner Stelle um das Fütterungsmedium angeordnet ist,
- Bestimmen der Fressrate des genannten Arthropoden-Versuchstieres über einen definierten Zeitraum in Gegenwart des einen oder der mehreren PR-Agonisten und der mindestens einen Testsubstanz im Fütterungsmedium, oder bestimmen der Fressrate des genannten Arthropoden-Versuchstieres über einen definierten Zeitraum in Gegenwart von mindestens einer Testsubstanz, die auf die Membran oder auf einem Mikroreservoir aufgebracht ist, welches über oder an irgendeiner Stelle um das Fütterungsmedium angeordnet ist,
- Vergleichen der Fressraten des Arthropoden-Versuchstieres in Gegenwart und Abwesenheit der mindestens einer Testsubstanz,
- Bestimmen der Eignung von mindestens der einen Testsubstanz als Fresshemmer auf Grundlage des Ergebnisses dieses Vergleichs.

## Revendications

1. Un produit comprenant un ou plusieurs antagonistes des récepteurs purinergiques (PR) pour une utilisation dans la prévention et/ou le traitement de l'infestation parasitaire d'un hôte humain et/ou animal par un parasite arthropode en exposant le parasite arthropode à un ou plusieurs antagonistes des PR, ou en mettant le parasite arthropode en contact avec un ou plusieurs antagonistes des PR.

2. Produit comprenant un ou plusieurs antagonistes des récepteurs purinergiques (PR) pour une utilisation dans la prévention et/ou le traitement de l'infestation parasitaire d'un hôte humain et/ou animal selon la revendication 1, dans lequel le ou les antagonistes des PR empêchent le parasite arthropode de se nourrir des tissus de l'hôte, y compris le sang.

3. Produit pour une utilisation dans la prévention et/ou le traitement de l'infestation parasitaire d'un hôte humain et/ou animal par un parasite arthropode selon la revendication 1 ou 2, dans lequel le produit comprend au moins un des éléments suivants:
(i) un antagoniste des PR P1, ou
(ii) un antagoniste des PR P2X sélectionné parmi un groupe constitué d'un antagoniste des PR P2X₂, P2X₃ ou P2X_{2/3},
(iii) ou une combinaison de ceux-ci.

4. Produit pour une utilisation dans la prévention et/ou le traitement de l'infestation parasitaire d'un hôte humain et/ou animal par un parasite arthropode selon l'une des revendications 1 à 3, dans lequel le ou les antagonistes des PR P1 sont une xanthine, telle que la 1,3-diméthylxanthine ou la 8-cyclopentyl-1,3-diméthylxanthine.

5. Produit pour une utilisation dans la prévention et/ou le traitement de l'infestation parasitaire d'un hôte humain et/ou animal par un parasite arthropode selon des revendications 1 à 4, dans lequel un ou plusieurs antagonistes des PR P2X₂, P2X₃ ou P2X_{2/3} sont choisis parmi un groupe constitué d'une naphtylamine polysulfonée telle que la suramine, du phosphate de pyridoxal-6-azophényl-2,4-disulfonate ("PPADS"), d'un composé d'acide anthraquinone-sulfonique tel que l'acide 1-amino-4-[[4-[[4-chloro-6-[[3 (ou 4)-sulfophényl]amino]-1,3,5-triazin-2-yl]amino]-3-sulfophényl]amino]-9,10-dihydro-9,10-dioxo-2-anthracènesulfonique ("Réactif Bleu-2"), un tricarboxylate tel que l'acide 5-[(3-phénoxyphényl)méthyl-[(1S)-1,2,3,4-tétrahydronaphtalén-1-yl]carbamoyl]benzène-1,2,4-tricarboxylique ("A-317491"), un alcaloïde de pyrazine tel que la 2,3,5,6-tétraméthylpyrazine, et un heptapeptide tel que la spinorphine.

6. Produit pour une utilisation dans la prévention et/ou le traitement de l'infestation parasitaire d'un hôte humain et/ou animal par un parasite arthropode selon l'une des revendications 1 à 5, comprenant de la suramine et de la 8-cyclopentyl-1,3-diméthylxanthine, ou de la suramine et de la 2,3,5,6-tétraméthylpyrazine, ou de la suramine et de la 1,3-diméthylxanthine, ou de la suramine et d'un tricarboxylate tel que l'acide 5-[(3-phénoxyphényl)méthyl-[(1S)-1,2,3,4-tétrahydronaphtalén-1-yl]carbamoyl]benzène-1,2,4-tricarboxylique ("A-317491"), ou de la suramine et de la spinorphine, ou comprenant de la suramine et de la 2,3,5,6-tétraméthylpyrazine et de la 8-cyclopentyl-1,3-diméthylxanthine, ou de la suramine et de la 2,3,5,6-tétraméthylpyrazine et de la 1,3-diméthylxanthine, ou de la suramine et de la spinorphine et de la 8-cyclopentyl-1,3-diméthylxanthine, ou de la suramine et de la spinorphine et de la 1,3-diméthylxanthine, ou de la suramine et de la 2,3,5,6-tétraméthylpyrazine et de l'A-317491, ou comprenant de la 2,3,5,6-tétraméthylpyrazine et de la 8-cyclopentyl-1,3-diméthylxanthine, ou de la 2,3,5,6-tétraméthylpyrazine et de la 1,3-diméthylxanthine, ou de la 2,3,5,6-tétraméthylpyrazine et de l'A-317491, ou comprenant du phosphate de pyridoxal-6-azophényl-2,4-disulfonate et de la 8-cyclopentyl-1,3-diméthylxanthine, ou du phosphate de pyridoxal-6-azophényl-2,4-disulfonate et de la 1,3-diméthylxanthine, ou comprenant du Réactif Bleu-2 et de la 8-cyclopentyl-1,3-diméthylxanthine, ou du Réactif Bleu-2 et de la 1,3-diméthylxanthine, ou comprenant de l'A317491 et de la 8-cyclopentyl-1,3-diméthylxanthine, ou de l'A317491 et de la 1,3-diméthylxanthine, ou comprenant de la spinorphine et de la 8-cyclopentyl-1,3-diméthylxanthine, ou de la spinorphine et de la 1,3-diméthylxanthine.

7. Produit pour une utilisation dans la prévention et/ou le traitement de l'infestation parasitaire d'un hôte humain et/ou animal par un parasite arthropode selon l'une des revendications 1 à 6, comprenant en outre au moins un modulateur métallique de PR.

8. Produit pour une utilisation dans la prévention et/ou le traitement de l'infestation parasitaire d'un hôte humain et/ou animal par un parasite arthropode selon l'une des revendications 1 à 7, ledit produit étant dirigé contre les ectoparasites arthropodes hématophages, par exemple contre les insectes piqueurs tels que les moustiques, les phlébotomes, les mouches tsé-tsé, les simulies, les taons des chevaux et cervidés, les mouches des étables, les mouches des cornes, les moucherons, les poux, les punaises de lit, les punaises triatomines, les puces, et/ou contre les acariens ou les tiques, tels que les espèces du groupe des Ixodidae ou des Argasidae, ou contre les ectoparasites crustacés.

9. Produit pour une utilisation dans la prévention et/ou le traitement de l'infestation parasitaire d'un hôte humain et/ou animal par un parasite arthropode selon l'une des revendications 1 à 8, dans lequel le produit est dirigé contre les endoparasites arthropodes, tels qu'un acarien endoparasite, un endoparasite crustacé, les larves endoparasites des familles de mouches Cuterebridae et Hypodermatidae, les œstres endoparasites, les mouches endoparasites du genre *Hypoderma,* ou les mouches endoparasites des familles Calliphoridae, Muscidae et Sacrophagidae.

10. Produit pour une utilisation dans la prévention et/ou le traitement de l'infestation parasitaire d'un hôte humain et/ou animal par un parasite arthropode selon l'une des revendications 1 à 9, dans lequel le produit comprend en outre un insecticide, un synergiste d'insecticide, un acaricide, un endectocide, un parasiticide, un répulsif, un chimio-stérilisant et/ou un tensioactif.

11. Produit pour une utilisation dans la prévention et/ou le traitement de l'infestation parasitaire d'un hôte humain et/ou animal par un parasite arthropode selon l'une des revendications 1 à 10, prévenant ou réduisant la propagation des maladies transmises par des vecteurs ectoparasites arthropodes, telles que
- les maladies transmises par les insectes piqueurs, par exemple le paludisme, la trypanosomiase, la surra, la leishmaniose, l'onchocercose, la filariose, la dengue, la fièvre jaune, le chikungunya, la peste, la dermatose nodulaire contagieuse, et les maladies causées par le virus Zika, le virus du Nil occidental, *Bacillus anthracis* et le virus de la fièvre catarrhale ovine, et/ou
- les maladies transmises par les tiques, par exemple la théilériose, la babésiose, l'anaplasmose, la cowdriose, la borréliose de Lyme, la borréliose bovine, la fièvre récurrente, la tularémie, la fièvre Q, la fièvre hémorragique de Crimée-Congo, l'encéphalite à tiques et l'encéphalomyélite ovine,
par exposition de l'arthropode vecteur de maladie à un ou plusieurs antagonistes des PR, ou en contactant un parasite arthropode avec un ou plusieurs antagonistes des PR.

12. Produit pour une utilisation dans la prévention et/ou le traitement de l'infestation parasitaire d'un hôte humain et/ou animal par un parasite arthropode selon l'une des revendications 1 à 11, dans lequel le produit est fourni sous forme de poudre ou sous forme de composition liquide, de préférence une émulsion dispersible dans l'eau ou une solution à base d'eau.

13. Produit pour une utilisation dans la prévention et/ou le traitement de l'infestation parasitaire d'un hôte humain et/ou animal par un parasite arthropode selon l'une des revendications 1 à 12, le produit étant adapté à une application topique sur les humains et/ou les animaux hôtes, à une application sur toute surface solide, à une pulvérisation et/ou à un trempage d'un tissu, tel qu'un vêtement, une moustiquaire ou une couverture pour un animal hôte.

14. Une composition pharmaceutique comprenant le produit pour une utilisation dans la prévention et/ou le traitement de l'infestation parasitaire d'un hôte humain et/ou animal par un parasite arthropode selon l'une des revendications 1 à 13.

15. Un procédé de criblage pour la sélection d'un antagoniste des PR d'un parasite arthropode, comprenant:
- mettre en place d'au moins un arthropode test de manière à exposer au moins une cellule animale exprimant un ou plusieurs PR, ou au moins une portion de cet animal test comprenant une ou plusieurs cellules sensibles aux purines, pour une mesure électrophysiologique, ou la mise en place d'un tissu ou d'un organoïde d'origine vertébrée ou invertébrée exprimant un ou plusieurs PR pour déterminer l'activité cellulaire,
- stimuler, par la présentation d'une dose fixe d'au moins un agoniste de PR, un ou plusieurs PR portés par la cellule animale, ou portés par la portion de l'animal, ou portés par un tissu ou un organoïde d'origine vertébrée ou invertébrée exprimant un ou plusieurs PR,
- déterminer la réponse de la cellule animale, ou de la portion de l'animal, comprenant le ou les PR, ou dudit tissu ou organoïde d'origine vertébrée ou invertébrée exprimant un ou plusieurs PR sur une période définie pendant la stimulation, par exemple en déterminant la fréquence des potentiels d'action,
- présenter un composé test en combinaison avec au moins un agoniste de PR au ou aux PR portés par la cellule animale, ou portés par la portion de l'animal, ou portés par un tissu ou un organoïde d'origine vertébrée ou invertébrée exprimant un ou plusieurs PR,
- déterminer la réponse du ou des PR portés par la cellule animale, ou portés par la portion de l'animal, ou portés par un tissu ou un organoïde d'origine vertébrée ou invertébrée sur une période définie pendant la présentation du composé test en combinaison avec au moins un agoniste de PR, par exemple en déterminant la fréquence des potentiels d'action,
- comparer la réponse de la cellule animale, ou de la portion de l'animal, comprenant le ou les PR, ou dudit tissu ou organoïde d'origine vertébrée ou invertébrée exprimant un ou plusieurs PR en l'absence du composé test à la réponse de la cellule animale, ou de la partie animale, ou dudit tissu ou organoïde d'origine vertébrée ou invertébrée lorsqu'elle est présentée avec le composé test,
- déterminer la pertinence du composé test pour une utilisation en tant qu'antagoniste des PR en fonction du résultat de cette comparaison.

16. Un procédé de criblage permettant de déterminer la capacité d'un antagoniste des PR à prévenir l'infestation d'un hôte humain et/ou animal par un parasite arthropode, comprenant:
- mettre en place au moins un arthropode test de manière à exposer au moins une cellule animale exprimant un ou plusieurs PR, ou au moins une partie dudit arthropode test comprenant une ou plusieurs cellules sensibles aux purines, à un milieu d'alimentation contenant une dose fixe d'au moins un agoniste de PR comme stimulant de l'alimentation, ou exposer au moins une cellule animale ou au moins une partie dudit arthropode test sur un substrat tel qu'une membrane ou un microréservoir placé(e) au-dessus ou à proximité d'un milieu d'alimentation contenant une dose fixe d'au moins un agoniste de PR comme stimulant alimentaire,
- déterminer le taux d'alimentation dudit arthropode test sur une période définie en présence d'au moins un agoniste des PR dans le milieu d'alimentation, ou déterminer le taux d'alimentation dudit arthropode test sur une période définie en présence d'au moins un composé test appliqué sur ladite membrane ou ledit microréservoir placé(e) au-dessus ou à proximité du milieu d'alimentation,
- présenter au moins un composé test en combinaison avec au moins un agoniste de PR comme stimulant de l'alimentation à ladite cellule animale ou à ladite partie dudit arthropode test, ou présenter au moins un composé test appliqué sur ladite membrane ou ledit microréservoir placé(e) au-dessus ou à proximité du milieu d'alimentation,
- déterminer le taux d'alimentation dudit arthropode test sur une période définie en présence d'au moins un agoniste de PR et d'au moins un composé test dans le milieu de culture, ou déterminer le taux d'alimentation dudit arthropode test sur une période définie en présence d'au moins un composé test appliqué sur ladite membrane ou ledit microréservoir placé(e) au-dessus ou à proximité du milieu d'alimentation,
- comparer les taux d'alimentation dudit arthropode test en présence et en absence d'au moins un composé test,
- déterminer l'aptitude d'au moins un composé test à être utilisé comme inhibiteur de l'alimentation en fonction du résultat de cette comparaison.
